(19) Europäisches Patentamt
European Patent Office
Office européen des brevets

(11) EP 2 197 894 B1

(12) # EUROPEAN PATENT SPECIFICATION

(45) Date of publication and mention of the grant of the patent:
**25.09.2013 Bulletin 2013/39**

(21) Application number: **08840436.3**

(22) Date of filing: **15.10.2008**

(51) Int Cl.:
***C07H 21/04*** *(2006.01)* ***C12Q 1/68*** *(2006.01)*
***C12N 15/10*** *(2006.01)*

(86) International application number:
**PCT/US2008/079908**

(87) International publication number:
**WO 2009/052128 (23.04.2009 Gazette 2009/17)**

(54) **DEGENERATE OLIGONUCLEOTIDES AND THEIR USES**

DEGENERIERTE OLIGONUKLEOTIDE UND DEREN VERWENDUNG

OLIGONUCLÉOTIDES DÉGÉNÉRÉS ET LEURS UTILISATIONS

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **15.10.2007 US 872272**

(43) Date of publication of application:
**23.06.2010 Bulletin 2010/25**

(73) Proprietor: **Sigma-Aldrich Co. LLC**
**St Louis MO 63103 (US)**

(72) Inventors:
- **WARD, Brian**
  **St. Louis**
  **Missouri 63119 (US)**
- **HEUERMANN, Kenneth E.**
  **Kirkwood**
  **Missouri 63122 (US)**

(74) Representative: **Turner, Rhiannon Rosalind et al**
**Greaves Brewster LLP**
**Copa House**
**Station Road**
**Cheddar, North Somerset BS27 3AH (GB)**

(56) References cited:
**WO-A2-2004/081225 US-A- 5 663 062**
**US-A1- 2005 202 490 US-A1- 2006 121 491**
**US-B1- 6 497 880 US-B1- 6 562 572**

- **CHEUNG V G AND NELSON S F: "Whole genome amplification using a degenerate oligonucleotide primer allows hundreds of genotypes to be performed on less than one nanogram of genomic DNA", PROCEEDINGS OF THE NATIONAL ACADEMY OF SCIENCES, NATIONAL ACADEMY OF SCIENCES, vol. 93, 1 December 1996 (1996-12-01), pages 14676-14679, XP002126260, ISSN: 0027-8424, DOI: DOI:10.1073/PNAS. 93.25.14676**

EP 2 197 894 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### FIELD OF THE INVENTION

The present invention generally relates to oligonucleotides comprising a semi-random sequences. In particular, the semi-random sequence comprises degenerate nucleotides that are substantially non-complementary. Furthermore, the degenerate oligonucleotides may be used to amplify a population of target nucleic acids.

### BACKGROUND OF THE INVENTION

In many fields of research and diagnostics, the types of analyses that can be performed are limited by the quantity of available nucleic acids. Because of this, a variety of techniques have been developed to amplify small quantities of nucleic acids. Among these are whole genome amplification (WGA) and whole transcriptome amplification (WTA) procedures, which are non-specific amplification techniques designed to provide an unbiased representation of the entire starting genome or transcriptome.

Many of these amplification techniques utilize degenerate oligonucleotide primers in which each oligonucleotide comprises a random sequence (i.e., each nucleotide may be any nucleotide) or a non-complementary variable sequence (i.e., each nucleotide may be either of two non-complementary nucleotides). For example, Cheung et al. (Proc. Natl. Acad. Sci. USA, 1996, 93:14676-14679) uses primers comprising a random 6-mer region for whole genome amplification. WO2004/081225 and US2005/0202490 use primers comprising non-complementary two-fold degenerate nucleotides for whole genome or whole transcriptome amplifications. Whereas random primer complementarity results in excessive primer-dimer formation, amplification utilizing non-complementary variable primers, having reduced sequence complexity, is characterized by incomplete coverage of the starting population of nucleic acids.

Thus, there is a need for oligonucleotide primers that are substantially non- complementary while still having a high degree of sequence diversity. Such primers would be able to hybridize to a maximal number of sequences throughout the target nucleic acid, while the tendency to self- hybridize or cross- hybridize with other primers would be minimized. Such primers would be extremely useful in WGA or WTA techniques.

### SUMMARY OF THE INVENTION

One aspect of the present invention encompasses a plurality of degenerate oligonucleotides in which each oligonucleotide comprises the formula $N_mX_pZ_q$, wherein N, X, and Z are degenerate nucleotides, and m, p, and q are integers. In particular, m either is 0 or is from 2 to 20, and p and q are from 0 to 20, provided, however, that either no two integers are 0 or both m and q are 0, and further provided that oligonucleotides comprising N, which have at least two N residues, have at least one X or Z residue separating the two N residues. N is a 4-fold degenerate nucleotide, i.e., it may be adenosine (A), or cytidine (C), or guanosine (G), or thymidine/uridine (T/U). X is a 3-fold degenerate nucleotide selected from B, D, H, or V, wherein B may be C, G, or T/U; D may be A, G, or T/U; H may be A, C, or T/U, and V may be A, C, or G. Z is a 2-fold degenerate nucleotide selected from K, M, R, or Y, wherein K may be G or T/U; M may be A or C; R may be A or G; and Y may be C or T/U. When no integers are 0 and the plurality of oligonucleotides comprise formula $N_mX_pZ_q$ the oligonucleotides range from 4 to 60 nucleotides in length; when m is 0 and the plurality of oligonucleotides comprise formula $X_pZ_q$, the sum total of p and q is at least 6; when p is 0 and the plurality of oligonucleotides comprise formula $N_mZ_q$, the sum total of m and q is at least 6; when q is 0 and the plurality of oligonucleotides comprise formula $N_mX_p$, the sum total of m and p is at least 6; and when both m and q are 0 and the formula of the oligonucleotides is $X_p$, p is from 8 to 20.

Another aspect of the invention provides a method for amplifying a population of target nucleic acids. The method comprises contacting the population of target nucleic acids with a plurality of oligonucleotide primers as described above to form a plurality of nucleic acid-primer duplexes. The method further comprises replicating the plurality of nucleic acid-primer duplexes to create a library of replicated strands. Furthermore, the amount of replicated strands in the library exceeds the amount of starting target nucleic acids, which indicates amplification of the population of target nucleic acids.

Yet another aspect of the invention provides a kit for amplifying a population of target nucleic acids. The kit comprises a plurality of oligonucleotide primers as described above and a replicating enzyme.

Other aspects and features of the invention are described in more detail herein.

### DESCRIPTION OF THE FIGURES

**Figure 1** illustrates real-time quantitative PCR of amplified cDNA and unamplified cDNA. The deltaC(t) values for each primer set are plotted for unamplified cDNA (light gray bars), D-amplified cDNA (dark gray bars), and K-amplified cDNA (white bars).

**Figure 2** illustrates a microarray analysis of amplified cDNA and unamplified cDNA. Log base 2 ratios of amplified cDNA targets are plotted against the log base 2 ratio for unamplified cDNA targets. (A) presents D-amplified cDNA and (B) presents K-amplified cDNA.

**Figure 3** presents agarose gel images of WTA products amplified from NaOH-degraded RNA with preferred interrupted N library synthesis primers or control primers (1 K9 and 1 D9). The molecular size standards (in bp) that were loaded on each gel are presented on left, and the times (in minutes) of RNA exposure to NaOH are presented on the right.

**Figure 4** presents agarose gel images of WTA products amplified with preferred interrupted N library synthesis primers or control primers (1 K9 and 1 D9). Library synthesis was performed in the presence (+) or absence (-) of RNA, and with either MMLV reverse transcriptase (M) or MMLV reverse transcriptase and Klenow exominus DNA polymerase (MK). Library amplification was catalyzed by either JUMPSTART™ Taq DNA polymerase (JST) or KLENTAQ™ DNA polymerase (KT). The molecular size standards (in bp) that were loaded on each gel are presented on left, and the different reaction conditions are indicated on the right.

**Figure 5** presents agarose gel images of WTA products amplified with the five most preferred interrupted N library synthesis primers, various combinations of the preferred primers, or control primers. Library synthesis was performed with various concentrations of each primer or primer set. The primer concentrations (10, 2, 0.4, or 0.08 μM, from left to right) are diagrammed by triangles at the top of the images. The primer(s) within a given set are listed to the right of the images.

## DETAILED DESCRIPTION OF THE INVENTION

It has been discovered that oligonucleotides comprising a mixture of 4-fold degenerate nucleotides, 3-fold degenerate nucleotides, and/or 2-fold degenerate nucleotides have reduced intramolecular and/or intermolecular interactions, while retaining adequate sequence diversity for the representative amplification of a target nucleic acid. These oligonucleotides comprising semi-random regions are able to hybridize to many sequences throughout the target nucleic acid and provide many priming sites for replication and amplification of the target nucleic acid. At the same time, however, these oligonucleotides generally neither self-hybridize to form primer secondary structures nor cross-hybridize to form primer-dimer pairs.

### *(I) Plurality of Oligonucleotides*

One aspect of the present invention encompasses a plurality of oligonucleotides comprising a semi-random sequence. The semi-random sequence of the oligonucleotides comprises nucleotides that are substantially non-complementary, thereby reducing intramolecular and intermolecular interactions for the plurality of oligonucleotides. The semi-random sequence of the oligonucleotides, however, still provides substantial sequence diversity to permit hybridization to a maximal number of sequences contained within a target population of nucleic acids. The oligonucleotides of the invention may further comprise a non-random sequence.

#### (a) semi-random sequence

The semi-random sequence of the plurality of oligonucleotides comprises degenerate nucleotides (see Table A). A degenerate nucleotide may have 2-fold degeneracy (i.e., it may be one of two nucleotides), 3-fold degeneracy (i.e., it may one of three nucleotides), or 4-fold degeneracy (i.e., it may be one of four nucleotides). Because the oligonucleotides of the invention are degenerate, they are mixtures of similar, but not identical, oligonucleotides. The total degeneracy of a oligonucleotide may be calculated as follows:

$$\text{Degeneracy} = 2^a \times 3^b \times 4^c$$

wherein "a" is the total number 2-fold degenerate nucleotides (previously defined as Z, above), "b" is the total number of 3-fold degenerate nucleotides (previously defined as X, above), and "c" is the total number of 4-fold nucleotides (previously defined as N, above).

Degenerate nucleotides may be complementary, non-complementary, or partially non-complementary (see Table A). Complementarity between nucleotides refers to the ability to form a Watson-Crick base pair through specific hydrogen bonds (e.g., A and T base pair via two hydrogen bonds; and C and G are base pair via three hydrogen bonds).

**Table A.** Degenerate Nucleotides.

| Symbol | Origin of Symbol | Meaning* | Complementarity |
|---|---|---|---|
| K | keto | G or T/U | Non-complementary |
| M | amino | A or C | Non-complementary |
| R | purine | A or G | Non-complementary |
| Y | pyrimidine | C or T/U | Non-complementary |
| S | strong interactions | C or G | Complimentary |
| W | weak interactions | A or T/U | Complementary |
| B | not A | C or G or T/U | Partially non-complementary |
| D | not C | A or G or T/U | Partially non-complementary |
| H | not G | A or C or T/U | Partially non-complementary |
| V | not T/U | A or C or G | Partially non-complementary |
| N | any | A or C or G or T/U | Complementary |
| *A = adenosine, C = cytidine, G = guanosine, T = thymidine, U = uridine | | | |

The term "oligonucleotide," as used herein, refers to a molecule comprising two or more nucleotides. The nucleotides may be deoxyribonucleotides or ribonucleotides. The oligonucleotides may comprise the standard four nucleotides (i.e., A, C, G, and T/U), as well as nucleotide analogs. A nucleotide analog refers to a nucleotide having a modified purine or pyrimidine base and/or a modified ribose moiety. A nucleotide analog may be a naturally occurring nucleotide (e.g., inosine) or a non-naturally occurring nucleotide. Non-limiting examples of modifications on the sugar or base moieties of a nucleotide include the addition (or removal) of acetyl groups, amino groups, carboxyl groups, carboxymethyl groups, hydroxyl groups, methyl groups, phosphoryl groups, and thiol groups, as well as the substitution of the carbon and nitrogen atoms of the bases with other atoms (e.g., 7-deaza purines). Nucleotide analogs also include dideoxy nucleotides, 2'-O-methyl nucleotides, locked nucleic acids (LNA), peptide nucleic acids (PNA), and morpholinos. The backbone of the oligonucleotides may comprise phosphodiester linkages, as well as phosphothioate, phosphoramidite, or phosphorodiamidate linkages.

The plurality of degenerate oligonucleotides of the invention comprise the formula $N_mX_pZ_q$, wherein N, X and Z are degenerate nucleotides as follows:

N is a 4-fold degenerate nucleotide selected from adenosine (A), cytidine (C), guanosine (G), and thymidine/uridine (T/U);

X is a 3-fold degenerate nucleotide selected from B, D, H, or V, wherein B is C, G, or T/U; D is A, G, or T/U; H is A, C, or T/U; and V is A, C, or G;

Z is a 2-fold degenerate nucleotide selected from K, M, R, or Y, wherein K is G or T/U; M is A or C; R is A or G; and Y is C or T/U; and

m, p, and q are integers, m either is 0 or is from 2 to 20, p and q are from 0 to 20; provided, however, that either no two integers are 0 or both m and q are 0, and further provided that oligonucleotides comprising N, which have at least two N residues, have at least one X or Z residue separating the two N residues;

when no integers are 0 and the plurality of oligonucleotides comprise formula $N_mX_pZ_q$ the oligonucleotides range from 4 to 60 nucleotides in length; when m is 0 and the plurality of oligonucleotides comprise formula $X_pZ_q$, the sum total of p and q is at least 6; when p is 0 and the plurality of oligonucleotides comprise formula $N_mZ_q$, the sum total of m and q is at least 6; when q is 0 and the plurality of oligonucleotides comprise formula $N_mX_p$, the sum total of m and p is at least 6; and when both m and q are 0 and the formula of the oligonucleotides is $X_p$, p is from 8 to 20.

The plurality of oligonucleotides comprise complementary 4-fold degenerate nucleotides and/or partially non-complementary 3-fold degenerate nucleotides and/or non-complementary 2-fold degenerate nucleotides. Furthermore, in oligonucleotides containing N residues, the at least two N residues are separated by at least one X or Z residue. Thus, partially non-complementary 3-fold degenerate nucleotides and/or non-complementary 2-fold degenerate nucleotides interrupt the complementary N residues. The oligonucleotides of the invention, therefore, are substantially non-complementary.

In some embodiments, in which no two integers of the formula $N_mX_pZ_q$ are zero, the plurality of oligonucleotides may, therefore, comprise either formula $N_{2-20}X_{1-20}Z_{1-20}$ (or NXZ), formula $N_0X_{1-20}Z_{1-20}$ (or XZ), formula $N_{2-20}X_0Z_{1-20}$ (or NZ), or formula $N_{2-20}X_{1-20}Z_0$ (or NX) (see Table B for specific formulas) . Accordingly, oligonucleotides comprising formula NXZ, may range from about 4 nucleotides to about 60 nucleotides in length. More specifically, oligonucleotides comprising formula NXZ may range from about 48 nucleotides to about 60 nucleotides in length, from about 36 nucleotides to about 48 nucleotides in length, from about 24 nucleotides to about 36 nucleotides in length, from about 14 nucleotides to about 24 nucleotides in length, or from about 4 nucleotides to about 14 nucleotides in length.

**Table B.** Exemplary oligonucleotide formulas.

| NXZ | XZ | NZ | NX |
|---|---|---|---|
| NBK | BK | NK | NB |
| NBM | BM | NM | ND |
| NBR | BR | NR | NH |
| NBY | BY | NY | NV |
| NDK | DK | | |
| NDM | DM | | |
| NDR | DR | | |
| NDY | DY | | |
| NHK | HK | | |
| NHM | HM | | |
| NHR | HR | | |
| NHY | HY | | |
| NVK | VK | | |
| NVM | VM | | |
| NVR | VR | | |
| NVY | VY | | |

In an alternate embodiment, the plurality of oligonucleotides may comprise the formula $N_mX_p$, wherein N and X are nucleotides as defined above, m ranges from 2 to 13, p ranges from 1 to 12, the sum total of m and p is 14, and the at least two N residues are separated by at least one X residue. In another embodiment, the plurality of oligonucleotides may comprise the formula $N_mX_p$, wherein N and X are nucleotides as defined above, m ranges from 2 to 12, p ranges from 1 to 11, the sum total of m and p is 13, and the at least two N residues are separated by at least one X residue. In still another embodiment, the plurality of oligonucleotides may comprise the formula $N_mX_p$, wherein N and X are nucleotides as defined above, m ranges from 2 to 11, p ranges from 1 to 10, the sum total of m and p is 12, and the at least two N residues are separated by at least one X residue. In another embodiment, the plurality of oligonucleotides may comprise the formula $N_mX_p$, wherein N and X are nucleotides as defined above, m ranges from 2 to 10, p ranges from 1 to 9, the sum total of m and p is 11, and the at least two N residues are separated by at least one X residue. In yet another embodiment, the plurality of oligonucleotides may comprise the formula $N_mX_p$, wherein N and X are nucleotides as defined above, m ranges from 2 to 9, p ranges from 1 to 8, the sum total of m and p is 10, and the at least two N residues are separated by at least one X residue. In still another embodiment, the plurality of oligonucleotides may comprise the formula $N_mX_p$, wherein N and X are nucleotides as defined above, m ranges from 2 to 7, p ranges from 1 to 6, the sum total of m and p is 8, and the at least two N residues are separated by at least one X residue. In another embodiment, the plurality of oligonucleotides may comprise the formula $N_mX_p$, wherein N and X are nucleotides as defined above, m ranges from 2 to 6, p ranges from about 1 to 5, the sum total of m and p is 7, and the at least two N residues are separated by at least one X residue. In yet another embodiment, the plurality of oligonucleotides may comprise the formula $N_mX_p$, wherein N and X are nucleotides as defined above, m ranges from 2 to 5, p ranges from 1 to 4, the sum total of m and p is 6, and the at least two N residues are separated by at least one X residue. In a preferred embodiment, the plurality of oligonucleotides may comprise the formula $N_mX_p$, wherein N and X are nucleotides as defined above, m ranges from 2 to 8, p ranges from 1 to 7, the sum total of m and p is 9, and the at least two N residues are separated by at least one X residue. Table C presents (5' to 3') sequences of this preferred embodiment, i.e., a 9-nucleotide long semi-random region.

**Table C.** Nucleotide sequences (5' to 3') of an exemplary semi-random region.

| | | | | | |
|---|---|---|---|---|---|
| XXXXXXNXN | XXNNXXNNX | XNXNNNXNN | NXXXNXXXN | NXNXNNNNN | NNXNXNNNX |
| XXXXXNXXN | XXNNXXNNN | XNXNNNNXX | NXXXNXXNX | NXNNXXXXX | NNXNXNNNN |
| XXXXXNXNX | XXNNXNXXX | XNXNNNNXN | NXXXNXXNN | NXNNXXXXN | NNXNNXXXX |
| XXXXXNXNN | XXNNXNXXN | XNXNNNNNX | NXXXNXNXX | NXNNXXXNX | NNXNNXXXN |
| XXXXXNNXN | XXNNXNXNX | XNXNNNNNN | NXXXNXNXN | NXNNXXXNN | NNXNNXXNX |
| XXXXNXXXN | XXNNXNXNN | XNNXXXXXN | NXXXNXNNX | NXNNXXNXX | NNXNNXXNN |
| XXXXNXXNX | XXNNXNNXX | XNNXXXXNX | NXXXNXNNN | NXNNXXNXN | NNXNNXNXX |
| XXXXNXXNN | XXNNXNNXN | XNNXXXXNN | NXXXNNXXX | NXNNXXNNX | NNXNNXNXN |
| XXXXNXNXX | XXNNXNNNX | XNNXXXNXX | NXXXNNXXN | NXNNXXNNN | NNXNNXNNX |
| XXXXNXNXN | XXNNXNNNN | XNNXXXNXN | NXXXNNXNX | NXNNXNXXX | NNXNNXNNN |
| XXXXNXNNX | XXNNNXXXN | XNNXXXNNX | NXXXNNXNN | NXNNXNXXN | NNXNNNXXX |
| XXXXNXNNN | XXNNNXXNX | XNNXXXNNN | NXXXNNNXX | NXNNXNXNX | NNXNNNXXN |
| XXXXNNXXN | XXNNNXXNN | XNNXXNXXX | NXXXNNNXN | NXNNXNXNN | NNXNNNXNX |
| XXXXNNXNX | XXNNNXNXX | XNNXXNXXN | NXXXNNNNX | NXNNXNNXX | NNXNNNXNN |
| XXXXNNXNN | XXNNNXNXN | XNNXXNXNX | NXXXNNNNN | NXNNXNNXN | NNXNNNNXX |
| XXXXNNNXN | XXNNNXNNX | XNNXXNXNN | NXXNXXXXX | NXNNXNNNX | NNXNNNNXN |
| XXXNXXXXX | XXNNNXNNN | XNNXXNNXX | NXXNXXXXN | NXNNXNNNN | NNXNNNNNX |
| XXXNXXXXN | XXNNNNXXN | XNNXXNNXN | NXXNXXXNX | NXNNNXXXX | NNXNNNNNN |
| XXXNXXXNX | XXNNNNXNX | XNNXXNNNX | NXXNXXXNN | NXNNNXXXN | NNNXXXXXN |
| XXXNXXXNN | XXNNNNXNN | XNNXXNNNN | NXXNXXNXX | NXNNNXXNX | NNNXXXXNX |
| XXXNXXNXX | XXNNNNNXN | XNNXNXXXX | NXXNXXNXN | NXNNNXXNN | NNNXXXXNN |
| XXXNXXNXN | XNXXXXXXN | XNNXNXXXN | NXXNXXNNX | NXNNNXNXX | NNNXXXNXX |
| XXXNXXNNX | XNXXXXXNX | XNNXNXXNX | NXXNXXNNN | NXNNNXNXN | NNNXXXNXN |
| XXXNXXNNN | XNXXXXXNN | XNNXNXXNN | NXXNXNXXX | NXNNNXNNX | NNNXXXNNX |
| XXXNXNXXX | XNXXXXNXX | XNNXNXNXX | NXXNXNXXN | NXNNNXNNN | NNNXXXNNN |
| XXXNXNXXN | XNXXXXNXN | XNNXNXNXN | NXXNXNXNX | NXNNNNXXX | NNNXXNXXX |
| XXXNXNXNX | XNXXXXNNX | XNNXNXNNX | NXXNXNXNN | NXNNNNXXN | NNNXXNXXN |
| XXXNXNXNN | XNXXXXNNN | XNNXNXNNN | NXXNXNNXX | NXNNNNXNX | NNNXXNXNX |
| XXXNXNNXX | XNXXXNXXX | XNNXNNXXX | NXXNXNNXN | NXNNNNXNN | NNNXXNXNN |
| XXXNXNNXN | XNXXXNXXN | XNNXNNXXN | NXXNXNNNX | NXNNNNNXX | NNNXXNNXX |
| XXXNXNNNX | XNXXXNXNX | XNNXNNXNX | NXXNXNNNN | NXNNNNNXN | NNNXXNNXN |
| XXXNXNNNN | XNXXXNXNN | XNNXNNXNN | NXXNNXXXX | NXNNNNNNX | NNNXXNNNX |
| XXXNNXXXN | XNXXXNNXX | XNNXNNNXX | NXXNNXXXN | NXNNNNNNN | NNNXXNNNN |
| XXXNNXXNX | XNXXXNNXN | XNNXNNNXN | NXXNNXXNX | NNXXXXXXN | NNNXNXXXX |
| XXXNNXXNN | XNXXXNNNX | XNNXNNNNX | NXXNNXXNN | NNXXXXXNX | NNNXNXXXN |
| XXXNNXNXX | XNXXXNNNN | XNNXNNNNN | NXXNNXNXX | NNXXXXXNN | NNNXNXXNX |
| XXXNNXNXN | XNXXNXXXX | XNNNXXXXN | NXXNNXNXN | NNXXXXNXX | NNNXNXXNN |
| XXXNNXNNX | XNXXNXXXN | XNNNXXXNX | NXXNNXNNX | NNXXXXNXN | NNNXNXNXX |

(continued)

| | | | | | |
|---|---|---|---|---|---|
| XXXNNXNNN | XNXXNXXNX | XNNNXXXNN | NXXNNXNNN | NNXXXXNNX | NNNXNXNXN |
| XXXNNNXXN | XNXXNXXNN | XNNNXXNXX | NXXNNNXXX | NNXXXXNNN | NNNXNXNNX |
| XXXNNNXNX | XNXXNXNXX | XNNNXXNXN | NXXNNNXXN | NNXXXNXXX | NNNXNXNNN |
| XXXNNNXNN | XNXXNXNXN | XNNNXXNNX | NXXNNNXNX | NNXXXNXXN | NNNXNNXXX |
| XXXNNNNXN | XNXXNXNNX | XNNNXXNNN | NXXNNNXNN | NNXXXNXNX | NNNXNNXXN |
| XXNXXXXXN | XNXXNXNNN | XNNNXNXXX | NXXNNNNXX | NNXXXNXNN | NNNXNNXNX |
| XXNXXXXNX | XNXXNNXXX | XNNNXNXXN | NXXNNNNXN | NNXXXNNXX | NNNXNNXNN |
| XXNXXXXNN | XNXXNNXXN | XNNNXNXNX | NXXNNNNNX | NNXXXNNXN | NNNXNNNXX |
| XXNXXXNXX | XNXXNNXNX | XNNNXNXNN | NXXNNNNNN | NNXXXNNNX | NNNXNNNXN |
| XXNXXXNXN | XNXXNNXNN | XNNNXNNXX | NXNXXXXXX | NNXXXNNNN | NNNXNNNNX |
| XXNXXXNNX | XNXXNNNXX | XNNNXNNXN | NXNXXXXXN | NNXXNXXXX | NNNXNNNNN |
| XXNXXXNNN | XNXXNNNXN | XNNNXNNNX | NXNXXXXNX | NNXXNXXXN | NNNNXXXXX |
| XXNXXNXXX | XNXXNNNNX | XNNNXNNNN | NXNXXXXNN | NNXXNXXNX | NNNNXXXXN |
| XXNXXNXXN | XNXXNNNNN | XNNNNXXXN | NXNXXXNXX | NNXXNXXNN | NNNNXXXNX |
| XXNXXNXNX | XNXNXXXXX | XNNNNXXNX | NXNXXXNXN | NNXXNXNXX | NNNNXXXNN |
| XXNXXNXNN | XNXNXXXXN | XNNNNXXNN | NXNXXXNNX | NNXXNXNXN | NNNNXXNXX |
| XXNXXNNXX | XNXNXXXNX | XNNNNXNXX | NXNXXXNNN | NNXXNXNNX | NNNNXXNXN |
| XXNXXNNXN | XNXNXXXNN | XNNNNXNXN | NXNXXNXXX | NNXXNXNNN | NNNNXXNNX |
| XXNXXNNNX | XNXNXXNXX | XNNNNXNNX | NXNXXNXXN | NNXXNNXXX | NNNNXXNNN |
| XXNXXNNNN | XNXNXXNXN | XNNNNXNNN | NXNXXNXNX | NNXXNNXXN | NNNNXNXXX |
| XXNXNXXXX | XNXNXXNNX | XNNNNNXXN | NXNXXNXNN | NNXXNNXNX | NNNNXNXXN |
| XXNXNXXXN | XNXNXXNNN | XNNNNNXNX | NXNXXNNXX | NNXXNNXNN | NNNNXNXNX |
| XXNXNXXNX | XNXNXNXXX | XNNNNNXNN | NXNXXNNXN | NNXXNNNXX | NNNNXNXNN |
| XXNXNXXNN | XNXNXNXXN | XNNNNNNXN | NXNXXNNNX | NNXXNNNXN | NNNNXNNXX |
| XXNXNXNXX | XNXNXNXNX | NXXXXXXXN | NXNXXNNNN | NNXXNNNNX | NNNNXNNXN |
| XXNXNXNXN | XNXNXNXNN | NXXXXXXNX | NXNXNXXXX | NNXXNNNNN | NNNNXNNNX |
| XXNXNXNNX | XNXNXNNXX | NXXXXXXNN | NXNXNXXXN | NNXNXXXXX | NNNNXNNNN |
| XXNXNXNNN | XNXNXNNXN | NXXXXXNXX | NXNXNXXNX | NNXNXXXXN | NNNNNXXXX |
| XXNXNNXXX | XNXNXNNNX | NXXXXXNXN | NXNXNXXNN | NNXNXXXNX | NNNNNXXXN |
| XXNXNNXXN | XNXNXNNNN | NXXXXXNNX | NXNXNXNXX | NNXNXXXNN | NNNNNXXNX |
| XXNXNNXNX | XNXNNXXXX | NXXXXXNNN | NXNXNXNXN | NNXNXXNXX | NNNNNXXNN |
| XXNXNNXNN | XNXNNXXXN | NXXXXNXXX | NXNXNXNNX | NNXNXXNXN | NNNNNXNXX |
| XXNXNNNXX | XNXNNXXNX | NXXXXNXXN | NXNXNXNNN | NNXNXXNNX | NNNNNXNXN |
| XXNXNNNXN | XNXNNXXNN | NXXXXNXNX | NXNXNNXXX | NNXNXXNNN | NNNNNXNNX |
| XXNXNNNNX | XNXNNXNXX | NXXXXNXNN | NXNXNNXXN | NNXNXNXXX | NNNNNXNNN |
| XXNXNNNNN | XNXNNXNXN | NXXXXNNXX | NXNXNNXNX | NNXNXNXXN | NNNNNNXXX |
| XXNNXXXXN | XNXNNXNNX | NXXXXNNXN | NXNXNNXNN | NNXNXNXNX | NNNNNNXXN |
| XXNNXXXNX | XNXNNXNNN | NXXXXNNNX | NXNXNNNXX | NNXNXNXNN | NNNNNNXNX |
| XXNNXXXNN | XNXNNNXXX | NXXXXNNNN | NXNXNNNXN | NNXNXNNXX | NNNNNNXNN |

(continued)

| | | | | | |
|---|---|---|---|---|---|
| XXNNXXNXX | XNXNNNXXN | NXXXNXXXX | NXNXNNNNX | NNXNXNNXN | NNNNNNNXN |
| XXNNXXNXN | XNXNNNXNX | | | | |

In still another alternate embodiment, the plurality of oligonucleotides may comprise formula $N_mX_p$, wherein N and X are nucleotides as defined above, m ranges from 2 to 13, p ranges from 1 to 12, and the sum total of m and p ranges from 6 to 14, the at least two N residues are separated by at least one X residue, and there are no more than three consecutive N residues. In this embodiment, therefore, partially non-complementary 3-fold degenerate nucleotides are interspersed throughout the sequence such that there are no long runs (≥4) of the complementary 4-fold degenerate nucleotide (N). In general, such a design may reduce self-hybridization and/or cross-hybridization within the plurality of oligonucleotides. In an exemplary embodiment, the plurality of oligonucleotides may comprise formula $N_mX_p$, wherein N and X are nucleotides as defined above, m ranges from 2 to 8, p ranges from 1 to 7, and the sum total of m and p is 9, the at least two N residues are separated by at least one X residue, and there are no more than three consecutive N residues. Table D lists the (5' to 3') sequences of this preferred embodiment, i.e., a 9-nucleotide long semi-random region containing no more that three consecutive N residues.

**Table D.** Nucleotide sequences (5' to 3') of an exemplary semi-random region having no more than 3 consecutive N residues.

| | | | | | |
|---|---|---|---|---|---|
| XXXXXXNXN | XXNXNNXXX | XNXNXNXXX | NXXXXXXNX | NXNXXNXXN | NNXXNXNNX |
| XXXXXNXXN | XXNXNNXXN | XNXNXNXXN | NXXXXXXNN | NXNXXNXNX | NNXXNXNNN |
| XXXXXNXNX | XXNXNNXNX | XNXNXNXNX | NXXXXXNXX | NXNXXNXNN | NNXXNNXXX |
| XXXXXNXNN | XXNXNNXNN | XNXNXNXNN | NXXXXXNXN | NXNXXNNXX | NNXXNNXXN |
| XXXXXNNXN | XXNXNNNXX | XNXNXNNXX | NXXXXXNNX | NXNXXNNXN | NNXXNNXNX |
| XXXXNXXXN | XXNXNNNXN | XNXNXNNXN | NXXXXXNNN | NXNXXNNNX | NNXXNNXNN |
| XXXXNXXNX | XXNNXXXXN | XNXNXNNNX | NXXXXNXXX | NXNXNXXXX | NNXXNNNXX |
| XXXXNXXNN | XXNNXXXNX | XNXNNXXXX | NXXXXNXXN | NXNXNXXXN | NNXXNNNXN |
| XXXXNXNXX | XXNNXXXNN | XNXNNXXXN | NXXXXNXNX | NXNXNXXNX | NNXNXXXXX |
| XXXXNXNXN | XXNNXXNXX | XNXNNXXNX | NXXXXNXNN | NXNXNXXNN | NNXNXXXXN |
| XXXXNXNNX | XXNNXXNXN | XNXNNXXNN | NXXXXNNXX | NXNXNXNXX | NNXNXXXNX |
| XXXXNXNNN | XXNNXXNNX | XNXNNXNXX | NXXXXNNXN | NXNXNXNXN | NNXNXXXNN |
| XXXXNNXXN | XXNNXXNNN | XNXNNXNXN | NXXXXNNNX | NXNXNXNNX | NNXNXXNXX |
| XXXXNNXNX | XXNNXNXXX | XNXNNXNNX | NXXXNXXXX | NXNXNXNNN | NNXNXXNXN |
| XXXXNNXNN | XXNNXNXXN | XNXNNXNNN | NXXXNXXXN | NXNXNNXXX | NNXNXXNNX |
| XXXXNNNXN | XXNNXNXNX | XNXNNNXXX | NXXXNXXNX | NXNXNNXXN | NNXNXXNNN |
| XXXNXXXXX | XXNNXNXNN | XNXNNNXXN | NXXXNXXNN | NXNXNNXNX | NNXNXNXXX |
| XXXNXXXXN | XXNNXNNXX | XNXNNNXNX | NXXXNXNXX | NXNXNNXNN | NNXNXNXXN |
| XXXNXXXNX | XXNNXNNXN | XNXNNNXNN | NXXXNXNXN | NXNXNNNXX | NNXNXNXNX |
| XXXNXXXNN | XXNNXNNNX | XNNXXXXXN | NXXXNXNNX | NXNXNNNXN | NNXNXNXNN |
| XXXNXXNXX | XXNNNXXXN | XNNXXXXNX | NXXXNXNNN | NXNNXXXXX | NNXNXNNXX |
| XXXNXXNXN | XXNNNXXNX | XNNXXXXNN | NXXXNNXXX | NXNNXXXXN | NNXNXNNXN |
| XXXNXXNNX | XXNNNXXNN | XNNXXXNXX | NXXXNNXXN | NXNNXXXNX | NNXNXNNNX |
| XXXNXXNNN | XXNNNXNXX | XNNXXXNXN | NXXXNNXNX | NXNNXXXNN | NNXNNXXXX |
| XXXNXNXXX | XXNNNXNXN | XNNXXXNNX | NXXXNNXNN | NXNNXXNXX | NNXNNXXXN |
| XXXNXNXXN | XXNNNXNNX | XNNXXXNNN | NXXXNNNXX | NXNNXXNXN | NNXNNXXNX |

(continued)

| | | | | | |
|---|---|---|---|---|---|
| XXXNXNXNX | XXNNNXNNN | XNNXXNXXX | NXXXNNNXN | NXNNXXNNX | NNXNNXXNN |
| XXXNXNXNN | XNXXXXXXN | XNNXXNXXN | NXXNXXXXX | NXNNXXNNN | NNXNNXNXX |
| XXXNXNNXX | XNXXXXXNX | XNNXXNXNX | NXXNXXXXN | NXNNXNXXX | NNXNNXNXN |
| XXXNXNNXN | XNXXXXXNN | XNNXXNXNN | NXXNXXXNX | NXNNXNXXN | NNXNNXNNX |
| XXXNXNNNX | XNXXXXNXX | XNNXXNNXX | NXXNXXXNN | NXNNXNXNX | NNXNNXNNN |
| XXXNNXXXN | XNXXXXNXN | XNNXXNNXN | NXXNXXNXX | NXNNXNXNN | NNXNNNXXX |
| XXXNNXXNX | XNXXXXNNX | XNNXXNNNX | NXXNXXNXN | NXNNXNNXX | NNXNNNXXN |
| XXXNNXXNN | XNXXXXNNN | XNNXNXXXX | NXXNXXNNX | NXNNXNNXN | NNXNNNXNX |
| XXXNNXNXX | XNXXXNXXX | XNNXNXXXN | NXXNXXNNN | NXNNXNNNX | NNXNNNXNN |
| XXXNNXNXN | XNXXXNXXN | XNNXNXXNX | NXXNXNXXX | NXNNNXXXX | NNNXXXXXN |
| XXXNNXNNX | XNXXXNXNX | XNNXNXXNN | NXXNXNXXN | NXNNNXXXN | NNNXXXXNX |
| XXXNNXNNN | XNXXXNXNN | XNNXNXNXX | NXXNXNXNX | NXNNNXXNX | NNNXXXXNN |
| XXXNNNXXN | XNXXXNNXX | XNNXNXNXN | NXXNXNXNN | NXNNNXXNN | NNNXXXNXX |
| XXXNNNXNX | XNXXXNNXN | XNNXNXNNX | NXXNXNNXX | NXNNNXNXX | NNNXXXNXN |
| XXXNNNXNN | XNXXXNNNX | XNNXNXNNN | NXXNXNNXN | NXNNNXNXN | NNNXXXNNX |
| XXNXXXXXN | XNXXNXXXX | XNNXNNXXX | NXXNXNNNX | NXNNNXNNX | NNNXXXNNN |
| XXNXXXXNX | XNXXNXXXN | XNNXNNXXN | NXXNNXXXX | NXNNNXNNN | NNNXXNXXX |
| XXNXXXXNN | XNXXNXXNX | XNNXNNXNX | NXXNNXXXN | NNXXXXXXN | NNNXXNXXN |
| XXNXXXNXX | XNXXNXXNN | XNNXNNXNN | NXXNNXXNX | NNXXXXXNX | NNNXXNXNX |
| XXNXXXNXN | XNXXNXNXX | XNNXNNNXX | NXXNNXXNN | NNXXXXXNN | NNNXXNXNN |
| XXNXXXNNX | XNXXNXNXN | XNNXNNNXN | NXXNNXNXX | NNXXXXNXX | NNNXXNNXX |
| XXNXXXNNN | XNXXNXNNX | XNNNXXXXN | NXXNNXNXN | NNXXXXNXN | NNNXXNNXN |
| XXNXXNXXX | XNXXNXNNN | XNNNXXXNX | NXXNNXNNX | NNXXXXNNX | NNNXXNNNX |
| XXNXXNXXN | XNXXNNXXX | XNNNXXXNN | NXXNNXNNN | NNXXXXNNN | NNNXNXXXX |
| XXNXXNXNX | XNXXNNXXN | XNNNXXNXX | NXXNNNXXX | NNXXXNXXX | NNNXNXXXN |
| XXNXXNXNN | XNXXNNXNX | XNNNXXNXN | NXXNNNXXN | NNXXXNXXN | NNNXNXXNX |
| XXNXXNNXX | XNXXNNXNN | XNNNXXNNX | NXXNNNXNX | NNXXXNXNX | NNNXNXXNN |
| XXNXXNNXN | XNXXNNNXX | XNNNXXNNN | NXXNNNXNN | NNXXXNXNN | NNNXNXNXX |
| XXNXXNNNX | XNXXNNNXN | XNNNXNXXX | NXNXXXXXX | NNXXXNNXX | NNNXNXNXN |
| XXNXNXXXX | XNXNXXXXX | XNNNXNXXN | NXNXXXXXN | NNXXXNNXN | NNNXNXNNX |
| XXNXNXXXN | XNXNXXXXN | XNNNXNXNX | NXNXXXXNX | NNXXXNNNX | NNNXNXNNN |
| XXNXNXXNX | XNXNXXXNX | XNNNXNXNN | NXNXXXXNN | NNXXNXXXXX | NNNXNNXXX |
| XXNXNXXNN | XNXNXXXNN | XNNNXNNXX | NXNXXXNXX | NNXXNXXXN | NNNXNNXXN |
| XXNXNXNXX | XNXNXXNXX | XNNNXNNXN | NXNXXXNXN | NNXXNXXNX | NNNXNNXNX |
| XXNXNXNXN | XNXNXXNXN | XNNNXNNNX | NXNXXXNNX | NNXXNXXNN | NNNXNNXNN |
| XXNXNXNNX | XNXNXXNNX | XNNNXNNNN | NXNXXXNNN | NNXXNXNXX | NNNXNNNXX |
| XXNXNXNNN | XNXNXXNNN | NXXXXXXXN | NXNXXNXXX | NNXXNXNXN | NNNXNNNXN |

In yet another alternate embodiment, the plurality of oligonucleotides may comprise the formula $N_mZ_q$, wherein N and Z are nucleotides as defined above, m ranges from 2 to 13, q ranges from 1 to 12, the sum total of m and q is 14, and the at least two N residues are separated by at least one Z residue. In another embodiment, the plurality of oligonucleotides may comprise the formula $N_mZ_q$, wherein N and Z are nucleotides as defined above, m ranges from 2 to 12, q ranges from 1 to 11, the sum total of m and q is 13, and the at least two N residues are separated by at least one Z residue. In still another embodiment, the plurality of oligonucleotides may comprise the formula $N_mZ_q$, wherein N and Z are nucleotides as defined above, m ranges from 2 to 11, q ranges from 1 to 10, the sum total of m and q is 12, and the at least two N residues are separated by at least one Z residue. In another embodiment, the plurality of oligonucleotides may comprise the formula $N_mZ_q$, wherein N and Z are nucleotides as defined above, m ranges from 2 to 10, q ranges from 1 to 9, the sum total of m and q is 11, and the at least two N residues are separated by at least one Z residue. In yet another embodiment, the plurality of oligonucleotides may comprise the formula $N_mZ_q$, wherein N and Z are nucleotides as defined above, m ranges from 2 to 9, q ranges from 1 to 8, the sum total of m and q is 10, and the at least two N residues are separated by at least one Z residue. In still another embodiment, the plurality of oligonucleotides may comprise the formula $N_mZ_q$, wherein N and Z are nucleotides as defined above, m ranges from 2 to 7, q ranges from 1 to 6, the sum total of m and q is 8, and the at least two N residues are separated by at least one Z residue. In another embodiment, the plurality of oligonucleotides may comprise the formula $N_mZ_q$, wherein N and Z are nucleotides as defined above, m ranges from 2 to 6, q ranges from 1 to 5, the sum total of m and q is 7, and the at least two N residues are separated by at least one Z residue. In yet another embodiment, the plurality of oligonucleotides may comprise the formula $N_mZ_q$, wherein N and Z are nucleotides as defined above, m ranges from 2 to 5, q ranges from 1 to 4, the sum total of m and q is 6, and the at least two N residues are separated by at least one Z residue. In a preferred embodiment, the plurality of oligonucleotides may comprise the formula $N_mZ_q$, wherein N and Z are nucleotides as defined above, m ranges from 2 to 8, q ranges from 1 to 7, the sum total of m and q is 9, and the at least two N residues are separated by at least one Z residue. Table E presents (5' to 3') sequences of this preferred embodiment, i.e., a 9-nucleotide long semi-random region.

**Table E.** Nucleotide sequences (5' to 3') of an exemplary semi-random region.

| | | | | | |
|---|---|---|---|---|---|
| ZZZZZZNZN | ZZNNZZNNZ | ZNZNNNZNN | NZZZNZZZN | NZNZNNNNN | NNZNZNNNZ |
| ZZZZZNZZN | ZZNNZZNNN | ZNZNNNNZZ | NZZZNZZNZ | NZNNZZZZZ | NNZNZNNNN |
| ZZZZZNZNZ | ZZNNZNZZZ | ZNZNNNNZN | NZZZNZZNN | NZNNZZZZN | NNZNNZZZZ |
| ZZZZNNZNN | ZZNNZNZZN | ZNZNNNNNZ | NZZZNZNZZ | NZNNZZZNZ | NNZNNZZZN |
| ZZZZZNNZN | ZZNNZNZNZ | ZNZNNNNNN | NZZZNZNZN | NZNNZZZNN | NNZNNZZNZ |
| ZZZZNZZZN | ZZNNZNZNN | ZNNZZZNZN | NZZZNZNNZ | NZNNZZNZZ | NNZNNZZNN |
| ZZZZNZZNZ | ZZNNZNNZZ | ZNNZZZZNZ | NZZZNZNNN | NZNNZZNZN | NNZNNZNZZ |
| ZZZZNZZNN | ZZNNZNNZN | ZNNZZZZNN | NZZZNNZZZ | NZNNZZNNZ | NNZNNZNZN |
| ZZZZNZNZZ | ZZNNZNNNZ | ZNNZZZNZZ | NZZZNNZZN | NZNNZZNNN | NNZNNZNNZ |
| ZZZZNZNZN | ZZNNZNNNN | ZNNZZZNZN | NZZZNNZNZ | NZNNZNZZZ | NNZNNZNNN |
| ZZZZNZNNZ | ZZNNNZZZN | ZNNZZZNNZ | NZZZNNZNN | NZNNZNZZN | NNZNNNZZZ |
| ZZZZNZNNN | ZZNNNZZNZ | ZNNZZZNNN | NZZZNNNZZ | NZNNZNZNZ | NNZNNNZZN |
| ZZZZNNZZN | ZZNNNZZNN | ZNNZZNZZZ | NZZZNNNZN | NZNNZNZNN | NNZNNNZNZ |
| ZZZZNNZNZ | ZZNNNZNZZ | ZNNZZNZZN | NZZZNNNNZ | NZNNZNNZZ | NNZNNNZNN |
| ZZZZNNZNN | ZZNNNZNZN | ZNNZZNZNZ | NZZZNNNNN | NZNNZNNZN | NNZNNNNZZ |
| ZZZZNNNZN | ZZNNNZNNZ | ZNNZZNZNN | NZZNZZZZZ | NZNNZNNNZ | NNZNNNNZN |
| ZZZNZZZZZ | ZZNNNZNNN | ZNNZZNNZZ | NZZNZZZZN | NZNNZNNNN | NNZNNNNNZ |
| ZZZNZZZZN | ZZNNNNZZN | ZNNZZNNZN | NZZNZZZNZ | NZNNNZZZZ | NNZNNNNNN |
| ZZZNZZZNZ | ZZNNNNZNZ | ZNNZZNNNZ | NZZNZZZNN | NZNNNZZZN | NNNZZZZZN |
| ZZZNZZZNN | ZZNNNNZNN | ZNNZZNNNN | NZZNZZNZZ | NZNNNZZNZ | NNNZZZZNZ |
| ZZZNZZNZZ | ZZNNNNNZN | ZNNZNZZZZ | NZZNZZNZN | NZNNNZZNN | NNNZZZZNN |
| ZZZNZZNZN | ZNZZZZZZN | ZNNZNZZZN | NZZNZZNNZ | NZNNNZNZZ | NNNZZZNZZ |
| ZZZNZZNNZ | ZNZZZZZNZ | ZNNZNZZNZ | NZZNZZNNN | NZNNNZNZN | NNNZZZNZN |

(continued)

| | | | | | |
|---|---|---|---|---|---|
| ZZZNZZNNN | ZNZZZZZNN | ZNNZNZZNN | NZZNZNZZZ | NZNNNZNNZ | NNNZZZNNZ |
| ZZZNZNZZZ | ZNZZZZNZZ | ZNNZNZNZZ | NZZNZNZZN | NZNNNZNNN | NNNZZZNNN |
| ZZZNZNZZN | ZNZZZZNZN | ZNNZNZNZN | NZZNZNZNZ | NZNNNNZZZ | NNNZZNZZZ |
| ZZZNZNZNZ | ZNZZZZNNZ | ZNNZNZNNZ | NZZNZNZNN | NZNNNNZZN | NNNZZNZZN |
| ZZZNZNZNN | ZNZZZZNNN | ZNNZNZNNN | NZZNZNNZZ | NZNNNNZNZ | NNNZZNZNZ |
| ZZZNZNNZZ | ZNZZZNZZZ | ZNNZNNZZZ | NZZNZNNZN | NZNNNNZNN | NNNZZNZNN |
| ZZZNZNNZN | ZNZZZNZZN | ZNNZNNZZN | NZZNZNNNZ | NZNNNNNZZ | NNNZZNNZZ |
| ZZZNZNNNZ | ZNZZZNZNZ | ZNNZNNZNZ | NZZNZNNNN | NZNNNNNZN | NNNZZNNZN |
| ZZZNZNNNN | ZNZZZNZNN | ZNNZNNZNN | NZZNNZZZZ | NZNNNNNNZ | NNNZZNNNZ |
| ZZZNNZZZN | ZNZZZNNZZ | ZNNZNNNZZ | NZZNNZZZN | NZNNNNNNN | NNNZZNNNN |
| ZZZNNZZNZ | ZNZZZNNZN | ZNNZNNNZN | NZZNNZZNZ | NNZZZZZZN | NNNZNZZZZ |
| ZZZNNZZNN | ZNZZZNNNZ | ZNNZNNNNZ | NZZNNZZNN | NNZZZZZNZ | NNNZNZZZN |
| ZZZNNZNZZ | ZNZZZNNNN | ZNNZNNNNN | NZZNNZNZZ | NNZZZZZNN | NNNZNZZNZ |
| ZZZNNZNZN | ZNZZNZZZZ | ZNNNZZZZN | NZZNNZNZN | NNZZZZNZZ | NNNZNZZNN |
| ZZZNNZNNZ | ZNZZNZZZN | ZNNNZZZNZ | NZZNNZNNZ | NNZZZZNZN | NNNZNZNZZ |
| ZZZNNZNNN | ZNZZNZZNZ | ZNNNZZZNN | NZZNNZNNN | NNZZZZNNZ | NNNZNZNZN |
| ZZZNNNZZN | ZNZZNZZNN | ZNNNZZNZZ | NZZNNNZZZ | NNZZZZNNN | NNNZNZNNZ |
| ZZZNNNZNZ | ZNZZNZNZZ | ZNNNZZNZN | NZZNNNZZN | NNZZZNZZZ | NNNZNZNNN |
| ZZZNNNZNN | ZNZZNZNZN | ZNNNZZNNZ | NZZNNNZNZ | NNZZZNZZN | NNNZNNZZZ |
| ZZZNNNNZN | ZNZZNZNNZ | ZNNNZZNNN | NZZNNNZNN | NNZZZNZNZ | NNNZNNZZN |
| ZZNZZZZZN | ZNZZNZNNN | ZNNNZNZZZ | NZZNNNNZZ | NNZZZNZNN | NNNZNNZNZ |
| ZZNZZZZNZ | ZNZZNNZZZ | ZNNNZNZZN | NZZNNNNZN | NNZZZNNZZ | NNNZNNZNN |
| ZZNZZZZNN | ZNZZNNZZN | ZNNNZNZNZ | NZZNNNNNZ | NNZZZNNZN | NNNZNNNZZ |
| ZZNZZZNZZ | ZNZZNNZNZ | ZNNNZNZNN | NZZNNNNNN | NNZZZNNNZ | NNNZNNNZN |
| ZZNZZZNZN | ZNZZNNZNN | ZNNNZNNZZ | NZNZZZZZZ | NNZZZNNNN | NNNZNNNNZ |
| ZZNZZZNNZ | ZNZZNNNZZ | ZNNNZNNZN | NZNZZZZZN | NNZZNZZZZ | NNNZNNNNN |
| ZZNZZZNNN | ZNZZNNNZN | ZNNNZNNNZ | NZNZZZZNZ | NNZZNZZZN | NNNNZZZZZ |
| ZZNZZNZZZ | ZNZZNNNNZ | ZNNNZNNNN | NZNZZZZNN | NNZZNZZNZ | NNNNZZZZN |
| ZZNZZNZZN | ZNZZNNNNN | ZNNNNZZZN | NZNZZZNZZ | NNZZNZZNN | NNNNZZZNZ |
| ZZNZZNZNZ | ZNZNZZZZZ | ZNNNNZZNZ | NZNZZZNZN | NNZZNZNZZ | NNNNZZZNN |
| ZZNZZNZNN | ZNZNZZZZN | ZNNNNZZNN | NZNZZZNNZ | NNZZNZNZN | NNNNZZNZZ |
| ZZNZZNNZZ | ZNZNZZZNZ | ZNNNNZNZZ | NZNZZZNNN | NNZZNZNNZ | NNNNZZNZN |
| ZZNZZNNZN | ZNZNZZZNN | ZNNNNZNZN | NZNZZNZZZ | NNZZNZNNN | NNNNZZNNZ |
| ZZNZZNNNZ | ZNZNZZNZZ | ZNNNNZNNZ | NZNZZNZZN | NNZZNNZZZ | NNNNZZNNN |
| ZZNZZNNNN | ZNZNZZNZN | ZNNNNZNNN | NZNZZNZNZ | NNZZNNZZN | NNNNZNZZZ |
| ZZNZNZZZZ | ZNZNZZNNZ | ZNNNNNZZN | NZNZZNZNN | NNZZNNZNZ | NNNNZNZZN |
| ZZNZNZZZN | ZNZNZZNNN | ZNNNNNZNZ | NZNZZNNZZ | NNZZNNZNN | NNNNZNZNZ |
| ZZNZNZZNZ | ZNZNZNZZZ | ZNNNNNZNN | NZNZZNNZN | NNZZNNNZZ | NNNNZNZNN |
| ZZNZNZZNN | ZNZNZNZZN | ZNNNNNNZN | NZNZZNNNZ | NNZZNNNZN | NNNNZNNZZ |

(continued)

| | | | | | |
|---|---|---|---|---|---|
| ZZNZNZNZZ | ZNZNZNZNZ | NZZZZZZZN | NZNZZNNNN | NNZZNNNNZ | NNNNZNNZN |
| ZZNZNZNZN | ZNZNZNZNN | NZZZZZZNZ | NZNZNZZZZ | NNZZNNNNN | NNNNZNNNZ |
| ZZNZNZNNZ | ZNZNZNNZZ | NZZZZZZNN | NZNZNZZZN | NNZNZZZZZ | NNNNZNNNN |
| ZZNZNZNNN | ZNZNZNNZN | NZZZZZNZZ | NZNZNZZNZ | NNZNZZZZN | NNNNNZZZZ |
| ZZNZNNZZZ | ZNZNZNNNZ | NZZZZZNZN | NZNZNZZNN | NNZNZZZNZ | NNNNNZZZN |
| ZZNZNNZZN | ZNZNZNNNN | NZZZZZNNZ | NZNZNZNZZ | NNZNZZZNN | NNNNNZZNZ |
| ZZNZNNZNZ | ZNZNNZZZZ | NZZZZZNNN | NZNZNZNZN | NNZNZZNZZ | NNNNNZZNN |
| ZZNZNNNZN | ZNZNNZZNN | NZZZZNZNZ | NZNZNNZZZ | NNZNZZNNN | NNNNNZNNZ |
| ZZNZNNNNZ | ZNZNNZNZZ | NZZZZNZNN | NZNZNNZZN | NNZNZNZZZ | NNNNNZNNN |
| ZZNZNNNNN | ZNZNNZNZN | NZZZZNNZZ | NZNZNNZNZ | NNZNZNZZN | NNNNNNZZZ |
| ZZNNZZZZN | ZNZNNZNNZ | NZZZZNNZN | NZNZNNZNN | NNZNZNZNZ | NNNNNNZZN |
| ZZNNZZZNZ | ZNZNNZNNN | NZZZZNNNZ | NZNZNNNZZ | NNZNZNZNN | NNNNNNZNZ |
| ZZNNZZZNN | ZNZNNNZZZ | NZZZZNNNN | NZNZNNNZN | NNZNZNNZZ | NNNNNNZNN |
| ZZNNZZNZZ | ZNZNNNZZZ | NZZZNZZZZ | NZNZNNNNZ | NNZNZNNZN | NNNNNNNZN |
| ZZNNZZNZN | ZNZNNNZNZ | | | | |

In another alternate embodiment, the plurality of oligonucleotides may comprise formula $N_mZ_q$, wherein N and Z are nucleotides as defined above, m ranges from 2 to 13, q ranges from 1 to 12, the sum total of m and q ranges from 6 to 14, the at least two N residues are separated by at least one Z residue, and there are no more than three consecutive N residues. In this embodiment, therefore, non-complementary 2-fold degenerate nucleotides are interspersed throughout the sequence such that there are no long runs (≥4) of the complementary 4-fold degenerate nucleotide (N). In general, such a design may reduce self-hybridization and/or cross-hybridization within the plurality of oligonucleotides. In an exemplary embodiment, the plurality of oligonucleotides may comprise formula $N_mZ_q$, wherein N and Z are nucleotides as defined above, m ranges from 2 to 8, q ranges from 1 to 7, the sum total of m and q is 9, the at least two N residues are separated by at least one Z residue, and there are no more than three consecutive N residues. Table F lists the (5' to 3') sequences of this preferred embodiment, i.e., a 9-nucleotide long semi-random region containing no more that three consecutive N residues.

**Table F.** Nucleotide sequences (5' to 3') of an exemplary semi-random region having no more than 3 consecutive N residues.

| | | | | | |
|---|---|---|---|---|---|
| ZZZZZZNZN | ZZNZNNZZZ | ZNZNZNZZZ | NZZZZZZNZ | NZNZZNZZN | NNZZNZNNZ |
| ZZZZZNZZN | ZZNZNNZZN | ZNZNZNZZN | NZZZZZZNN | NZNZZNZNZ | NNZZNZNNN |
| 77777NZNZ | ZZNZNNZNZ | ZNZNZNZNZ | N77777NZZ | NZNZZNZNN | NNZZNNZZZ |
| ZZZZZNZNN | ZZNZNNZNN | ZNZNZNZNN | NZZZZZNZN | NZNZZNNZZ | NNZZNNZZN |
| ZZZZZNNZN | ZZNZNNNZZ | ZNZNZNNZZ | NZZZZZNNZ | NZNZZNNZN | NNZZNNZNZ |
| ZZZZNZZZN | ZZNZNNZZN | ZNZNZNNZN | NZZZZZNNN | NZNZZNNNZ | NNZZNNZNN |
| ZZZZNZZNZ | ZZNNZZZZN | ZNZNZNNNZ | NZZZZNZZZ | NZNZNZZZZ | NNZZNNNZZ |
| ZZZZNZZNN | ZZNNZZZNZ | ZNZNNZZZZ | NZZZZNZZN | NZNZNZZZN | NNZZNNNZN |
| ZZZZNZNZZ | ZZNNZZZNN | ZNZNNZZZN | NZZZZNZNZ | NZNZNZZNZ | NNZNZZZZZ |
| ZZZZNZNZN | ZZNNZZNZZ | ZNZNNZZNZ | NZZZZNZNN | NZNZNZZNN | NNZNZZZZN |
| ZZZZNZNNZ | ZZNNZZNZN | ZNZNNZZNN | NZZZZNNZZ | NZNZNZNZZ | NNZNZZZNZ |
| ZZZZNZNNN | ZZNNZZNNZ | ZNZNNZNZZ | NZZZZNNZN | NZNZNZNZN | NNZNZZZNN |
| ZZZZNNZZN | ZZNNZZNNN | ZNZNNZNZN | NZZZZNNNZ | NZNZNZNNZ | NNZNZZNZZ |

(continued)

| | | | | | |
|---|---|---|---|---|---|
| ZZZZNNZNZ | ZZNNZNZZZ | ZNZNNZNNZ | NZZZNZZZZ | NZNZNZNNN | NNZNZZNZN |
| ZZZZNNZNN | ZZNNZNZZN | ZNZNNZNNN | NZZZNZZZN | NZNZNNZZZ | NNZNZZNNZ |
| ZZZZNNNZN | ZZNNZNZNZ | ZNZNNNZZZ | NZZZNZZNZ | NZNZNNZZN | NNZNZZNNN |
| ZZZNZZZZZ | ZZNNZNZNN | ZNZNNNZZN | NZZZNZZNN | NZNZNNZNZ | NNZNZNZZZ |
| ZZZNZZZZN | ZZNNZNNZZ | ZNZNNNZNZ | NZZZNZNZZ | NZNZNNZNN | NNZNZNZZN |
| ZZZNZZZNZ | ZZNNZNNZN | ZNZNNNZNN | NZZZNZNZN | NZNZNNNZZ | NNZNZNZNZ |
| ZZZNZZZNN | ZZNNZNNNZ | ZNNZZZZZN | NZZZNZNNZ | NZNZNNNZN | NNZNZNZNN |
| ZZZNZZNZZ | ZZNNNZZZN | ZNNZZZZNZ | NZZZNZNNN | NZNNZZZZZ | NNZNZNNZZ |
| ZZZNZZNZN | ZZNNNZZNZ | ZNNZZZZNN | NZZZNNZZZ | NZNNZZZZN | NNZNZNNZN |
| ZZZNZZNNZ | ZZNNNZZNN | ZNNZZZNZZ | NZZZNNZZN | NZNNZZZNZ | NNZNZNNNZ |
| ZZZNZZNNN | ZZNNNZNZZ | ZNNZZZNZN | NZZZNNZNZ | NZNNZZZNN | NNZNNZZZZ |
| ZZZNZNZZZ | ZZNNNZNZN | ZNNZZZNNZ | NZZZNNZNN | NZNNZZNZZ | NNZNNZZZN |
| ZZZNZNZZN | ZZNNNZNNZ | ZNNZZZNNN | NZZZNNNZZ | NZNNZZNZN | NNZNNZZNZ |
| ZZZNZNZNZ | ZZNNNZNNN | ZNNZZNZZZ | NZZZNNNZN | NZNNZZNNZ | NNZNNZZNN |
| ZZZNZNZNN | ZNZZZZZZN | ZNNZZNZZN | NZZNZZZZZ | NZNNZZNNN | NNZNNZNZZ |
| ZZZNZNNZZ | ZNZZZZZNZ | ZNNZZNZNZ | NZZNZZZZN | NZNNZNZZZ | NNZNNZNZN |
| ZZZNZNNZN | ZNZZZZZNN | ZNNZZNZNN | NZZNZZZNZ | NZNNZNZZN | NNZNNZNNZ |
| ZZZNZNNNZ | ZNZZZZNZZ | ZNNZZNNZZ | NZZNZZZNN | NZNNZNZNZ | NNZNNZNNN |
| ZZZNNZZZN | ZNZZZZNZN | ZNNZZNNZN | NZZNZZNZZ | NZNNZNZNN | NNZNNNZZZ |
| ZZZNNZZNZ | ZNZZZZNNZ | ZNNZZNNNZ | NZZNZZNZN | NZNNZNNZZ | NNZNNNZZN |
| ZZZNNZZNN | ZNZZZZNNN | ZNNZNZZZZ | NZZNZZNNZ | NZNNZNNZN | NNZNNNZNZ |
| ZZZNNZNZZ | ZNZZZNZZZ | ZNNZNZZZN | NZZNZZNNN | NZNNZNNNZ | NNZNNNZNN |
| ZZZNNZNZN | ZNZZZNZZN | ZNNZNZZNZ | NZZNZNZZZ | NZNNNZZZZ | NNNZZZZZN |
| ZZZNNZNNZ | ZNZZZNZNZ | ZNNZNZZNN | NZZNZNZZN | NZNNNZZZN | NNNZZZZNZ |
| ZZZNNZNNN | ZNZZZNZNN | ZNNZNZNZZ | NZZNZNZNZ | NZNNNZZNZ | NNNZZZZNN |
| ZZZNNNZZN | ZNZZZNNZZ | ZNNZNZNZN | NZZNZNZNN | NZNNNZZNN | NNNZZZNZZ |
| ZZZNNNZNZ | ZNZZZNNZN | ZNNZNZNNZ | NZZNZNNZZ | NZNNNZNZZ | NNNZZZNZN |
| ZZZNNNZNN | ZNZZZNNNZ | ZNNZNZNNN | NZZNZNNZN | NZNNNZNZN | NNNZZZNNZ |
| ZZNZZZZZN | ZNZZNZZZZ | ZNNZNNZZZ | NZZNZNNNZ | NZNNNZNNZ | NNNZZZNNN |
| ZZNZZZZNZ | ZNZZNZZZN | ZNNZNNZZN | NZZNNZZZZ | NZNNNZNNN | NNNZZNZZZ |
| ZZNZZZZNN | ZNZZNZZNZ | ZNNZNNZNZ | NZZNNZZZN | NNZZZZZZN | NNNZZNZZN |
| ZZNZZZNZZ | ZNZZNZZNN | ZNNZNNZNN | NZZNNZZNZ | NNZZZZZNZ | NNNZZNZNZ |
| ZZNZZZNZN | ZNZZNZNZZ | ZNNZNNNZZ | NZZNNZZNN | NN77777NN | NNNZZNZNN |
| ZZNZZZNNZ | ZNZZNZNZN | ZNNZNNNZN | NZZNNZNZZ | NNZZZZNZZ | NNNZZNNZZ |
| ZZNZZZNNN | ZNZZNZNNZ | ZNNNZZZZN | NZZNNZNZN | NNZZZZNZN | NNNZZNNZN |
| ZZNZZNZZZ | ZNZZNZNNN | ZNNNZZZNZ | NZZNNZNNZ | NNZZZZNNZ | NNNZZNNNZ |
| ZZNZZNZZN | ZNZZNNZZZ | ZNNNZZZNN | NZZNNZNNN | NNZZZZNNN | NNNZNZZZZ |
| ZZNZZNZNZ | ZNZZNNZZN | ZNNNZZNZZ | NZZNNNZZZ | NNZZZNZZZ | NNNZNZZZN |
| ZZNZZNZNN | ZNZZNNZNZ | ZNNNZZNZN | NZZNNNZZN | NNZZZNZZN | NNNZNZZNZ |

(continued)

| | | | | | |
|---|---|---|---|---|---|
| ZZNZZNNZZ | ZNZZNNZNN | ZNNNZZNNZ | NZZNNNZNZ | NNZZZNZNZ | NNNZNZZNN |
| ZZNZZNNZN | ZNZZNNNZZ | ZNNNZZNNN | NZZNNNZNN | NNZZZNZNN | NNNZNZNZZ |
| ZZNZZNNNZ | ZNZZNNNZN | ZNNNZNZZZ | NZNZZZZZZ | NNZZZNNZZ | NNNZNZNZN |
| ZZNZNZZZZ | ZNZNZZZZZ | ZNNNZNZZN | NZNZZZZZN | NNZZZNNZN | NNNZNZNNZ |
| ZZNZNZZZN | ZNZNZZZZN | ZNNNZNZNZ | NZNZZZZNZ | NNZZZNNNZ | NNNZNZNNN |
| ZZNZNZZNZ | ZNZNZZZNZ | ZNNNZNZNN | NZNZZZZNN | NNZZNZZZZ | NNNZNNZZZ |
| ZZNZNZZNN | ZNZNZZZNN | ZNNNZNNZZ | NZNZZZNZZ | NNZZNZZZN | NNNZNNZZN |
| ZZNZNZNZZ | ZNZNZZNZZ | ZNNNZNNZN | NZNZZZNZN | NNZZNZZNZ | NNNZNNZNZ |
| ZZNZNZNZN | ZNZNZZNZN | ZNNNZNNNZ | NZNZZZNNZ | NNZZNZZNN | NNNZNNZNN |
| ZZNZNZNNZ | ZNZNZZNNZ | ZNNNZNNNN | NZNZZZNNN | NNZZNZNZZ | NNNZNNNZZ |
| ZZNZNZNNN | ZNZNZZNNN | NZZZZZZZN | NZNZZNZZZ | NNZZNZNZN | NNNZNNNZN |

In another alternate embodiment, the plurality of oligonucleotides may comprise the formula $X_pZ_q$, wherein X and Z are nucleotides as defined above, p and q range from 1 to 13, and the sum total of p and q is 14. In another embodiment, the plurality of oligonucleotides may comprise the formula $X_pZ_q$, wherein X and Z are nucleotides as defined above, p and q range from 1 to 12, and the sum total of p and q is 13. In yet another embodiment, the plurality of oligonucleotides may comprise the formula $X_pZ_q$, wherein X and Z are nucleotides as defined above, p and q range from 1 to 11, and the sum total of p and q is 12. In still another embodiment, the plurality of oligonucleotides may comprise the formula $X_pZ_q$, wherein X and Z are nucleotides as defined above, p and q range from 1 to 10, and the sum total of p and q is 11. In another embodiment, the plurality of oligonucleotides may comprise the formula $X_pZ_q$, wherein X and Z are nucleotides as defined above, p and q range from 1 to 9, and the sum total of p and q is 10. In still another alternate embodiment, the plurality of oligonucleotides may comprise the formula $X_pZ_q$, wherein X and Z are nucleotides as defined above, p and q range from 1 to 8, and the sum total of p and q is 9. In still another embodiment, the plurality of oligonucleotides may comprise the formula $X_pZ_q$, wherein X and Z are nucleotides as defined above, p and q range from 1 to 7, and the sum total of p and q is 8. In yet another embodiment, the plurality of oligonucleotides may comprise the formula $X_pZ_q$, wherein X and Z are nucleotides as defined above, p and q range from 1 to 6, and the sum total of p and q is 7. In a further embodiment, the plurality of oligonucleotides may comprise the formula $X_pZ_q$, wherein X and Z are nucleotides as defined above, p and q range from 1 to 5, and the sum total of p and q is 6.

In still other embodiments, in which both m and q are 0, the plurality of oligonucleotides comprises the formula $X_p$, wherein X is a 3-fold degenerate nucleotide and p is an integer from 4 to 20. The plurality of oligonucleotides having these formulas may range from about 8 nucleotides to about 14 nucleotides in length, or from about 14 nucleotides to about 20 nucleotides in length. In a preferred embodiment, the plurality of oligonucleotides may be about 9 nucleotides in length.

**(b) optional non-random sequence**

The oligonucleotides described above may further comprise a non- random sequence comprising standard (non- degenerate) nucleotides. The non- random sequence is located at the 5' end of each oligonucleotide. In general, the sequence of non- degenerate nucleotides is constant among the oligonucleotides of a plurality. The constant non-degenerate sequence typically comprises a known sequence, such as a universal priming site. Non- limiting examples of suitable universal priming sites include T7 promoter sequence, T3 promoter sequence, SP6 promoter sequence, M13 forward sequence, or M13 reverse sequence. Alternatively the constant non- degenerate sequence may comprise essentially any artificial sequence that is not present in the nucleic acid that is to be amplified. In one embodiment, the constant non- degenerate sequence may comprise the sequence 5'- GTAGGTTGAGGATAGGAGGGTTAGG- 3' (SEQ ID NO: 3) . In another embodiment, the constant non- degenerate sequence may comprise the sequence 5'- GTGGTGTGTTGGGTGTGTTTGG- 3' (SEQ ID NO: 28) .

The constant non-degenerate sequence may range from about 6 nucleotides to about 100 nucleotides in length. In one embodiment, the constant, non-degenerate sequence may range from about 10 nucleotides to about 40 nucleotides in length. In another embodiment, the constant non-degenerate sequence may range from about 14 nucleotides to about 30 nucleotides in length. In yet another embodiment, the constant non-degenerate sequence may range from about 18 nucleotides to about 26 nucleotides in length. In still another embodiment, the constant non-degenerate sequence may range from about 22 nucleotides to about 25 nucleotides in length.

In some embodiments, additional nucleotides may be added to the 5' end of the constant non-degenerate sequence of each oligonucleotide of the plurality. For example, nucleotides may be added to increase the melting temperature of the plurality of oligonucleotides. The additional nucleotides may comprise G residues, C residues, or a combination thereof. The number of additional nucleotides may range from about 1 nucleotide to about 10 nucleotides, preferably from about 3 nucleotides to about 6 nucleotides, and more preferably about 4 nucleotides.

***(II) Method for Amplifying a Population of Target Nucleic Acids***

Another aspect of the invention provides a method for amplifying a population of target nucleic acids by creating a library of amplifiable molecules, which then may be further amplified. The library of amplifiable molecules is generated in a sequence independent manner by using the plurality of degenerate oligonucleotide primers of the invention to provide a plurality of replication initiation sites throughout the target nucleic acid. The semi-random sequence of the degenerate oligonucleotide primers minimizes intramolecular and intermolecular interactions among the plurality of oligonucleotide primers while still providing sequence diversity, thereby facilitating replication of the entire target nucleic acid. Thus, the target nucleic acid may be amplified without compromising the representation of any given sequence and without significant bias (i.e., 3' end bias). The amplified target nucleic acid may be a whole genome or a whole transcriptome.

**(a) creating a library**

A library of amplifiable molecules representative of the population of target nucleic acids may be generated by contacting the target nucleic acids with a plurality of degenerate oligonucleotide primers of the invention. The degenerate oligonucleotide primers hybridize at random sites scattered somewhat equally throughout the target nucleic acid to provide a plurality of priming sites for replication of the target nucleic acid. The target nucleic acid may be replicated by an enzyme with strand-displacing activity, such that replicated strands are displaced during replication and serve as templates for additional rounds of replication. Alternatively, the target nucleic acid may be replicated via a two-step process, i.e., first strand cDNA is synthesized with a reverse transcriptase and second strand cDNA is synthesized with an enzyme without strand-displacing activity. As a consequence of either method, the amount of replicated strands exceeds the amount of starting target nucleic acids, indicating amplification of the target nucleic acid.

*(i) target nucleic acid*

The population of target nucleic acids can and will vary. In one embodiment, the population of target nucleic acids may be genomic DNA. Genomic DNA refers to one or more chromosomal DNA molecules occurring naturally in the nucleus or an organelle (e.g., mitochondrion, chloroplast, or kinetoplast) of a eukaryotic cell, a eubacterial cell, an archaeal cell, or a virus. These molecules contain sequences that are transcribed into RNA, as well as sequences that are not transcribed into RNA. As such, genomic DNA may comprise the whole genome of an organism or it may comprise a portion of the genome, such as a single chromosome or a fragment thereof.

In another embodiment, the population of target nucleic acids may be a population of RNA molecules. The RNA molecules may be messenger RNA molecules or small RNA molecules. The population of RNA molecules may comprise a transcriptome, which is defined as the set of all RNA molecules expressed in one cell or a population of cells. The set of RNA molecules may include messenger RNAs and/or microRNAs and other small RNAs. The term, transcriptome, may refer to the total set of RNA molecules in a given organism or the specific subset of RNA molecules present in a particular cell type.

The population of target nucleic acids may be derived from eukaryotes, eubacteria, archaea, or viruses. Non-limiting examples of suitable eukaryotes include humans, mice, mammals, vertebrates, invertebrates, plants, fungi, yeast, and protozoa. In a preferred embodiment, the population of nucleic acids is derived from a human. Non-limiting sources of target nucleic acids include a genomic DNA preparation, a total RNA preparation, a poly(A)$^+$ RNA preparation, a poly(A)$^-$ RNA preparation, a small RNA preparation, a single cell, a cell lysate, cultured cells, a tissue sample, a fixed tissue, a frozen tissue, an embedded tissue, a biopsied tissue, a tissue swab, or a biological fluid. Suitable body fluids include, but are not limited to, whole blood, buffy coats, serum, saliva, cerebrospinal fluid, pleural fluid, lymphatic fluid, milk, sputum, semen, and urine.

In some embodiments, the target nucleic acid may be randomly fragmented prior to contact with the plurality of oligonucleotide primers. The target nucleic acid may be randomly fragmented by mechanical means, such as physically shearing the nucleic acid by passing it through a narrow capillary or orifice, sonicating the nucleic acid, and/or-nebulizing the nucleic acid. Alternatively, the nucleic acid may be randomly fragmented by chemical means, such as acid hydrolysis, alkaline hydrolysis, formalin fixation, hydrolysis by metal complexes (e.g., porphyrins), and/or hydrolysis by hydroxyl radicals. The target nucleic acid may also be randomly fragmented by thermal means, such as heating the nucleic acid in a solution of low ionic strength and neutral pH. The temperature may range from about 90°C to about 100°C, and preferably about 95°C. The solution of low ionic strength may comprise from about 10 mM to about 20 mM of Tris-HCl and from about 0.1 mM to about 1 mM of EDTA, with a pH of about 7.5 to about 8.5. The duration of the heating period may range from about 1 minute to about 10 minutes. Alternatively, the nucleic acid may be fragmented by enzymatic means, such as partial digestion with DNase I or an RNase. Alternatively, DNA may be fragmented by digestion with a restriction endonuclease that recognizes multiple tetra-nucleotide recognition sequences (e.g., *Cvi*JI) in the presence of a divalent cation. Depending upon the method used to fragment the nucleic acid, the size of the fragments may range from about 100 base pairs to about 5000 base pairs, or from about 50 nucleotides to about 2500 nucleotides.

The amount of nucleic acid available as target can and will vary depending upon the type and quality of the nucleic acid. In general, the amount of target nucleic acid may range from about 0.1 picograms (pg) to about 1,000 nanograms (ng). In embodiments in which the target nucleic acid is genomic DNA, the amount of target DNA may be about 1 ng for simple genomes such as those from bacteria, about 10 ng for a complex genome such as that of human, about 5 pg for a single human cell, or about 200 ng for partially degraded DNA extracted from fixed tissue. In embodiments in which the target nucleic acid is high quality total RNA, the amount of target RNA may range from about 0.1 pg to about 50 ng, or more preferably from about 10 pg to about 500 pg. In other embodiments in which the target nucleic acid is partially degraded total RNA, the amount of target RNA may range from about 25 ng to about 1,000 ng. For embodiments in which the target nucleic acid is RNA from a single cell, one skilled in the art will appreciate that the amount of RNA in a cell varies among different cell types.

*(ii) Plurality of oligonucleotide primers*

The plurality of oligonucleotide primers that is contacted with the target nucleic acid was described above in section (I) (a) . The oligonucleotide primers comprise a semi- random region comprising a mixture of fully (i.e., 4- fold) degenerate and partially (i.e., 3- fold and/or 2- fold) degenerate nucleotides. The partially degenerate nucleotides are dispersed among the fully degenerate nucleotides such at least one 2- fold or 3- fold degenerate nucleotide separates the at least two 4- fold degenerate nucleotides. The presence of non- complementary 2- fold degenerate nucleotides and/or partially non- complementary 3- fold degenerate nucleotides reduces the ability of the oligonucleotide primers comprising fully degenerate nucleotides to self- hybridize and/or cross- hybridize (and form primer- dimers), while still providing high sequence diversity.

In a preferred embodiment, the plurality of oligonucleotide primers used in the method of the invention comprise the formula $N_mX_p$, $N_mZ_q$, or a combination thereof, wherein N, X, and Z are degenerate nucleotides as defined above, m is from 2 to 13, p and q are each from 1 to 12, and the sum total of the two integers is from 6 to 14, and the at least two N residues are separated by at least one X or Z residue. In another preferred embodiment, the plurality of oligonucleotide primers used in the method comprise the formula $N_m$, $X_p$, $N_mZ_q$, or a combination thereof, wherein N, X, and Z are degenerate nucleotides as defined above, m is an integer from 2 to 8, p and q are integers from 1 to 7, the sum total of the two integers is 9, the at least two N residues are separated by at least one X or Z residue, and there are no more than three consecutive N residues (see Tables D and F) . In preferred embodiments, X is D and Y is K. In an especially preferred embodiment, the plurality of oligonucleotide primers used in the method of the invention have the following (5'- 3') sequences: KNNNKNKNK, NKNNKNNKK, and NNNKNKKNK. The preferred oligonucleotide primers may further comprise a constant non- degenerate sequence at the 5' end of each oligonucleotide, as described above in section (I) (b) .

The plurality of oligonucleotide primers contacted with the target nucleic acid may have a single sequence. For example, the (5'- 3') sequence of the plurality of degenerate oligonucleotide primers may be XNNNXNXNX. The degeneracy of this oligonucleotide primer may be calculated using the formula presented above (i.e., degeneracy = 82, 944 = $3^4$ x $4^5$) . Alternatively, the plurality of oligonucleotide primers contacted with the target nucleic acid may be a mixture of degenerate oligonucleotide primers having different sequences. The mixture may comprise two degenerate oligonucleotide primers, three degenerate oligonucleotide primers, four degenerate oligonucleotide primers, etc. As an example, the mixture may comprise three degenerate oligonucleotide primers having the following (5'- 3') sequences: XNNNXNXNX, NNNXNXXNX, XXXNNXXNX. In this example, the degeneracy of the mixture of oligonucleotide primers is 212, 544 [= ($3^4$ x $4^5$) + ($3^4$ x $4^5$) + ($3^6$ x $4^3$) ] . The mixture may comprise degenerate oligonucleotide primers comprising 3- fold degenerate nucleotides and/or 2- fold degenerate nucleotides (i.e., formulas $N_m$Xp and/or $N_mZ_q$) .

Because of the large number of sequences represented in the plurality of degenerate oligonucleotide primers of the invention, a subset of oligonucleotide primers will generally have many complementary sequences dispersed throughout the population of target nucleic acids. Accordingly, the subset of complementary oligonucleotide primers will hybridize with the target nucleic acid, thereby forming a plurality of nucleic acid-primer duplexes and providing a plurality of priming sites for nucleic acid replication.

In some embodiments, in addition to the plurality of oligonucleotide primers, an oligo dT or anchor oligo dT primer may also be contacted with the population of target nucleic acids. The anchor oligo dT primer may comprise (5' to 3') a string of deoxythymidylic acid (dT) residues followed by two additional ribonucleotides represented by VN, wherein V is either G, C, or A and N is either G, C, A, or U. The VN ribonucleotide anchor allows the primer to hybridize only at the 5' end of the poly(A) tail of a target messenger RNA, such that the messenger RNA may be reverse transcribed into cDNA. One skilled in the art will appreciate that an oligo dT primer may comprise other nucleotides and/or other features.

*(iii) replicating the target nucleic acid*

The primed target nucleic acid may be replicated by an enzyme with strand-displacing activity. Examples of suitable strand-displacement polymerases include, but are not limited to, Exo-Minus Klenow DNA polymerase (i.e., large fragment of DNA Pol I that lacks both 5'→3' and 3'→5' exonuclease activities), Exo-Minus T7 DNA polymerase (i.e., SEQUENASE™ Version 2.0, USB Corp., Cleveland, OH), Phi29 DNA polymerase, Bst DNA polymerase, Bca polymerase, Vent DNA polymerase, 9°Nm DNA polymerase, MMLV reverse transcriptase, AMV reverse transcriptase, HIV reverse transcriptase, variants thereof, or combinations thereof. In one embodiment, the strand-displacing polymerase may be Exo-Minus Klenow DNA polymerase. In another embodiment, the strand-displacing polymerase may be MMLV reverse transcriptase. In yet another embodiment, the strand-displacing polymerase may comprise both MMLV reverse transcriptase and Exo-Minus Klenow DNA polymerase.

Alternatively, the primed target nucleic acid may be replicated via a two- step process. That is, the first strand of cDNA may be synthesized by a reverse transcriptase and then the second strand of cDNA may be synthesized by an enzyme without strand- displacing activity, such as Taq DNA polymerase.

The strand-displacing or replicating enzyme is incubated with the target nucleic acid and the plurality of degenerate oligonucleotide primers under conditions that permit hybridization between complementary sequences, as well as extension of the hybridized primer, i.e., replication of the nucleic acid. The incubation conditions are generally selected to allow hybridization between complementary sequences, but preclude hybridization between mismatched sequences (i.e., those with no or limited complementarity). The incubation conditions are also selected to optimize primer extension and promote strand-displacing activity. During replication, displaced single strands are generated that become new templates for oligonucleotide primer hybridization and primer extension. Thus, the incubation conditions generally comprise a solution of optimal pH, ionic strength, and $Mg^{2+}$ ion concentration, with incubation at a temperature that permits both hybridization and replication.

The library synthesis buffer generally comprises a pH modifying or buffering agent that is operative at a pH of about 6.5 to about 9.5, and preferably at a pH of about 7.5. Representative examples of suitable pH modifying agents include Tris buffers, MOPS, HEPES, Bicine, Tricine, TES, or PIPES. The library synthesis buffer may comprise a monovalent salt such as NaCl, at a concentration that ranges from about 1 mM to about 200 mM. The concentration of $MgCl_2$ in the library synthesis buffer may range from about 5 mM to about 10 mM. The requisite mixture of deoxynucleotide triphosphates (i.e., dNTPs) may be provided in the library synthesis buffer, or it may be provided separately. The incubation temperature may range from about 12°C to about 70°C, depending upon the polymerase used. The duration of the incubation may range from about 5 minutes to about 4 hours. In one embodiment, the incubation may comprise a single isothermal step, e.g., at about 30°C for about 1 hour. In another embodiment, the incubation may be performed by cycling through several temperature steps (e.g., 16°C, 24°C, and 37°C) for a short period of time (e.g., about 1-2 minutes) for a certain number of cycles (e.g., about 15-20 cycles). In yet another embodiment, the incubation may comprise sequential isothermal steps lasting from about 10 to 30 minutes. As an example, the incubation may comprise steps of 18°C for 10 minutes, 25°C for 10 minutes, 37°C for 30 minutes, and 42°C for 10 minutes. The reaction buffer may further comprise a factor that promotes stand-displacement, such as a single-stranded DNA binding protein (SSB) or a helicase. The SSB or helicase may be of bacterial, viral, or eukaryotic origin. The replication reaction may be terminated by adding a sufficient amount of EDTA to chelate the $Mg^{2+}$ ions and/or by heat-inactivating the enzyme.

Replication of the randomly-primed target nucleic acid by a strand-displacing enzyme creates a library of overlapping molecules that range from about 100 base pairs to about 2000 base pairs in length, with an average length of about 400 to about 500 base pairs. In some embodiments, the library of replicated strands may be flanked by a constant non-degenerate end sequence that corresponds to the constant non-degenerate sequence of the plurality of oligonucleotide primers.

**(b) amplifying the library**

The method may further comprise the step of amplifying the library through a polymerase chain reaction (PCR) process. In some embodiments, the library of replicated strands may be flanked by a constant non-degenerate end sequence, as described above. In other embodiments, at least one adaptor may be ligated to each end of the replicated strands of the library, such that the library of molecules is amplifiable. The adaptor may comprise a universal priming sequence, as described above, or a homopolymeric sequence, such as poly-G or poly-C. Suitable ligase enzymes and ligation techniques are well known in the art.

In some embodiments, PCR may be performed using a single amplification primer that is complementary to the constant end sequence of the library molecules. In other embodiments, PCR may be performed using a pair of amplification primers. In all embodiments, a thermostable DNA polymerase catalyzes the PCR amplification process. Non-limiting examples of suitable thermostable DNA polymerases include Taq DNA polymerase, Pfu DNA polymerase, Tli (also known as Vent) DNA polymerase, Tfl DNA polymerase, Tth DNA polymerase, variants thereof, and combinations thereof. The PCR process may comprise 3 steps (i.e., denaturation, annealing, and extension) or 2 steps (i.e., denaturation and annealing/extension). The temperature of the annealing or annealing/extension step can and will vary, depending upon the amplification primer. That is, its nucleotide sequence, melting temperature, and/or concentration. The temperature of the annealing or annealing/extending step may range from about 50°C to about 75°C. In a preferred embodiment, the temperature of the annealing or annealing/extending step may be about 70°C. The duration of the PCR steps may also vary. The duration of the denaturation step may range from about 10 seconds to about 2 minutes, and the duration of the annealing or annealing/extending step may be range from about 15 seconds to about 10 minutes. The total number of cycles may also vary, depending upon the quantity and quality of the target nucleic acid. The number of cycles may range from about 5 cycles to about 50 cycles, from about 10 cycles to about 30 cycles, and more preferably from about 14 cycles to about 20 cycles.

PCR amplification of the library will generally be performed in the presence of a suitable amplification buffer. The library amplification buffer may comprise a pH modifying agent, a divalent cation, a monovalent cation, and a stabilizing agent, such as a detergent or BSA. Suitable pH modifying agents include those known in the art that will maintain the pH of the reaction from about 8.0 to about 9.5. Suitable divalent cations include magnesium and/or manganese, and suitable monovalent cations include potassium, sodium, and/or lithium. Detergents that may be included include poly (ethylene glycol) 4- nonphenyl 3- sulfopropyl ether potassium salt, 3- [(3- cholamidopropyl) dimethylammonio]- 1- propanesulfonate, 3- [(3- cholamidopropyl) dimethylarnmonio]- 2- hydroxy- 1- propanesulfonate, Tween 20, and Nonidet NP40. Other agents that may be included in the amplification buffer include glycerol and/or polyethylene glycol. The amplification buffer may also comprise the requisite mixture of dNTPs. In some embodiments, the PCR amplification may be performed in the presence of modified nucleotide such that the amplified library is labeled for downstream analyses. Non- limiting examples of suitable modified nucleotides include fluorescently labeled nucleotides, aminoallyl-dUTP, bromo- dUTP, or digoxigenin- labeled nucleotide triphosphates.

The percentage of target nucleic acid that is represented in the amplified library can and will vary, depending upon the type and quality of the target nucleic acid. The amplified library may represent at least about 50%, about 60%, about 70%, about 80%, about 85%, about 90%, about 95%, about 97%, about 99%, or about 99.5% of the target nucleic acid. The fold of amplification may also vary, depending upon the target nucleic acid. The fold of amplification may be about 100- fold, 300- fold, about 1000- fold, about 10, 000- fold, about 100, 000- fold, or about 1, 000, 000- fold. For example, about 5 ng to about 10 ng of a target nucleic acid may be amplified into about 5 $\mu$g to about 50 $\mu$g of amplified library molecules. Furthermore, the amplified library may be re- amplified by PCR.

The amplified library may be purified to remove residual amplification primers and nucleotides prior to subsequent uses. Methods of nucleic acid purification, such as spin column chromatography or filtration techniques, are well known in the art.

The downstream use of the amplified library may vary. Non-limiting uses of the amplified library include quantitative real-time PCR, microarray analysis, sequencing, restriction fragment length polymorphism (RFLP) analysis, single nucleotide polymorphism (SNP) analysis, microsatellite analysis, short tandem repeat (STR) analysis, comparative genomic hybridization (CGH), fluorescent in situ hybridization (FISH), and chromatin immunoprecipitation (ChiP).

### *(III) Kit for Amplifying a Population of Target Nucleic Acids*

A further aspect of the invention encompasses a kit for amplifying a population of target nucleic acids. The kit comprises a plurality of oligonucleotide primers, as defined above in section (I), and a replicating enzyme, as defined above in section (II) (a) (iii) .

In a preferred embodiment, the plurality of oligonucleotide primers of the kit may comprise the formula $N_mXp$, $N_mZ_q$, or a combination thereof, wherein N, X, and Z are degenerate nucleotides as defined above, m is from 2 to 13, p and q are each from 1 to 11, and the sum total of the two integers is from 6 to 14, and the at least two N residues are separated by at least one X or Z residue. In an exemplary embodiment, the plurality of oligonucleotide primers of the kit comprise the formula $N_mX_p$, $N_mZ_q$, or a combination thereof, wherein N, X, and Z are degenerate nucleotides as defined above, m is from 2 to 8, p and q are each from 1 to 7, the sum total of m and p or m and q is 9, the at least two N residues are separated by at least one X or Z residue, and there are no more than three consecutive N residues. In preferred embodiments, X is D and Y is K. In an especially preferred embodiment, the plurality of oligonucleotide primers of the kit have the following (5'- 3') sequences: KNNNKNKNK, NKNNKNNKK, and NNNKNKKNK. In some embodiments, the plurality of oligonucleotide primers may further comprise an oligo dT primer. The plurality of oligonucleotide primers of the kit may also further comprise a constant non- degenerate sequence at the 5' end of each primer, as described above in section (I) (b) .

The kit may further comprise a library synthesis buffer, as defined in section (II) (a) (iii) . Another optional component of the kit is means to fragment a target nucleic acid, as described above in section (II) (a) (i) . The kit may also further comprise a thermostable DNA polymerase, at least one amplification primer, and a library amplification buffer, as described in section (II) (b) .

## DEFINITIONS

To facilitate understanding of the invention, a number of terms are defined below.

The terms "complementary or complementarity," as used herein, refer to the ability to form at least one Watson-Crick base pair through specific hydrogen bonds. The terms "non-complementary or non-complementarity" refer to the inability to form at least one Watson-Crick base pair through specific hydrogen bonds.

"Genomic DNA" refers to one or more chromosomal polymeric deoxyribonucleic acid molecules occurring naturally in the nucleus or an organelle (e.g., mitochondrion, chloroplast, or kinetoplast) of a eukaryotic cell, a eubacterial cell, an archaeal cell, or a virus. These molecules contain sequences that are transcribed into RNA, as well as sequences that are not transcribed into RNA.

The term "hybridization," as used herein, refers to the process of hydrogen bonding, or base pairing, between the bases comprising two complementary single-stranded nucleic acid molecules to form a double-stranded hybrid. The "stringency" of hybridization is typically determined by the conditions of temperature and ionic strength. Nucleic acid hybrid stability is generally expressed as the melting temperature or $T_m$, which is the temperature at which the hybrid is 50% denatured under defined conditions. Equations have been derived to estimate the $T_m$ of a given hybrid; the equations take into account the G+C content of the nucleic acid, the nature of the hybrid (e.g., DNA:DNA, DNA:RNA, etc.), the length of the nucleic acid probe, etc. (e.g., Sambrook et al. (1989) Molecular Cloning: A Laboratory Manual, Cold Spring Harbor Press, Cold Spring Harbor, NY, chapter 9). In many reactions that are based upon hybridization, e.g., polymerase reactions, amplification reactions, ligation reactions, etc., the temperature of the reaction typically determines the stringency of the hybridization.

The term "primer," as generally used, refers to a nucleic acid strand or an oligonucleotide having a free 3' hydroxyl group that serves as a starting point for DNA replication.

The term "transcriptome," as used herein, is defined as the set of all RNA molecules expressed in one cell or a population of cells. The set of RNA molecules may include messenger RNAs and/or microRNAs and other small RNAs. The term may refer to the total set of RNA molecules in a given organism, or to the specific subset of RNA molecules present in a particular cell type.

## EXAMPLES

The following examples are included to demonstrate various embodiments of the invention. It should be appreciated by those of skill in the art that the techniques disclosed in the examples that follow represent techniques discovered by the inventors to function well in the practice of the invention and shall be interpreted as illustrative and not in a limiting sense.

### Example 1. Analysis of a D9 Library Synthesis Primer.

In an attempt to increase the degeneracy of primers used in WGA and WTA applications, a library synthesis primer was synthesized whose semi-random region comprised nine D residues (D9). The primer also comprised a constant (universal) 5' region. The ability of this primer to efficiently amplify a large number of amplicons was compared to that of a standard library synthesis primer whose semi-random region comprised nine K residues (K9) (e.g., that provided in the Rubicon TRANSPLEX™ Whole Transcriptome Amplification (WTA) Kit, Sigma-Aldrich, St. Louis, MO). Both K9 and D9 amplified cDNAs were compared to unamplified cDNA by qPCR and microarray analyses.

(a) unamplified control cDNA synthesis

Single- stranded cDNA was prepared from 30 micrograms of total human liver RNA (cat. # 7960; Ambion, Austin, TX) and Universal Human Reference (UHR) total RNA (cat. # 74000; Stratagene, La Jolla, CA) at a concentration of 1 microgram of total RNA per 50- microliter reaction, using 1 μM oligo $dT_{19}$ primer following the procedure described for MMLV- reverse transcriptase (cat. # M1302; Sigma- Aldrich) .

(b) D-amplified cDNA synthesis

One microgram of human liver or UHR total RNA per 25 microliters and 1 μM of an oligo dT primer (5'- $GTAGGTTGAGGATAGGAGGGTTAGGT_{19}$- 3'; SEQ ID NO: 1) were incubated at 70°C for 5 minutes, quick cooled on ice, and followed immediately by addition of 10 unit/ microliter MMLV- reverse transcriptase (Sigma- Aldrich), 1 x PCR Buffer (cat. # P2192; Sigma- Aldrich), magnesium chloride (cat. # M8787; Sigma- Aldrich) added to 3 mM final concentration, 500 μM dNTPs, and 2.5% (volume) Ribonuclease Inhibitor (cat. #R2520; Sigma- Aldrich) and incubated at 37° C for 5 minutes, 42° C for 45 minutes, 94° C for 5 minutes, and quick- chilled on ice.

Complementary second cDNA strand was synthesized using 1 μM of the D9 library synthesis primer (5'- $GTAGGTTGAGGATAGGAGGGTTAGGD_9$- 3'; SEQ ID NO: 2), 0.165 units/ microliter JUMPSTART™ Taq DNA polymerase (cat. # D3443; Sigma- Aldrich), 0.18 unit/ microliter Klenow exo- minus DNA polymerase (cat. # 7057Z; USB, Cleveland, OH), 1x PCR Buffer (see above), 5.5 mM added magnesium chloride (see above) and 500 μM dNTPs. The mixture was incubated at 18° C for 5 minutes, 25° C for 5 minutes, 37° C for 5 minutes, and 72° C for 15 minutes.

Double- stranded cDNAs were amplified using 0.05 units/ microliter JUMPSTART™ Taq (see above), 1X PCR Buffer (cat. # D4545, without magnesium chloride, Sigma- Aldrich), 1.5 mM magnesium chloride (see above), 200 μM dNTPs and 2 μM of the universal primer 5'- GTAGGTTGAGGATAGGAGGGTTAGG- 3' (SEQ ID NO: 3) . Thermocycling parameters were: 94° C for 90 seconds, then seventeen cycles of 94° C for 30 seconds, 65° C for 30 seconds, and 72° C for 2 minutes.

(c) K-amplified cDNA synthesis

Amplified cDNA was prepared from 0.2 micrograms total RNAs (see above) using the synthesis components and procedures of the Rubicon Transplex™ WTA Kit (see above).

(d) RNA removal and cDNA purification

Total RNA template in unamplified control cDNA and amplified cDNAs was degraded by addition (in sequence) of 1/3 final cDNA/amplification reaction volume of 0.5 M EDTA and 1/3 final cDNA/amplification reaction volume of 1 M NaOH, with incubation at 65° C for 15 minutes. Reactions were then neutralized with 5/6 final cDNA/amplification reaction volume of 1 M Tris HCl, pH 7.4, and purified using the GenElute PCR Cleanup kit as described (cat.# NA1020; Sigma-Aldrich).

(e) quantitative PCR (qPCR) analysis

Amplified cDNAs and unamplified control cDNAs were analyzed by real- time quantitative PCR, using conditions prescribed for 2X SYBR® Green JUMPSTART™ Taq (cat. # S4438; Sigma- Aldrich), with 250 nM human primers pairs (see Table 1) . Cycling conditions were 1 cycle at 94° C for 1.5 minutes, and 30 cycles at 94° C for 30 seconds; 60° C for 30 seconds; and 72° C for 2.5 minutes.

**Table 1.** Primers used in qPCR.

| Primer Set | Gene | Primer 1 Sequence (5'-3') | SEQ ID NO: | Primer 2 Sequence (5'-3') | SEQ ID NO: |
|---|---|---|---|---|---|
| 1 | M55047 | TGCTTAGACCCGT AGTTTCC | 4 | CTTGACAAAATGC TGTGTTCC | 5 |
| 2 | sts-N90764 | CGTTTAATTCTGTG GCCAGG | 6 | AGCCAAGTACCCC GACTACG | 7 |
| 3 | WI-13668 | TGTTAACAATTTGC ATAACAAAAGC | 8 | TGATTAATTTGCGA GACTAACTTTG | 9 |
| 4 | shgc-79529 | GTTTCGAATCCCA GGAATTAAGC | 10 | CACAATCAGCAAC AAAATCATCC | 11 |
| 5 | shgc-11640 | GCAAACAAAGCAT GCTTCAA | 12 | TTCTCCCAGCTTT GAGACGT | 13 |

(continued)

| Primer Set | Gene | Primer 1 Sequence (5'-3') | SEQ ID NO: | Primer 2 Sequence (5'-3') | SEQ ID NO: |
|---|---|---|---|---|---|
| 6 | SHGC-36464 | TATTTAAAATGTGG GCAAGATATCA | 14 | TGGTGTAAATAAA GACCTTGCTATC | 15 |
| 7 | kiaa0108 | TTTGTTACTTGCTA CCCTGAG | 16 | CAACCATCATCTTC CACAGTC | 17 |
| 8 | stSG53466 | AGACCACACCAGA AACCCTG | 18 | GAATTTTGGTTTCT TGCTTTGG | 19 |
| 9 | SHGC153324 | CCAGGGTTCGAAT CTCAGTCTTA | 20 | GATTTCTAAACTTA CGGCCCCAC | 21 |
| 10 | 1314 | AAAGAGTGTCTT GTCTTGACTTAT C | 22 | TTATCTGAGCCC TTAATAGTAAATC | 23 |
| 11 | stSG62388 | AATCAAAAGGCC AACAGTGG | 24 | TTCAGTGTTAAT GGAGCCAGG | 25 |
| 12 | sts-AA035504 | TCTCAGAGCAGA GTTTGGGC | 26 | CCTGCACTTGGA CCTGACC | 27 |

The C (t) value, which represents the PCR cycle during which the fluorescence exceeded a defined threshold level, was determined for each reaction. The average delta C (t) [$\Delta$C (t) ] was calculated and subtracted from individual $\Delta$C (t) values for that PCR template type. Figure 1 presents the $\Delta C\ (t)_{Liver\text{-}UHR}$ for each population of cDNAs as a function of the different primer sets. The results indicate that the ratio of human liver and UHR cDNA amplicon concentrations, as represented by the $\Delta$C (t) s, for the D- amplified cDNAs and the K- amplified cDNAs closely reflected the ratio of initial mRNA levels represented in the unamplified total RNA.

<u>(f) microarray analysis</u>

Target cDNA was labeled using the Kreatech ULS™ system (Kreatech Biotechnology, Amsterdam, Netherlands; the labeling was performed by Mogene, LC, NIDUS Center for Scientific Enterprise, 893 North Warson Road, Saint Louis, MO, 63141). Purified unamplified cDNA, D-amplified cDNA and K-amplified cDNA were submitted to Mogene, LC for microarray analysis. For this, 750 nanograms of target were incubated with the Agilent Whole Genome Chip (cat. # G4112A; Agilent Technologies, Santa Clara, CA).

Figure 2 presents the ratio spot intensities representing human liver and UHR target for each array probe. The log base 2 ratios of amplified cDNAs targets were plotted against the log base 2 ratio for unamplified cDNA target. Only intensities of approximately 5X background (> 250) were included in this analysis. The results reveal that D-amplified (Figure 2A) and K-amplified (Figure 2B) cDNAs had similar profiles.

**Example 2. Selection of 384 Highly Degenerate Primers.**

To further increase the degeneracy of library synthesis primers, the semi-random region was modified to include N residues, as well as either D or K residues. It was reasoned that addition ofNs would increase the sequence diversity. Table 2 lists 256 possible KN sequences (including the control K9 sequence, also called 1K9) and Table 3 lists 256 possible DN sequences (including the control D9 sequence, also called 1D9).

In an effort to minimize the number of primers to investigate, and provide a workable example, it was decided to limit the number of primers to evaluate to 384. The first cut was to eliminate any sequence containing 4 or more contiguous N residues, as it was assumed that four or more degenerate Ns could provide a substantial opportunity for primer dimer formation. This reduced the number of K or D interrupted N sequences from 256 to 208. The remaining 16 primers (i.e., 208 to 192) were eliminated on the basis of 3' diversity and self complementarity. Of the sixteen, six comprised the eight possible $N_1X_8$ sequences where maximal 3' degeneracy was maintained by keeping the two candidate sequences with N near the 3' end saving the penultimate position because 50% of the pool would be self complimentary at the final two 3' nucleotides. The remaining 10 sequences were eliminated on the basis of self-complementarity (i.e., degenerate sequences that were palindromic about a central N pairing K/D's with N, e.g. NKNNN**N**KKNK, NNKK**N**NNKK, etc.). Table 4 lists the final 384 interrupted N sequences that were selected for subsequent screening.

**Table 2.** Possible 9-mer KN sequences.

| | | | | |
|---|---|---|---|---|
| KKKKKKKKK | KKNKNNKKK | NNNKKNNKK | KNKNNKKNK | NKNNKKNNK |
| NKKKKKKKK | NKNKNNKKK | KKKNKNNKK | NNKNNKKNK | KNNNKKNNK |
| KNKKKKKKK | KNNKNNKKK | NKKNKNNKK | KKNNNKKNK | NNNNKKNNK |
| NNKKKKKKK | NNNKNNKKK | KNKNKNNKK | NKNNNKKNK | KKKKNKNNK |
| KKNKKKKKK | KKKNNNKKK | NNKNKNNKK | KNNNNKKNK | NKKKNKNNK |
| NKNKKKKKK | NKKNNNKKK | KKNNKNNKK | NNNNNKKNK | KNKKNKNNK |
| KNNKKKKKK | KNKNNNKKK | NKNNKNNKK | KKKKKNKNK | NNKKNKNNK |
| NNNKKKKKK | NNKNNNKKK | KNNNKNNKK | NKKKKNKNK | KKNKNKNNK |
| KKKNKKKKK | KKNNNNKKK | NNNNKNNKK | KNKKKNKNK | NKNKNKNNK |
| NKKNKKKKK | NKNNNNKKK | KKKKNNNKK | NNKKKNKNK | KNNKNKNNK |
| KNKNKKKKK | KNNNNNKKK | NKKKNNNKK | KKNKKNKNK | NNNKNKNNK |
| NNKNKKKKK | NNNNNNKKK | KNKKNNNKK | NKNKKNKNK | KKKNNKNNK |
| KKNNKKKKK | KKKKKKNKK | NNKKNNNKK | KNNKKNKNK | NKKNNKNNK |
| NKNNKKKKK | NKKKKKNKK | KKNKNNNKK | NNNKKNKNK | KNKNNKNNK |
| KNNNKKKKK | KNKKKKNKK | NKNKNNNKK | KKKNKNKNK | NNKNNKNNK |
| NNNNKKKKK | NNKKKKNKK | KNNKNNNKK | NKKNKNKNK | KKNNNKNNK |
| KKKKNKKKK | KKNKKKNKK | NNNKNNNKK | KNKNKNKNK | NKNNNKNNK |
| NKKKNKKKK | NKNKKKNKK | KKKNNNNKK | NNKNKNKNK | KNNNNKNNK |
| KNKKNKKKK | KNNKKKNKK | NKKNNNNKK | KKNNKNKNK | NNNNNKNNK |
| NNKKNKKKK | NNNKKKNKK | KNKNNNNKK | NKNNKNKNK | KKKKKNNNK |
| KKNKNKKKK | KKKNKKNKK | NNKNNNNKK | KNNNKNKNK | NKKKKNNNK |
| NKNKNKKKK | NKKNKKNKK | KKNNNNNKK | NNNNKNKNK | KNKKKNNNK |
| KNNKNKKKK | KNKNKKNKK | NKNNNNNKK | KKKKNNKNK | NNKKKNNNK |
| NNNKNKKKK | NNKNKKNKK | KNNNNNNKK | NKKKNNKNK | KKNKKNNNK |
| KKKNNKKKK | KKNNKKNKK | NNNNNNNKK | KNKKNNKNK | NKNKKNNNK |
| NKKNNKKKK | NKNNKKNKK | KKKKKKKNK | NNKKNNKNK | KNNKKNNNK |
| KNKNNKKKK | KNNNKKNKK | NKKKKKKNK | KKNKNNKNK | NNNKKNNNK |
| NNKNNKKKK | NNNNKKNKK | KNKKKKKNK | NKNKNNKNK | KKKNKNNNK |
| KKNNNKKKK | KKKKNKNKK | NNKKKKKNK | KNNKNNKNK | NKKNKNNNK |
| NKNNNKKKK | NKKKNKNKK | KKNKKKKNK | NNNKNNKNK | KNKNKNNNK |
| KNNNNKKKK | KNKKNKNKK | NKNKKKKNK | KKKNNNKNK | NNKNKNNNK |
| NNNNNKKKK | NNKKNKNKK | KNNKKKKNK | NKKNNNKNK | KKNNKNNNK |
| KKKKKNKKK | KKNKNKNKK | NNNKKKKNK | KNKNNNKNK | NKNNKNNNK |
| NKKKKNKKK | NKNKNKNKK | KKKNKKKNK | NNKNNNKNK | KNNNKNNNK |
| KNKKKNKKK | KNNKNKNKK | NKKNKKKNK | KKNNNNKNK | NNNNKNNNK |
| NNKKKNKKK | NNNKNKNKK | KNKNKKKNK | NKNNNNKNK | KKKKNNNNK |
| KKNKKNKKK | KKKNNKNKK | NNKNKKKNK | KNNNNNKNK | NKKKNNNNK |

(continued)

| | | | | |
|---|---|---|---|---|
| NKNKKNKKK | NKKNNKNKK | KKNNKKKNK | NNNNNNKNK | KNKKNNNNK |
| KNNKKNKKK | KNKNNKNKK | NKNNKKKNK | KKKKKKNNK | NNKKNNNNK |
| NNNKKNKKK | NNKNNKNKK | KNNNKKKNK | NKKKKKNNK | KKNKNNNNK |
| KKKNKNKKK | KKNNNKNKK | NNNNKKKNK | KNKKKKNNK | NKNKNNNNK |
| NKKNKNKKK | NKNNNKNKK | KKKKNKKNK | NNKKKKNNK | KNNKNNNNK |
| KNKNKNKKK | KNNNNKNKK | NKKKNKKNK | KKNKKKNNK | NNNKNNNNK |
| NNKNKNKKK | NNNNNKNKK | KNKKNKKNK | NKNKKKNNK | KKKNNNNNK |
| KKNNKNKKK | KKKKKNNKK | NNKKNKKNK | KNNKKKNNK | NKKNNNNKK |
| NKNNKNKKK | NKKKKNNKK | KKNKNKKNK | NNNKKKNNK | KNKNNNNNK |
| KNNNKNKKK | KNKKKNNKK | NKNKNKKNK | KKKNKKNNK | NNKNNNNNK |
| NNNNKNKKK | NNKKKNNKK | KNNKNKKNK | NKKNKKNNK | KKNNNNNNK |
| KKKKNNKKK | KKNKKNNKK | NNNKNKKNK | KNKNKKNNK | NKNNNNNNK |
| NKKKNNKKK | NKNKKNNKK | KKKNNKKNK | NNKNKKNNK | KNNNNNNKK |
| KNKKNNKKK | KNNKKNNKK | NKKNNKKNK | KKNNKKNNK | NNNNNNNNK |
| NNKKNNKKK | | | | |

**Table 3.** Possible 9-mer DN sequences.

| | | | | |
|---|---|---|---|---|
| DDDDDDDDD | DDNDNNDDD | NNNDDNNDD | DNDNNDDND | NDNNDDNND |
| NDDDDDDDD | NDNDNNDDD | DDDNDNNDD | NNDNNDDND | DNNNDDNND |
| DNDDDDDDD | DNNDNNDDD | NDDNDNNDD | DDNNNDDND | NNNNDDNND |
| NNDDDDDDD | NNNDNNDDD | DNDNDNNDD | NDNNNDDND | DDDDNDNND |
| DDNDDDDDD | DDDNNNDDD | NNDNDNNDD | DNNNNDDND | NDDDNDNND |
| NDNDDDDDD | NDDNNNDDD | DDNNDNNDD | NNNNNDDND | DNDDNDNND |
| DNNDDDDDD | DNDNNNDDD | NDNNDNNDD | DDDDDNDND | NNDDNDNND |
| NNNDDDDDD | NNDNNNDDD | DNNNDNNDD | NDDDDNDND | DDNDNDNND |
| DDDNDDDDD | DDNNNNDDD | NNNNDNNDD | DNDDDNDND | NDNDNDNND |
| NDDNDDDDD | NDNNNNDDD | DDDDNNNDD | NNDDDNDND | DNNDNDNND |
| DNDNDDDDD | DNNNNNDDD | NDDDNNNDD | DDNDDNDND | NNNDNDNND |
| NNDNDDDDD | NNNNNNDDD | DNDDNNNDD | NDNDDNDND | DDDNNDNND |
| DDNNDDDDD | DDDDDDNDD | NNDDNNNDD | DNNDDNDND | NDDNNDNND |
| NDNNDDDDD | NDDDDDNDD | DDNDNNNDD | NNNDDNDND | DNDNNDNND |
| DNNNDDDDD | DNDDDDNDD | NDNDNNNDD | DDDNDNDND | NNDNNDNND |
| NNNNDDDDD | NNDDDDNDD | DNNDNNNDD | NDDNDNDND | DDNNNDNND |
| DDDDNDDDD | DDNDDDNDD | NNNDNNNDD | DNDNDNDND | NDNNNDNND |
| NDDDNDDDD | NDNDDDNDD | DDDNNNNDD | NNDNDNDND | DNNNNDNND |
| DNDDNDDDD | DNNDDDNDD | NDDNNNNDD | DDNNDNDND | NNNNNDNND |
| NNDDNDDDD | NNNDDDNDD | DNDNNNNDD | NDNNDNDND | DDDDDNNND |
| DDNDNDDDD | DDDNDDNDD | NNDNNNNDD | DNNNDNDND | NDDDDNNND |
| NDNDNDDDD | NDDNDDNDD | DDNNNNNDD | NNNNDNDND | DNDDDNNND |

(continued)

| | | | | |
|---|---|---|---|---|
| DNNDNDDDD | DNDNDDNDD | NDNNNNNDD | DDDNNDND | NNDDDNNND |
| NNNDNDDDD | NNDNDDNDD | DNNNNNNDD | NDDDNNDND | NDNDDNNND |
| DDDNNDDDD | DDNNDDNDD | NNNNNNNDD | DNDDNNDND | NDNDDNNND |
| NDDNNDDDD | NDNNDDNDD | DDDDDDDND | NNDDNNDND | DNNDDNNND |
| DNDNNDDDD | DNNNDDNDD | NDDDDDDND | DDNDNNDND | NNNDDNNND |
| NNDNNDDDD | NNNNDDNDD | DNDDDDDND | NDNDNNDND | DDDNDNNND |
| DDNNNDDDD | DDDDNDNDD | NNDDDDDND | DNNDNNDND | NDDNDNNND |
| NDNNNDDDD | NDDDNDNDD | DDNDDDDND | NNNDNNDND | DNDNDNNND |
| DNNNNDDDD | DNDDNDNDD | NDNDDDDND | DDDNNNDND | NNDNDNNND |
| NNNNNDDDD | NNDDNDNDD | DNNDDDDND | NDDNNNDND | DDNNDNNND |
| DDDDDNDDD | DDNDNDNDD | NNNDDDDND | DNDNNNDND | NDNNDNNND |
| NDDDDNDDD | NDNDNDNDD | DDDNDDDND | NNDNNNDND | DNNNDNNND |
| DNDDDNDDD | DNNDNDNDD | NDDNDDDND | DDNNNNDND | NNNNDNNND |
| NNDDDNDDD | NNNDNDNDD | DNDNDDDND | NDNNNNDND | DDDDNNNND |
| DDNDDNDDD | DDDNNDNDD | NNDNDDDND | DNNNNNDND | NDDDNNNND |
| NDNDDNDDD | NDDNNDNDD | DDNNDDDND | NNNNNNDND | DNDDNNNND |
| DNNDDNDDD | DNDNNDNDD | NDNNDDDND | DDDDDDNND | NNDDNNNND |
| NNNDDNDDD | NNDNNDNDD | DNNNDDDND | NDDDDDNND | DDNDNNNND |
| DDDNDNDDD | DDNNNDNDD | NNNNDDDND | DNDDDDNND | NDNDNNNND |
| NDDNDNDDD | NDNNNDNDD | DDDDNDDND | NNDDDDNND | DNNDNNNND |
| DNDNDNDDD | DNNNNDNDD | NDDDNDDND | DDNDDDNND | NNNDNNNND |
| NNDNDNDDD | NNNNNDNDD | DNDDNDDND | NDNDDDNND | DDDNNNNND |
| DDNNDNDDD | DDDDDNNDD | NNDDNDDND | DNNDDDNND | NDDNNNNND |
| NDNNDNDDD | NDDDDNNDD | DDNDNDDND | NNNDDDNND | DNDNNNNND |
| DNNNDNDDD | DNDDDNNDD | NDNDNDDND | DDDNDDNND | NNDNNNNND |
| NNNNDNDDD | NNDDDNNDD | DNNDNDDND | NDDNDDNND | DDNNNNNND |
| DDDDNNDDD | DDNDDNNDD | NNNDNDDND | DNDNDDNND | NDNNNNNND |
| NDDDNNDDD | NDNDDNNDD | DDDNNDDND | NNDNDDNND | DNNNNNNND |
| DNDDNNDDD | DNNDDNNDD | NDDNNDDND | DDNNDDNND | NNNNNNNND |
| NNDDNNDDD | | | | |

**Table 4.** The 384 Interrupted N Sequences Selected for Further Screening.

| Name | Sequence (5'-3') | Name | Sequence (5'-3') | Name | Sequence (5'-3') |
|---|---|---|---|---|---|
| 1K3 | KNNNKNNNK | 24K6 | KNKNNKKKK | 25D5 | DNDNDNDND |
| 2K3 | NKNNKNNNK | 25K6 | KNNKNKKKK | 26D5 | DNNDDNDND |
| 3K3 | NNKNNNKNK | 26K6 | KNKKKNNKK | 27D5 | DNNNDNDDD |
| 4K3 | NNNKNKNNK | 27K6 | KNKKKNKNK | 28D5 | DNDNDDNND |
| 5K3 | NNKNKNNNK | 28K6 | KNKNKNKKK | 29D5 | DNNDDDNND |
| 6K3 | NNNKKNNNK | 29K6 | KNNKKNKKK | 30D5 | DNNNDDNDD |

(continued)

| Name | Sequence (5'-3') | Name | Sequence (5'-3') | Name | Sequence (5'-3') |
|---|---|---|---|---|---|
| 1K4 | KKNNNKNNK | 30K6 | KNKKKKNNK | 31D5 | DNNNDDDND |
| 2K4 | KKNNKNNNK | 31K6 | KNKNKKNKK | 32D5 | NDDDNNNDD |
| 3K4 | KNNKNNNKK | 32K6 | KNNKKKNKK | 33D5 | NDDDNNDND |
| 4K4 | KNKNNNKNK | 33K6 | KNKNKKKNK | 34D5 | NDDNNNDDD |
| 5K4 | KNNKNNKNK | 34K6 | KNNKKKKNK | 35D5 | NDNDNNDDD |
| 6K4 | KNKNNKNNK | 35K6 | KNNNKKKKK | 36D5 | NDDDNDNND |
| 7K4 | KNNKNKNNK | 36K6 | NKKKNNKKK | 37D5 | NDDNNDNDD |
| 8K4 | KNKNKNNNK | 37K6 | NKKKNKNKK | 38D5 | NDNDNDNDD |
| 9K4 | KNNKKNNNK | 38K6 | NKKKNKKNK | 39D5 | NDDNNDDND |
| 10K4 | KNNNKNNKK | 39K6 | NKKNNKKKK | 40D5 | NDNDNDDND |
| 11K4 | KNNNKNKNK | 40K6 | NKNKNKKKK | 41D5 | NDNNNDDDD |
| 12K4 | KNNNKKNNK | 41K6 | NKKKKNNKK | 42D5 | NDDDDNNND |
| 13K4 | NKNKNNNKK | 42K6 | NKKKKNKNK | 43D5 | NDDNDNNDD |
| 14K4 | NKKNNNKNK | 43K6 | NKKNKNKKK | 44D5 | NDNDDNNDD |
| 15K4 | NKNKNKNNK | 44K6 | NKNKKNKKK | 45D5 | NDDNDNDND |
| 16K4 | NKNNNKNKK | 45K6 | NKKKKKNNK | 46D5 | NDNDDNDND |
| 17K4 | NKKNKNNNK | 46K6 | NKKNKKNKK | 47D5 | NDNNDNDDD |
| 18K4 | NKNKKNNNK | 47K6 | NKNKKKNKK | 48D5 | NDDNDDNND |
| 19K4 | NKNNKNNKK | 48K6 | NKKNKKKNK | 49D5 | NDNDDDNND |
| 20K4 | NKNNKNKNK | 49K6 | NKNKKKKNK | 50D5 | NDNNDDNDD |
| 21K4 | NKNNKKNNK | 50K6 | NKNNKKKKK | 51D5 | NDNNDDDND |
| 22K4 | NNKKNNKNK | 51K6 | NNKKNKKKK | 52D5 | NNDDNNDDD |
| 23K4 | NNKNNNKKK | 52K6 | NNKKKNKKK | 53D5 | NNDDNDNDD |
| 24K4 | NNKKNKNNK | 53K6 | NNKKKKNKK | 54D5 | NNDDNDDND |
| 25K4 | NNNKNKNKK | 54K6 | NNKKKKKNK | 55D5 | NNDNNDDDD |
| 26K4 | NNKNNKKNK | 55K6 | NNKNKKKKK | 56D5 | NNNDNDDDD |
| 27K4 | NNNKNKKNK | 56K6 | NNNKKKKKK | 57D5 | NNDDDNNDD |
| 28K4 | NNKKKNNNK | 1K7 | KKKKNNKKK | 58D5 | NNDDDNDND |
| 29K4 | NNKNKNNKK | 2K7 | KKKKNKNKK | 59D5 | NNDNDNDDD |
| 30K4 | NNNKKNNKK | 3K7 | KKKKNKKNK | 60D5 | NNNDDNDDD |
| 31K4 | NNKNKNKNK | 4K7 | KKKNNKKKK | 61D5 | NNDDDDNND |
| 32K4 | NNNKKNKNK | 5K7 | KKNKNKKKK | 62D5 | NNDNDDNDD |
| 33K4 | NNKNKKNNK | 6K7 | KKKKKNNKK | 63D5 | NNNDDDNDD |
| 34K4 | NNNKKKNNK | 7K7 | KKKKKNKNK | 64D5 | NNDNDDDND |
| 1K5 | KKNKNNNKK | 8K7 | KKKNKNKKK | 65D5 | NNNDDDDND |
| 2K5 | KKKNNNKNK | 9K7 | KKNKKNKKK | 1D6 | DDDDNNNDD |
| 3K5 | KKNKNNKNK | 10K7 | KKKKKKNNK | 2D6 | DDDDNNDND |
| 4K5 | KKKNNKNNK | 11K7 | KKKNKKNKK | 3D6 | DDDNNNDDD |

(continued)

| Name | Sequence (5'-3') | Name | Sequence (5'-3') | Name | Sequence (5'-3') |
|---|---|---|---|---|---|
| 5K5 | KKNKNKNNK | 12K7 | KKNKKKNKK | 4D6 | DDNDNNDDD |
| 6K5 | KKNNNKNKK | 13K7 | KKKNKKKNK | 5D6 | DDDDNDNND |
| 7K5 | KKNNNKKNK | 14K7 | KKNKKKKNK | 6D6 | DDDNNDNDD |
| 8K5 | KKKNKNNNK | 15K7 | KKNNKKKKK | 7D6 | DDNDNDNDD |
| 9K5 | KKNKKNNNK | 16K7 | KNKKNKKKK | 8D6 | DDDNNDDND |
| 10K5 | KKNNKNNKK | 17K7 | KNKKKNKKK | 9D6 | DDNDNDDND |
| 11K5 | KKNNKNKNK | 18K7 | KNKKKKNKK | 10D6 | DDNNNDDDD |
| 12K5 | KKNNKKNNK | 19K7 | KNKKKKKNK | 11D6 | DDDDDNNND |
| 13K5 | KNKKNNNKK | 20K7 | KNKNKKKKK | 12D6 | DDDNDNNDD |
| 14K5 | KNKKNNKNK | 21K7 | KNNKKKKKK | 13D6 | DDNDDNNDD |
| 15K5 | KNKNNNKKK | 22K7 | NKKKNKKKK | 14D6 | DDDNDNDND |
| 16K5 | KNNKNNKKK | 23K7 | NKKKKNKKK | 15D6 | DDNDDNDND |
| 17K5 | KNKKNKNNK | 24K7 | NKKKKKNKK | 16D6 | DDNNDNDDD |
| 18K5 | KNKNNKNKK | 25K7 | NKKKKKKNK | 17D6 | DDDNDDNND |
| 19K5 | KNNKNKNKK | 26K7 | NKKNKKKKK | 18D6 | DDNDDDNND |
| 20K5 | KNKNNKKNK | 27K7 | NKNKKKKKK | 19D6 | DDNNDDNDD |
| 21K5 | KNNKNKKNK | 28K7 | NNKKKKKKK | 20D6 | DDNNDDDND |
| 22K5 | KNKKKNNNK | 1K8 | KKKKKNKKK | 21D6 | DNDDNNDDD |
| 23K5 | KNKNKNNKK | 2K8 | KKKKKKNKK | 22D6 | DNDDNDNDD |
| 24K5 | KNNKKNNKK | 1K9 | KKKKKKKKK | 23D6 | DNDDNDDND |
| 25K5 | KNKNKNKNK | 1D3 | DNNNDNNND | 24D6 | DNDNNDDDD |
| 26K5 | KNNKKNKNK | 2D3 | NDNNDNNND | 25D6 | DNNDNDDDD |
| 27K5 | KNNNKNKKK | 3D3 | NNDNNNDND | 26D6 | DNDDDNNDD |
| 28K5 | KNKNKKNNK | 4D3 | NNNDNDNND | 27D6 | DNDDDNDND |
| 29K5 | KNNKKKNNK | 5D3 | NNDNDNNND | 28D6 | DNDNDNDDD |
| 30K5 | KNNNKKNKK | 6D3 | NNNDDNNND | 29D6 | DNNDDNDDD |
| 31K5 | KNNNKKKNK | 1D4 | DDNNNDNND | 30D6 | DNDDDDNND |
| 32K5 | NKKKNNNKK | 2D4 | DDNNDNNND | 31D6 | DNDNDDNDD |
| 33K5 | NKKKNNKNK | 3D4 | DNNDNNNDD | 32D6 | DNNDDDNDD |
| 34K5 | NKKNNNKKK | 4D4 | DNDNNNDND | 33D6 | DNDNDDDND |
| 35K5 | NKNKNNKKK | 5D4 | DNNDNNDND | 34D6 | DNNDDDDND |
| 36K5 | NKKKNKNNK | 6D4 | DNDNNDNND | 35D6 | DNNNDDDDD |
| 37K5 | NKKNNKNKK | 7D4 | DNNDNDNND | 36D6 | NDDDNNDDD |
| 38K5 | NKNKNKNKK | 8D4 | DNDNDNNND | 37D6 | NDDDNDNDD |
| 39K5 | NKKNNKKNK | 9D4 | DNNDDNNND | 38D6 | NDDDNDDND |
| 40K5 | NKNKNKKNK | 10D4 | DNNNDNNDD | 39D6 | NDDNNDDDD |
| 41K5 | NKNNNKKKK | 11D4 | DNNNDNDND | 40D6 | NDNDNDDDD |
| 42K5 | NKKKKNNNK | 12D4 | DNNNDDNND | 41D6 | NDDDDNNDD |

(continued)

| Name | Sequence (5'-3') | Name | Sequence (5'-3') | Name | Sequence (5'-3') |
|---|---|---|---|---|---|
| 43K5 | NKKNKNNKK | 13D4 | NDNDNNNDD | 42D6 | NDDDDNDND |
| 44K5 | NKNKKNNKK | 14D4 | NDDNNNDND | 43D6 | NDDNDNDDD |
| 45K5 | NKKNKNKNK | 15D4 | NDNDNDNND | 44D6 | NDNDDNDDD |
| 46K5 | NKNKKNKNK | 16D4 | NDNNNDNDD | 45D6 | NDDDDDNND |
| 47K5 | NKNNKNKKK | 17D4 | NDDNDNNND | 46D6 | NDDNDDNDD |
| 48K5 | NKKNKKNNK | 18D4 | NDNDDNNND | 47D6 | NDNDDDNDD |
| 49K5 | NKNKKKNNK | 19D4 | NDNNDNNDD | 48D6 | NDDNDDDND |
| 50K5 | NKNNKKNKK | 20D4 | NDNNDNDND | 49D6 | NDNDDDDND |
| 51K5 | NKNNKKKNK | 21D4 | NDNNDDNND | 50D6 | NDNNDDDDD |
| 52K5 | NNKKNNKKK | 22D4 | NNDDNNDND | 51D6 | NNDDNDDDD |
| 53K5 | NNKKNKNKK | 23D4 | NNDNNNDDD | 52D6 | NNDDDNDDD |
| 54K5 | NNKKNKKNK | 24D4 | NNDDNDNND | 53D6 | NNDDDDNDD |
| 55K5 | NNKNNKKKK | 25D4 | NNNDNDNDD | 54D6 | NNDDDDDND |
| 56K5 | NNNKNKKKK | 26D4 | NNDNNDDND | 55D6 | NNDNDDDDD |
| 57K5 | NNKKKNNKK | 27D4 | NNNDNDDND | 56D6 | NNNDDDDDD |
| 58K5 | NNKKKNKNK | 28D4 | NNDDDNNND | 1D7 | DDDDNNDDD |
| 59K5 | NNKNKNKKK | 29D4 | NNDNDNNDD | 2D7 | DDDDNDNDD |
| 60K5 | NNNKKNKKK | 30D4 | NNNDDNNDD | 3D7 | DDDDNDDND |
| 61K5 | NNKKKKNNK | 31D4 | NNDNDNDND | 4D7 | DDDNNDDDD |
| 62K5 | NNKNKKNKK | 32D4 | NNNDDNDND | 5D7 | DDNDNDDDD |
| 63K5 | NNNKKKNKK | 33D4 | NNDNDDNND | 6D7 | DDDDDNNDD |
| 64K5 | NNKNKKKNK | 34D4 | NNNDDDNND | 7D7 | DDDDDNDND |
| 65K5 | NNNKKKKNK | 1D5 | DDNDNNNDD | 8D7 | DDDNDNDDD |
| 1K6 | KKKKNNNKK | 2D5 | DDDNNNDND | 9D7 | DDNDDNDDD |
| 2K6 | KKKKNNKNK | 3D5 | DDNDNNDND | 10D7 | DDDDDDNND |
| 3K6 | KKKNNNKKK | 4D5 | DDDNNDNND | 11D7 | DDDNDDNDD |
| 4K6 | KKNKNNKKK | 5D5 | DDNDNDNND | 12D7 | DDNDDDNDD |
| 5K6 | KKKKNKNNK | 6D5 | DDNNNDNDD | 13D7 | DDDNDDDND |
| 6K6 | KKKNNKNKK | 7D5 | DDNNNDDND | 14D7 | DDNDDDDND |
| 7K6 | KKNKNKNKK | 8D5 | DDDNDNNND | 15D7 | DDNNDDDDD |
| 8K6 | KKKNNKKNK | 9D5 | DDNDDNNND | 16D7 | DNDDNDDDD |
| 9K6 | KKNKNKKNK | 10D5 | DDNNDNDND | 17D7 | DNDDDNDDD |
| 10K6 | KKNNNKKKK | 11D5 | DDNNDNDND | 18D7 | DNDDDDNDD |
| 11K6 | KKKKKNNNK | 12D5 | DDNNDDNND | 19D7 | DNDDDDDND |
| 12K6 | KKKNKNNKK | 13D5 | DNDDNNNDD | 20D7 | DNDNDDDDD |
| 13K6 | KKNKKNNKK | 14D5 | DNDDNNDND | 21D7 | DNNDDDDDD |
| 14K6 | KKKNKNKNK | 15D5 | DNDNNNDDD | 22D7 | NDDDNDDDD |
| 15K6 | KKNKKNKNK | 16D5 | DNNDNNDDD | 23D7 | NDDDDNDDD |

(continued)

| Name | Sequence (5'-3') | Name | Sequence (5'-3') | Name | Sequence (5'-3') |
|---|---|---|---|---|---|
| 16K6 | KKNNKNKKK | 17D5 | DNDDNDNND | 24D7 | NDDDDDNDD |
| 17K6 | KKKNKKNNK | 18D5 | DNDNNDNDD | 25D7 | NDDDDDDND |
| 18K6 | KKNKKKNNK | 19D5 | DNNDNDNDD | 26D7 | NDDNDDDDD |
| 19K6 | KKNNKKNKK | 20D5 | DNDNNDDND | 27D7 | NDNDDDDDD |
| 20K6 | KKNNKKKNK | 21D5 | DNNDNDDND | 28D7 | NNDDDDDDD |
| 21K6 | KNKKNNKKK | 22D5 | DNDDDNNND | 1D8 | DDDDDNDDD |
| 22K6 | KNKKNKNKK | 23D5 | DNDNDNNDD | 2D8 | DDDDDDNDD |
| 23K6 | KNKKNKKNK | 24D5 | DNNDDNNDD | 1D9 | DDDDDDDDD |

**Example 3. Identification of the Five Best Interrupted N Library Synthesis Primers.**

The 384 interrupted N sequences were used to generate 384 library synthesis primers. Each primer comprised a constant 5' universal sequence (5'- GTGGTGTGTTGGGTGTGTTTGG- 3'; SEQ ID NO: 28) and one of the 9- mer interrupted N sequences listed in Table 4. The primers were screened by using them in whole transcriptome amplifications (WTA) . The WTA screening process was performed in three steps: 1) library synthesis, 2) library amplification, and 3) gene specific qPCR.

(a) library synthesis and amplification

Each library synthesis reaction comprised 2.5 μl of 1.66 ng/μl total RNA (liver) and 2.5 μl of 5 μM of one of the 384 library synthesis primers. The mixture was heated to 70° C for 5 minutes, and then cooled on ice. To each reaction mixture, 2.5 μl of the library master mix was added (the master mix contained 1.5 mM dNTPs, 3X MMLV reaction buffer, 24 Units/μl of MMLV reverse transcriptase, and 1.2 Units/μl of Klenow exo-minus DNA polymerase, as described above). The reaction was mixed and incubated at 18° C for 10 minutes, 25° C for 10 minutes, 37° C for 30 minutes, 42° C for 10 minutes, 95° C for 5 minutes, and then stored at 4° C until dilution.

Each library reaction product was diluted by adding 70 μl of $H_2O$. The library was amplified by mixing 10 μl of diluted library and 10 μl of 2X amplification mix (2X SYBR® Green JUMPSTART™ Taq READYMIX™ and 5 μM of universal primer, 5'-GTGGTGTGTTGGGTGTGTTTGG-3'; SEQ ID NO:28). The WTA mixture was subjected to 25 cycles of 94° C for 30 seconds and 70° C for 5 minutes.

(b) qPCR reactions

Each WTA product was diluted with 180 μl of $H_2O$ and subjected to a series of "culling" qPCRs, as outline below in Table 5. The gene-specific primers used in these qPCR reactions are listed in Table 6. Each reaction mixture contained 10 μl of diluted WTA product library and 10 μl of 2X amplification mix (2X SYBR® Green JUMPSTART™ Taq READYMIX™ and 0.5 μM of each gene-specific primer). The mixture was heated to 94° C for 2 minutes and then 40 cycles of 94° C for 30 seconds, 60° C for 30 seconds, and 72° C for 30 seconds. The plates were read at 72, 76, 80, and 84° C (MJ Opticom Monitor 2 thermocycler; MJ Research, Waltham, MA). The Ct value, which represents the PCR cycle during which the fluorescence exceeded a defined threshold level, was determined for each reaction.

**Table 5.** Screening Strategy.

| Screen | No. of Reactions | Gene |
|---|---|---|
| 1 | 384 | beta actin |
| 2 | 96 | NM_001799 |
| 3a | 48 | NM_001570-[22348]-01 |
| 3b | 48 | Human B2M Reference Gene |
| 4a | 16 | ATP6V1G1 |
| 4b | 16 | CTNNB1 |

(continued)

| Screen | No. of Reactions | Gene |
|---|---|---|
| 4c | 16 | GAPDH |
| 4d | 16 | GPI |
| 4e | 16 | NM 000942 |
| 4f | 16 | NM 003234 |

**Table 6.** Sequences of Gene-Specific PCR Primers.

| Gene | Primer 1 (5'-3') | SEQID NO: | Primer 2 (5'-3') | SEQID NO: |
|---|---|---|---|---|
| beta actin | CTGGAACGGTGAAGGT | 29 | AAGGGACTTCCTGTAAC | 30 |
| | GACA | | AATGCA | |
| NM_001799 | CTCAGTTGGTGTGCCC AAAGTTTCA | 31 | TAGCAGAGTTACTTCTA AGGGTTC | 32 |
| NM_001570 -[22348]-01 | GATCATCCTGAACTGG AAACC | 33 | GCCTTTCTTACAGAAGC TGCCAAA | 34 |
| Human B2M Ref. Gene | CGGCATCTTCAAACCT CCATGA | 35 | GCCTGCCGTGTGAACC ATGTGACTTTGTC | 36 |
| ATP6V1G1 | TGGACAACCTCTTGGC TTTT | 37 | TAAAATGCCACTCCACA GCA | 38 |
| CTNNB1 | TTGAAAATCCAGCGTG GACA | 39 | TCGAGTCATTGCATACT GTC | 40 |
| GAPDH | GAAGGTGAAGGTCGG AGTC | 41 | GAAGATGGTGATGGGA TTTC | 41 |
| GPI | AGGCTGCTGCCACATA AGGT | 43 | CCAAGGCTCCAAGCAT GAAT | 44 |
| NM_000942 | CAAAGTCACCGTCAAG GTGTAT | 45 | GGAACAGTCTTTCCGAA GAGACCAA | 46 |
| NM_003234 | CAGACTAACAACAGAT TTCGGGAAT | 47 | GAGGAAGTGATACTCC ACTCTCAT | 48 |

The first qPCR screen comprised amplification of the beta actin gene. The reactions were performed in four 96- well plates. To mitigate plate- to- plate variation, each plate's average Ct was calculated and the delta Ct (ΔCt) of each reaction on a plate was determined as Ct (avg)- Ct (reaction) . Data from the four qPCR plates were combined into a single table and sorted on delta Ct (Table 7) . Inspection of the table revealed no apparent plate biasing (i.e. the distribution of delta Cts appeared statistically distributed between the four plates) .

**Table 7.** First qPCR Screen – Amplification of Beta Actin.

| Plate | DNA name | Sequence (5'-3') | Ct (dR) | delta Ct | Plate | DNA name | Sequence (5'-3') | Ct (dR) | delta Ct |
|---|---|---|---|---|---|---|---|---|---|
| 1 | 8D7 | DDDNDNDDD | NoCt | NA | 1 | 15D7 | DDNNDDDDD | 16.54 | 0 |
| 2 | 16D7 | DNDDNDDDD | 10.33 | 3.14 | 2 | 43D6 | NDDNDNDDD | 13.48 | -0.01 |
| 3 | 1D9 | DDDDDDDDD | 10.92 | 2.23 | 2 | 43K5 | NKKNKNNKK | 13.48 | -0.01 |
| 2 | 13K6 | KKNKKNNKK | 11.66 | 1.81 | 1 | 13K7 | KKKNKKKNK | 16.55 | -0.01 |
| 2 | 19D7 | DNDDDDDND | 11.81 | 1.66 | 1 | 5D4 | DNNDNNDND | 16.56 | -0.02 |
| 2 | 45K6 | NKKKKKNNK | 11.94 | 1.53 | 2 | 9D6 | DDNDNDDND | 13.49 | -0.02 |
| 2 | 17D7 | DNDDDNDDD | 12.02 | 1.45 | 2 | 13K5 | KNKKNNNKK | 13.5 | -0.03 |

**Table 7.** First qPCR Screen – Amplification of Beta Actin.

| Plate | DNA name | Sequence (5'-3') | Ct (dR) | delta Ct | Plate | DNA name | Sequence (5'-3') | Ct (dR) | delta Ct |
|---|---|---|---|---|---|---|---|---|---|
| 3 | 24D7 | NDDDDDNDD | 11.8 | 1.35 | 1 | 9D4 | DNNDDNNND | 16.57 | -0.03 |
| 2 | 18K4 | NKNKKNNNK | 12.15 | 1.32 | 1 | 33K6 | KNKNKKKNK | 16.57 | -0.03 |
| 1 | 2K5 | KKKNNNKNK | 15.22 | 1.32 | 1 | 4D4 | DNDNNNDND | 16.58 | -0.04 |
| 4 | 56K6 | NNNKKKKKK | 14.82 | 1.31 | 1 | 7D4 | DNNDNDNND | 16.58 | -0.04 |
| 3 | 54D6 | NNDDDDDND | 11.9 | 1.25 | 1 | 38D5 | NDNDNDNDD | 16.58 | -0.04 |
| 3 | 25D7 | NDDDDDDND | 11.91 | 1.24 | 4 | 3D3 | NNDNNNDND | 16.17 | -0.04 |
| 2 | 40D6 | NDNDNDDDD | 12.26 | 1.21 | 4 | 59K5 | NNKNKNKKK | 16.17 | -0.04 |
| 2 | 18D7 | DNDDDDNDD | 12.28 | 1.19 | 3 | 56D5 | NNNDNDDDD | 13.2 | -0.05 |
| 3 | 27D7 | NDNDDDDDD | 11.96 | 1.19 | 3 | 55D6 | NNDNDDDDD | 13.2 | -0.05 |
| 2 | 8K6 | KKKNNKKNK | 12.28 | 1.19 | 3 | 52K6 | NNKKKNKKK | 13.2 | -0.05 |
| 3 | 54D5 | NNDDNDDND | 12.01 | 1.14 | 1 | 37K6 | NKKKNKNKK | 16.59 | -0.05 |
| 4 | 60K5 | NNNKKNKKK | 15 | 1.13 | 2 | 11K5 | KKNNKNKNK | 13.53 | -0.06 |
| 4 | 29K6 | KNNKKNKKK | 15.02 | 1.11 | 2 | 19K7 | KNKKKKKNK | 13.53 | -0.06 |
| 2 | 11K4 | KNNNKNKNK | 12.41 | 1.06 | 2 | 23K7 | NKKKKNKKK | 13.53 | -0.06 |
| 2 | 14K6 | KKKNKNKNK | 12.42 | 1.05 | 3 | 27K7 | NKNKKKKKK | 13.21 | -0.06 |
| 2 | 20D7 | DNDNDDDDD | 12.44 | 1.03 | 4 | 31D4 | NNDNDNDND | 16.19 | -0.06 |
| 4 | 3K7 | KKKKNKKNK | 15.11 | 1.02 | 1 | 2D6 | DDDDNNDND | 16.6 | -0.06 |
| 2 | 8D6 | DDDNNDDND | 12.48 | 0.99 | 2 | 10K5 | KKNNKNNKK | 13.54 | -0.07 |
| 4 | 27K4 | NNNKNKKNK | 15.15 | 0.98 | 3 | 18K6 | KKNKKKNNK | 13.22 | -0.07 |
| 4 | 32K4 | NNNKKNKNK | 15.18 | 0.95 | 4 | 27K6 | KNKKKNKNK | 16.2 | -0.07 |
| 4 | 31D5 | DNNNDDDND | 15.2 | 0.93 | 4 | 57D5 | NNDDDNNDD | 16.21 | -0.08 |
| 1 | 38K5 | NKNKNKNKK | 15.63 | 0.91 | 1 | 37D5 | NDDNNDNDD | 16.64 | -0.1 |
| 3 | 1D8 | DDDDDNDDD | 12.27 | 0.88 | 4 | 26K5 | KNNKKNKNK | 16.24 | -0.11 |
| 1 | 34K5 | NKKNNNKKK | 15.66 | 0.88 | 2 | 23D7 | NDDDDNDDD | 13.59 | -0.12 |
| 2 | 9D5 | DDNDDNNND | 12.61 | 0.86 | 2 | 47K5 | NKNNKNKKK | 13.6 | -0.13 |
| 2 | 14D6 | DDDNDNDND | 12.61 | 0.86 | 2 | 12D5 | DDNNDDNND | 13.61 | -0.14 |
| 1 | 65D5 | NNNDDDDND | 15.69 | 0.85 | 3 | 22D6 | DNDDNDNDD | 13.29 | -0.14 |
| 1 | 35K6 | KNNNKKKKK | 15.69 | 0.85 | 3 | 24D4 | NNDDNDNND | 13.3 | -0.15 |
| 3 | 24K4 | NNKKNKNNK | 12.32 | 0.83 | 1 | 6D6 | DDDNNDNDD | 16.69 | -0.15 |
| 3 | 19K4 | NKNNKNNKK | 12.34 | 0.81 | 1 | 7K6 | KKNKNKNKK | 16.69 | -0.15 |
| 2 | 48D5 | NDDNDDNND | 12.67 | 0.8 | 4 | 34D4 | NNNDDDNND | 16.29 | -0.16 |
| 3 | 28D7 | NNDDDDDDD | 12.35 | 0.8 | 4 | 1K4 | KKNNNKNNK | 16.29 | -0.16 |
| 3 | 1K9 | KKKKKKKKK | 12.35 | 0.8 | 3 | 51K5 | NKNNKKKNK | 13.31 | -0.16 |
| 1 | 5K6 | KKKKNKNNK | 15.75 | 0.79 | 3 | 21K6 | KNKKNNKKK | 13.32 | -0.17 |
| 1 | 5D6 | DDDDNDNND | 15.76 | 0.78 | 4 | 2D3 | NDNNDNNND | 16.3 | -0.17 |
| 2 | 14K4 | NKKNNNKNK | 12.7 | 0.77 | 4 | 5D3 | NNDNDNNND | 16.3 | -0.17 |
| 2 | 15K4 | NKNKNKNNK | 12.7 | 0.77 | 4 | 31K5 | KNNNKKKNK | 16.3 | -0.17 |
| 2 | 41D5 | NDNNNDDDD | 12.7 | 0.77 | 1 | 34K6 | KNNKKKKNK | 16.72 | -0.18 |
| 1 | 8K5 | KKKNKNNNK | 15.77 | 0.77 | 4 | 58K5 | NNKKKNKNK | 16.31 | -0.18 |
| 2 | 12K4 | KNNNKKNNK | 12.72 | 0.75 | 3 | 55D5 | NNDNNDDDD | 13.34 | -0.19 |
| 1 | 36K5 | NKKKNKNNK | 15.79 | 0.75 | 1 | 14D7 | DDNDDDDND | 16.73 | -0.19 |
| 1 | 9K7 | KKNKKNKKK | 15.79 | 0.75 | 4 | 4D7 | DDDNNDDDD | 16.32 | -0.19 |
| 4 | 4K7 | KKKNNKKKK | 15.38 | 0.75 | 2 | 10D5 | DDNNDNNDD | 13.67 | -0.2 |
| 2 | 48K5 | NKKNKKNNK | 12.73 | 0.74 | 2 | 40K6 | NKNKNKKKK | 13.67 | -0.2 |
| 4 | 6K3 | NNNKKNNNK | 15.39 | 0.74 | 3 | 19D4 | NDNNDNNDD | 13.36 | -0.21 |
| 4 | 4K3 | NNNKNKNNK | 15.41 | 0.72 | 1 | 34D5 | NDDNNNDDD | 16.75 | -0.21 |
| 4 | 57K5 | NNKKKNNKK | 15.42 | 0.71 | 1 | 3K6 | KKKNNNKKK | 16.76 | -0.22 |
| 1 | 6K5 | KKNNNKNKK | 15.84 | 0.7 | 2 | 15D4 | NDNDNDNND | 13.7 | -0.23 |

**Table 7.** First qPCR Screen – Amplification of Beta Actin.

| Plate | DNA name | Sequence (5'-3') | Ct (dR) | delta Ct | Plate | DNA name | Sequence (5'-3') | Ct (dR) | delta Ct |
|---|---|---|---|---|---|---|---|---|---|
| 2 | 13K4 | NKNKNNNKK | 12.78 | 0.69 | 3 | 49K6 | NKNKKKKNK | 13.38 | -0.23 |
| 3 | 24K5 | KNNKKNNKK | 12.46 | 0.69 | 1 | 38D6 | NDDDNDDND | 16.78 | -0.24 |
| 3 | 49D5 | NDNDDDNND | 12.49 | 0.66 | 4 | 5K3 | NNKNKNNNK | 16.38 | -0.25 |
| 2 | 16K5 | KNNKNNKKK | 12.81 | 0.66 | 1 | 7D6 | DDNDNDNDD | 16.79 | -0.25 |
| 4 | 62K5 | NNKNKKNKK | 15.47 | 0.66 | 1 | 8D4 | DNDNDNNND | 16.81 | -0.27 |
| 2 | 9K5 | KKNKKNNNK | 12.84 | 0.63 | 1 | 7K4 | KNNKNKNNK | 16.81 | -0.27 |
| 3 | 24K7 | NKKKKKNKK | 12.52 | 0.63 | 4 | 4D3 | NNNDNDNND | 16.4 | -0.27 |
| 1 | 15K7 | KKNNKKKKK | 15.91 | 0.63 | 2 | 43D5 | NDDNDNNDD | 13.74 | -0.27 |
| 3 | 16D6 | DDNNDNDDD | 12.53 | 0.62 | 3 | 2K8 | KKKKKKNKK | 13.42 | -0.27 |
| 4 | 6K7 | KKKKKNNKK | 15.51 | 0.62 | 4 | 29D4 | NNDNDNNDD | 16.41 | -0.28 |
| 2 | 12D6 | DDDNDNNDD | 12.86 | 0.61 | 1 | 6K4 | KNKNNNKNNK | 16.82 | -0.28 |
| 2 | 22D7 | NDDDNDDDD | 12.86 | 0.61 | 2 | 14D4 | NDDNNNDND | 13.76 | -0.29 |
| 2 | 21K7 | KNNKKKKKK | 12.86 | 0.61 | 1 | 33D5 | NDDDNNDND | 16.83 | -0.29 |
| 4 | 5D7 | DDNDNDDDD | 15.52 | 0.61 | 1 | 33D6 | DNDNDDDND | 16.83 | -0.29 |
| 4 | 7D7 | DDDDDNDND | 15.53 | 0.6 | 2 | 13D4 | NDNDNNNDD | 13.77 | -0.3 |
| 4 | 5K7 | KKNKNKKKK | 15.53 | 0.6 | 3 | 53K5 | NNKKNKNKK | 13.45 | -0.3 |
| 4 | 61K5 | NNKKKKNNK | 15.54 | 0.59 | 1 | 5K5 | KKNKNKNNK | 16.84 | -0.3 |
| 2 | 16K7 | KNKKNKKKK | 12.88 | 0.59 | 1 | 7K5 | KKNNNKKNK | 16.84 | -0.3 |
| 3 | 25K4 | NNNKNKNKK | 12.58 | 0.57 | 1 | 6K6 | KKKNNKNKK | 16.84 | -0.3 |
| 1 | 13D7 | DDDNDDDND | 15.97 | 0.57 | 2 | 42D5 | NDDDDNNND | 13.78 | -0.31 |
| 3 | 26D7 | NDDNDDDDD | 12.59 | 0.56 | 2 | 15K6 | KKNKKNKNK | 13.78 | -0.31 |
| 1 | 11K7 | KKKNKKNKK | 15.98 | 0.56 | 3 | 55K5 | NNKNNKKKK | 13.47 | -0.32 |
| 4 | 30K5 | KNNNKKNKK | 15.58 | 0.55 | 1 | 3K5 | KKNKNNKNK | 16.86 | -0.32 |
| 4 | 27D5 | DNNNDNDDD | 15.59 | 0.54 | 2 | 11D6 | DDDDDNNND | 13.8 | -0.33 |
| 2 | 13D5 | DNDDNNNDD | 12.94 | 0.53 | 3 | 19D5 | DNNDNDNDD | 13.48 | -0.33 |
| 4 | 30D5 | DNNNDDNDD | 15.61 | 0.52 | 3 | 53D5 | NNDDNDNDD | 13.48 | -0.33 |
| 3 | 54K5 | NNKKNKKNK | 12.63 | 0.52 | 1 | 39D6 | NDDNNDDDD | 16.87 | -0.33 |
| 4 | 63D5 | NNNDDDNDD | 15.62 | 0.51 | 1 | 37K5 | NKKNNKNKK | 16.87 | -0.33 |
| 4 | 32D4 | NNNDDNDND | 15.63 | 0.5 | 3 | 23D4 | NNDNNNDDD | 13.5 | -0.35 |
| 3 | 54K6 | NNKKKKKNK | 12.66 | 0.49 | 1 | 65K5 | NNNKKKKNK | 16.89 | -0.35 |
| 2 | 42D6 | NDDDDNDND | 13 | 0.47 | 1 | 2D5 | DDDNNNDND | 16.9 | -0.36 |
| 3 | 48D6 | NDDNDDDND | 12.68 | 0.47 | 1 | 4K6 | KKNKNNKKK | 16.9 | -0.36 |
| 3 | 55K6 | NNKNKKKKK | 12.68 | 0.47 | 3 | 1K8 | KKKKKNKKK | 13.51 | -0.36 |
| 4 | 64D5 | NNDNDDDND | 15.67 | 0.46 | 2 | 16D4 | NDNNNDNDD | 13.84 | -0.37 |
| 4 | 30K4 | NNNKKNNKK | 15.67 | 0.46 | 3 | 18K5 | KNKNNKNKK | 13.52 | -0.37 |
| 4 | 1K7 | KKKKNNKKK | 15.67 | 0.46 | 1 | 8D5 | DDDNDNNND | 16.92 | -0.38 |
| 3 | 21D6 | DNDDNNDDD | 12.7 | 0.45 | 1 | 32D6 | DNNDDDNDD | 16.92 | -0.38 |
| 3 | 49K5 | NKNKKKNNK | 12.7 | 0.45 | 4 | 2D7 | DDDDNDNDD | 16.51 | -0.38 |
| 2 | 18K7 | KNKKKKNKK | 13.02 | 0.45 | 2 | 44D6 | NDNDDNDDD | 13.86 | -0.39 |
| 1 | 5D5 | DDNDNDNND | 16.09 | 0.45 | 4 | 25D6 | DNNDNDDDD | 16.53 | -0.4 |
| 4 | 61D5 | NNDDDDNND | 15.68 | 0.45 | 3 | 26D4 | NNDNNDDND | 13.56 | -0.41 |
| 4 | 28K5 | KNKNKKNNK | 15.68 | 0.45 | 4 | 30D6 | DNDDDDNND | 16.54 | -0.41 |
| 3 | 51K6 | NNKKNKKKK | 12.71 | 0.44 | 2 | 20K7 | KNKNKKKKK | 13.88 | -0.41 |
| 4 | 2K3 | NKNNKNNNK | 15.7 | 0.43 | 1 | 7D5 | DDNNNDDND | 16.95 | -0.41 |
| 1 | 6D5 | DDNNNDNDD | 16.11 | 0.43 | 3 | 18D6 | DDNDDDNND | 13.57 | -0.42 |
| 2 | 41K5 | NKNNNKKKK | 13.05 | 0.42 | 3 | 50K5 | NKNNKKNKK | 13.57 | -0.42 |
| 2 | 41K6 | NKKKKNNKK | 13.05 | 0.42 | 4 | 27D4 | NNNDNDDND | 16.55 | -0.42 |
| 4 | 2K7 | KKKKNKNKK | 15.71 | 0.42 | 2 | 46K6 | NKKNKKNKK | 13.91 | -0.44 |

**Table 7.** First qPCR Screen – Amplification of Beta Actin.

| Plate | DNA name | Sequence (5'-3') | Ct (dR) | delta Ct | Plate | DNA name | Sequence (5'-3') | Ct (dR) | delta Ct |
|---|---|---|---|---|---|---|---|---|---|
| 2 | 17K4 | NKKNKNNNK | 13.06 | 0.41 | 4 | 6D7 | DDDDDNNDD | 16.57 | -0.44 |
| 3 | 53D6 | NNDDDDNDD | 12.75 | 0.4 | 1 | 8K7 | KKKNKNKKK | 16.99 | -0.45 |
| 2 | 22K7 | NKKKNKKKK | 13.07 | 0.4 | 3 | 20K6 | KKNNKKKNK | 13.62 | -0.47 |
| 4 | 28K4 | NNKKKNNNK | 15.74 | 0.39 | 3 | 22K6 | KNKKNKNKK | 13.64 | -0.49 |
| 4 | 33K4 | NNKNKKNNK | 15.74 | 0.39 | 1 | 36K6 | NKKKNNKKK | 17.03 | -0.49 |
| 4 | 63K5 | NNNKKKNKK | 15.74 | 0.39 | 2 | 11K6 | KKKKKNNNK | 13.97 | -0.5 |
| 3 | 17K5 | KNKKNKNNK | 12.77 | 0.38 | 3 | 49D6 | NDNDDDDND | 13.66 | -0.51 |
| 4 | 29K4 | NNKNKNNKK | 15.76 | 0.37 | 1 | 1D5 | DDNDNNNDD | 17.06 | -0.52 |
| 3 | 2D8 | DDDDDDNDD | 12.79 | 0.36 | 4 | 28D4 | NNDDDNNND | 16.65 | -0.52 |
| 4 | 59D5 | NNDNDNDDD | 15.77 | 0.36 | 3 | 21D4 | NDNNDDNND | 13.67 | -0.52 |
| 2 | 13D6 | DDNDDNNDD | 13.12 | 0.35 | 3 | 25D4 | NNNDNDNDD | 13.68 | -0.53 |
| 1 | 4K5 | KKKNNKNNK | 16.19 | 0.35 | 3 | 17K6 | KKKNKKNNK | 13.68 | -0.53 |
| 3 | 21D5 | DNNDNDDND | 12.81 | 0.34 | 2 | 9K6 | KKNKNKKNK | 14 | -0.53 |
| 3 | 26K4 | NNKNNKKNK | 12.81 | 0.34 | 4 | 58D5 | NNDDDNDND | 16.66 | -0.53 |
| 2 | 42K6 | NKKKKNKNK | 13.14 | 0.33 | 1 | 3D4 | DNNDNNNDD | 17.08 | -0.54 |
| 4 | 27K5 | KNNNKNKKK | 15.8 | 0.33 | 3 | 20K5 | KNKNNKKNK | 13.7 | -0.55 |
| 3 | 23D5 | DNDNDNNDD | 12.83 | 0.32 | 1 | 40K5 | NKNKNKKNK | 17.09 | -0.55 |
| 3 | 23K4 | NNKNNNKKK | 12.83 | 0.32 | 1 | 3D6 | DDDNNNDDD | 17.1 | -0.56 |
| 3 | 21K5 | KNNKNKKNK | 12.83 | 0.32 | 4 | 28D6 | DNDNDNDDD | 16.69 | -0.56 |
| 2 | 15K5 | KNKNNNKKK | 13.15 | 0.32 | 1 | 38K6 | NKKKNKKNK | 17.11 | -0.57 |
| 4 | 29D5 | DNNDDDNND | 15.82 | 0.31 | 1 | 39K5 | NKKNNKKNK | 17.12 | -0.58 |
| 4 | 25K6 | KNNKNKKKK | 15.82 | 0.31 | 4 | 34K4 | NNNKKKNNK | 16.73 | -0.6 |
| 3 | 50D6 | NDNNDDDDD | 12.85 | 0.3 | 4 | 30D4 | NNNDDNNDD | 16.74 | -0.61 |
| 2 | 12K6 | KKKNKNNKK | 13.17 | 0.3 | 1 | 40D5 | NDNDNDDND | 17.15 | -0.61 |
| 4 | 26K6 | KNKKKNNKK | 15.83 | 0.3 | 3 | 23K5 | KNKNKNNKK | 13.77 | -0.62 |
| 3 | 56K5 | NNNKNKKKK | 12.86 | 0.29 | 1 | 35K5 | NKNKNNKKK | 17.17 | -0.63 |
| 1 | 35D5 | NDNDNNDDD | 16.25 | 0.29 | 1 | 12K7 | KKNKKKNKK | 17.18 | -0.64 |
| 1 | 10K4 | KNNNKNNKK | 16.25 | 0.29 | 4 | 31K4 | NNKNKNKNK | 16.78 | -0.65 |
| 1 | 34D6 | DNNDDDDND | 16.25 | 0.29 | 4 | 6D3 | NNNDDNNND | 16.79 | -0.66 |
| 4 | 29D6 | DNNDDNDDD | 15.84 | 0.29 | 1 | 3D5 | DDNDNNDND | 17.2 | -0.66 |
| 3 | 17D5 | DNDDNDNND | 12.88 | 0.27 | 3 | 50D5 | NDNNDDNDD | 13.82 | -0.67 |
| 3 | 26K7 | NKKNKKKKK | 12.88 | 0.27 | 3 | 23K6 | KNKKNKKNK | 13.82 | -0.67 |
| 4 | 25D5 | DNDNDNDND | 15.87 | 0.26 | 1 | 6D4 | DNDNNDNND | 17.21 | -0.67 |
| 3 | 23D6 | DNDDNDDND | 12.89 | 0.26 | 1 | 14K7 | KKNKKKKNK | 17.21 | -0.67 |
| 3 | 22D5 | DNDDDNNND | 12.9 | 0.25 | 1 | 37D6 | NDDDNDNDD | 17.22 | -0.68 |
| 4 | 30K6 | KNKKKKNNK | 15.88 | 0.25 | 3 | 19K5 | KNNKNKNKK | 13.83 | -0.68 |
| 2 | 44D5 | NDNDDNNDD | 13.23 | 0.24 | 2 | 12D4 | DNNNDDNND | 14.16 | -0.69 |
| 3 | 48K6 | NKKNKKKNK | 12.91 | 0.24 | 2 | 14K5 | KNKKNNKNK | 14.16 | -0.69 |
| 3 | 25K7 | NKKKKKKNK | 12.91 | 0.24 | 1 | 32K6 | KNNKKKNKK | 17.23 | -0.69 |
| 2 | 18D4 | NDNDDNNND | 13.25 | 0.22 | 4 | 32K5 | NKKKNNNKK | 16.83 | -0.7 |
| 3 | 21K4 | NKNNKKNNK | 12.94 | 0.21 | 4 | 64K5 | NNKNKKKNK | 16.83 | -0.7 |
| 3 | 50K6 | NKNNKKKKK | 12.94 | 0.21 | 2 | 45D5 | NDDNDNDND | 14.18 | -0.71 |
| 1 | 8K4 | KNKNKNNNK | 16.34 | 0.2 | 4 | 26D6 | DNDDDNNDD | 16.84 | -0.71 |
| 2 | 11D5 | DDNNDNDND | 13.28 | 0.19 | 4 | 3D7 | DDDDNDDND | 16.84 | -0.71 |
| 2 | 46D5 | NDNDDNDND | 13.29 | 0.18 | 1 | 10D4 | DNNNDNNDD | 17.26 | -0.72 |
| 3 | 16K6 | KKNNKNKKK | 12.97 | 0.18 | 4 | 2D4 | DDNNDNNND | 16.86 | -0.73 |
| 2 | 14D5 | DNDDNNDND | 13.3 | 0.17 | 1 | 11D7 | DDDNDDNDD | 17.27 | -0.73 |
| 1 | 2K6 | KKKKNNKNK | 16.38 | 0.16 | 4 | 1D3 | DNNNDNNND | 16.95 | -0.82 |

**Table 7.** First qPCR Screen – Amplification of Beta Actin.

| Plate | DNA name | Sequence (5'-3') | Ct (dR) | delta Ct | Plate | DNA name | Sequence (5'-3') | Ct (dR) | delta Ct |
|---|---|---|---|---|---|---|---|---|---|
| 3 | 28K7 | NNKKKKKKK | 12.99 | 0.16 | 3 | 19K6 | KKNNKKNKK | 13.97 | -0.82 |
| 4 | 62D5 | NNDNDDNDD | 15.97 | 0.16 | 2 | 12K5 | KKNNKKNNK | 14.34 | -0.87 |
| 2 | 10D6 | DDNNNDDDD | 13.33 | 0.14 | 3 | 52D5 | NNDDNNDDD | 14.03 | -0.88 |
| 2 | 16K4 | NKNNNKNKK | 13.33 | 0.14 | 2 | 15D5 | DNDNNNDDD | 14.36 | -0.89 |
| 1 | 36D6 | NDDDNNDDD | 16.4 | 0.14 | 2 | 43K6 | NKKNKNKKK | 14.4 | -0.93 |
| 4 | 1K3 | KNNNKNNNK | 15.99 | 0.14 | 3 | 20D4 | NDNNDNDND | 14.09 | -0.94 |
| 2 | 41D6 | NDDDDNNDD | 13.34 | 0.13 | 2 | 47K6 | NKNKKKNKK | 14.41 | -0.94 |
| 2 | 21D7 | DNNDDDDDD | 13.34 | 0.13 | 3 | 18D5 | DNDNNDNDD | 14.11 | -0.96 |
| 1 | 39K6 | NKKNNKKKK | 16.41 | 0.13 | 1 | 12D7 | DDNDDDNDD | 17.5 | -0.96 |
| 4 | 33D4 | NNDNDDNND | 16.01 | 0.12 | 1 | 35D6 | DNNNDDDDD | 17.51 | -0.97 |
| 4 | 26D5 | DNNDDNDND | 16.01 | 0.12 | 3 | 22D4 | NNDDNNDND | 14.14 | -0.99 |
| 1 | 10K7 | KKKKKKNNK | 16.42 | 0.12 | 4 | 31D6 | DNDNDDNDD | 17.12 | -0.99 |
| 2 | 17D4 | NDDNDNNND | 13.36 | 0.11 | 1 | 33K5 | NKKKNNKNK | 17.54 | -1 |
| 3 | 20D6 | DDNNDDDND | 13.04 | 0.11 | 4 | 32D5 | NDDDNNNDD | 17.14 | -1.01 |
| 2 | 46D6 | NDDNDDNDD | 13.37 | 0.1 | 3 | 19D6 | DDNNDDNDD | 14.16 | -1.01 |
| 3 | 24D5 | DNNDDNNDD | 13.05 | 0.1 | 2 | 17K7 | KNKKKNKKK | 14.48 | -1.01 |
| 3 | 53K6 | NNKKKKNKK | 13.05 | 0.1 | 4 | 2K4 | KKNNKNNNK | 17.18 | -1.05 |
| 4 | 28D5 | DNDNDDNND | 16.03 | 0.1 | 3 | 52K5 | NNKKNNKKK | 14.22 | -1.07 |
| 1 | 1K6 | KKKKNNNKK | 16.44 | 0.1 | 1 | 5K4 | KNNKNNKNK | 17.63 | -1.09 |
| 4 | 28K6 | KNKNKNKKK | 16.04 | 0.09 | 4 | 31K6 | KNKNKKNKK | 17.23 | -1.1 |
| 2 | 45K5 | NKKNKNKNK | 13.39 | 0.08 | 4 | 24D6 | DNDNNDDDD | 17.24 | -1.11 |
| 1 | 4K4 | KNKNNNKNK | 16.46 | 0.08 | 1 | 1K5 | KKNKNNNKK | 17.67 | -1.13 |
| 4 | 25K5 | KNKNKNKNK | 16.05 | 0.08 | 1 | 9K4 | KNNKKNNNK | 17.68 | -1.14 |
| 4 | 3K3 | NNKNNNKNK | 16.06 | 0.07 | 1 | 10D7 | DDDDDDNND | 17.68 | -1.14 |
| 4 | 1D4 | DDNNNDNND | 16.08 | 0.05 | 1 | 4D5 | DDDNNDNND | 17.69 | -1.15 |
| 2 | 11D4 | DNNNDNDND | 13.42 | 0.05 | 3 | 22K5 | KNKKKNNNK | 14.35 | -1.2 |
| 3 | 20K4 | NKNNKNKNK | 13.11 | 0.04 | 4 | 1D7 | DDDDNNDDD | 17.34 | -1.21 |
| 2 | 44K5 | NKNKKNNKK | 13.43 | 0.04 | 1 | 3K4 | KNNKNNNKK | 17.76 | -1.22 |
| 2 | 47D5 | NDNNDNDDD | 13.44 | 0.03 | 4 | 27D6 | DNDDDNDND | 17.41 | -1.28 |
| 2 | 46K5 | NKNKKNKNK | 13.44 | 0.03 | 3 | 17D6 | DDDNDDNND | 14.46 | -1.31 |
| 1 | 36D5 | NDDDNDNND | 16.51 | 0.03 | 1 | 39D5 | NDDNNDDND | 17.9 | -1.36 |
| 4 | 7K7 | KKKKKNKNK | 16.1 | 0.03 | 1 | 9D7 | DDNDDNDDD | 17.95 | -1.41 |
| 3 | 51D5 | NDNNDDDND | 13.13 | 0.02 | 3 | 20D5 | DNDNNDDND | 14.7 | -1.55 |
| 4 | 24K6 | KNKNNKKKK | 16.11 | 0.02 | 4 | 29K5 | KNNKKKNNK | 17.68 | -1.55 |
| 2 | 10K6 | KKNNNKKKK | 13.46 | 0.01 | 3 | 52D6 | NNDDDNDDD | 14.84 | -1.69 |
| 2 | 44K6 | NKNKKNKKK | 13.46 | 0.01 | 3 | 51D6 | NNDDNDDDD | 14.96 | -1.81 |
| 1 | 1D6 | DDDDNNNDD | 16.53 | 0.01 | 4 | 56D6 | NNNDDDDDD | 18.51 | -2.38 |
| 4 | 60D5 | NNNDDNDDD | 16.12 | 0.01 | 2 | 16D5 | DNNDNNDDD | 16.85 | -3.38 |
| 2 | 45D6 | NDDDDDNND | 13.47 | 0 | 2 | 47D6 | NDNDDDNDD | 17.38 | -3.91 |
| 1 | 4D6 | DDNDNNDDD | 16.54 | 0 | 2 | 15D6 | DDNDDNDND | 19.11 | -5.64 |
| 3 | 22K4 | NNKKNNKNK | 13.15 | 0 | 2 | 42K5 | NKKKKNNNK | 24.63 | -11.16 |

The top 96 WTA products (shaded in Table 7) were then subjected to a second qPCR screen using primers for NM_001799 in a single plate. Table 8 presents the efficiency of amplification and Ct value for each reaction. The WTA products were ranked from lowest Ct to highest Ct.

**Table 8.** Second qPCR Screen – Amplification of NM_001799.

| DNA name | Sequence (5'-3') | Efficiency | Ct (dR) | DNA name | Sequence (5'-3') | Efficiency | Ct (dR) |
|---|---|---|---|---|---|---|---|
| 1K9 | KKKKKKKKK | 80.08% | 17.31 | 9K7 | KKNKKNKKK | 21.93% | 30.7 |
| 54D5 | NNDDNDDND | 57.92% | 17.54 | 14K4 | NKKNNNKNK | 53.68% | 31.19 |
| 32D4 | NNNDDNDND | 85.97% | 18.17 | 21K7 | KNNKKKKKK | 22.03% | 31.2 |
| 61D5 | NNDDDDNND | 79.33% | 18.49 | 2K5 | KKKNNNKNK | 35.00% | 32.12 |
| 34K5 | NKKNNNKKK | 46.90% | 18.62 | 62K5 | NNKNKKNKK | 11.41% | 32.14 |
| 1D8 | DDDDDNDDD | 96.17% | 18.82 | 9K5 | KKNKKNNNK | 46.20% | 32.16 |
| 6K7 | KKKKKNNKK | 69.67% | 18.84 | 17D7 | DNDDDNDDD | 38.26% | 32.43 |
| 1D9 | DDDDDDDDD | 83.07% | 19.03 | 18K7 | KNKKKKNKK | 47.58% | 32.61 |
| 5K6 | KKKKNKNNK | 84.51% | 19.05 | 5D5 | DDNDNDNND | 40.41% | 32.74 |
| 24D7 | NDDDDDNDD | 72.39% | 19.12 | 27D7 | NDNDDDDDD | 45.11% | 32.76 |
| 61K5 | NNKKKKNNK | 80.13% | 19.52 | 11K7 | KKKNKKNKK | 45.26% | 33.14 |
| 13D7 | DDDNDDDND | 81.62% | 19.54 | 57K5 | NNKKKNNKK | 54.33% | 33.28 |
| 25D7 | NDDDDDDND | 88.34% | 19.65 | 49K5 | NKNKKKNNK | 7.11% | 33.49 |
| 30K4 | NNNKKNNKK | 90.85% | 19.72 | 35K6 | KNNNKKKKK | 44.63% | 33.51 |
| 24K5 | KNNKKNNKK | 83.17% | 19.73 | 49D5 | NDNDDDNND | 40.46% | 33.67 |
| 54D6 | NNDDDDDND | 90.44% | 19.86 | 12D6 | DDDNDNNDD | 50.40% | 33.96 |
| 65D5 | NNNDDDDND | 62.60% | 19.98 | 8D6 | DDDNNDDND | 63.04% | 34.12 |
| 30D5 | DNNNDDNDD | 94.49% | 20.1 | 7D7 | DDDDDNDND | 43.04% | 34.12 |
| 4K7 | KKKNNKKKK | 75.22% | 20.13 | 64D5 | NNDNDDDND | 56.63% | 34.14 |
| 36K5 | NKKKNKNNK | 93.89% | 20.21 | 6K5 | KKNNNKNKK | 59.05% | 34.19 |
| 27K4 | NNNKNKKNK | 89.10% | 20.26 | 5K7 | KKNKNKKKK | 38.63% | 34.25 |
| 24K4 | NNKKNKNNK | 73.40% | 20.27 | 14D6 | DDDNDNDND | 54.21% | 34.37 |
| 54K5 | NNKKNKKNK | 86.13% | 20.43 | 29K6 | KNNKKNKKK | 4.11% | 36.29 |
| 12K4 | KNNNKKNNK | 104.05% | 20.47 | 15K7 | KKNNKKKKK | 8.82% | 37.83 |
| 8K6 | KKKNNKKNK | 100.82% | 20.61 | 13K4 | NKNKNNNKK | 6.22% | 39.83 |
| 4K3 | NNNKNKNNK | 87.94% | 20.64 | 40D6 | NDNDNDDDD | N/A | N/A |
| 25K4 | NNNKNKNKK | 70.83% | 20.75 | 42D6 | NDDDDNDND | N/A | N/A |
| 54K6 | NNKKKKKNK | 81.43% | 20.85 | 13D5 | DNDDNNNDD | N/A | N/A |
| 41D5 | NDNNNDDDD | 93.97% | 20.93 | 20D7 | DNDNDDDDD | N/A | N/A |
| 15K4 | NKNKNKNNK | 77.28% | 20.97 | 45K6 | NKKKKKNNK | N/A | N/A |
| 9D5 | DDNDDNNND | 85.41% | 21 | 14K6 | KKKNKNKNK | N/A | N/A |
| 27D5 | DNNNDNDDD | 70.42% | 21.15 | 48K5 | NKKNKKNNK | N/A | N/A |
| 24K7 | NKKKKKNKK | 74.31% | 21.16 | 48D5 | NDDNDDNND | N/A | N/A |
| 11K4 | KNNNKNKNK | 100.68% | 21.36 | 56K6 | NNNKKKKKK | N/A | N/A |
| 18K4 | NKNKKNNNK | 87.46% | 21.5 | 1K7 | KKKKNNKKK | N/A | N/A |
| 38K5 | NKNKNKNKK | 60.88% | 21.89 | 28K5 | KNKNKKNNK | N/A | N/A |
| 31D5 | DNNNDDDND | 85.38% | 21.92 | 60K5 | NNNKKNKKK | N/A | N/A |
| 19D7 | DNDDDDDND | 85.09% | 22.12 | 6K3 | NNNKKNNNK | N/A | N/A |
| 16D7 | DNDDNDDDD | 86.72% | 22.31 | 32K4 | NNNKKNKNK | N/A | N/A |

**Table 8.** Second qPCR Screen – Amplification of NM_001799.

| DNA name | Sequence (5'-3') | Efficiency | Ct (dR) | DNA name | Sequence (5'-3') | Efficiency | Ct (dR) |
|---|---|---|---|---|---|---|---|
| 16K7 | KNKKNKKKK | 93.38% | 22.44 | 30K5 | KNNNKKNKK | N/A | N/A |
| 21D6 | DNDDNNDDD | 84.90% | 22.69 | 5D7 | DDNDNDDDD | N/A | N/A |
| 3K7 | KKKKNKKNK | 72.22% | 22.78 | 63D5 | NNNDDDNDD | N/A | N/A |
| 55K6 | NNKNKKKKK | 92.61% | 22.97 | 16D6 | DDNNDNDDD | N/A | N/A |
| 19K4 | NKNNKNNKK | 76.80% | 23.6 | 48D6 | NDDNDDDND | N/A | N/A |
| 18D7 | DNDDDDNDD | 95.54% | 24.73 | 26D7 | NDDNDDDDD | N/A | N/A |
| 16K5 | KNNKNNKKK | 85.72% | 25.04 | 28D7 | NNDDDDDDD | N/A | N/A |
| 22D7 | NDDDNDDDD | 79.40% | 25.32 | 5D6 | DDDDNDNND | N/A | N/A |
| 13K6 | KKNKKNNKK | 69.91% | 27.65 | 8K5 | KKKNKNNNK | N/A | N/A |

The 48 WTA products with the lowest Cts (shaded in Table 8) were then qPCR amplified using primers for NM_ 001570- [22348]- 01 (screen 3a) and Human B2M Reference Gene (screen 3b), again in a single plate. Since the HB2M Reference gene was not particularly diagnostic, the WTA products were ranked on the basis of lowest Cts for NM_ 001570- [22348]- 01 (see Table 9) .

**Table 9.** Third qPCR Screen.

| | | NM_001570-[22348]-01 | | Human B2M Reference Gene | |
|---|---|---|---|---|---|
| DNA Name | Sequence (5'-3') | Efficiency | C(t) | Efficiency | C(t) |
| 61K5 | NNKKKKNNK | 89.73% | 20.62 | 104.79% | 15.96 |
| 24K7 | NKKKKKNKK | 78.90% | 20.64 | 92.38% | 16.63 |
| 3K7 | KKKKNKKNK | 88.21% | 21.08 | 87.42% | 15.8 |
| 11K4 | KNNNKNKNK | 98.83% | 21.13 | 82.51% | 16.02 |
| 25K4 | NNNKNKNKK | 70.12% | 21.15 | 52.40% | 16.72 |
| 41D5 | NDNNNDDDD | 90.41% | 21.49 | 81.38% | 16.33 |
| 16D7 | DNDDNDDDD | 91.62% | 21.49 | 90.46% | 16.96 |
| 54K6 | NNKKKKKNK | 74.28% | 22.69 | 93.76% | 16.04 |
| 15K4 | NKNKNKNNK | 77.62% | 22.96 | 63.86% | 16.89 |
| 6K7 | KKKKKNNKK | 82.93% | 23.27 | 106.46% | 15.47 |
| 55K6 | NNKNKKKKK | 73.93% | 24.07 | 101.32% | 17.43 |
| 19K4 | NKNNKNNKK | 65.68% | 25.39 | 96.74% | 17.19 |
| 8K6 | KKKNNKKNK | 57.01% | 27.69 | 76.50% | 16.27 |
| 27K4 | NNNKNKKNK | 67.81% | 29.01 | 85.25% | 16.99 |
| 13K6 | KKNKKNNKK | 44.87% | 32.06 | 77.82% | 17.06 |
| 18K4 | NKNKKNNNK | 40.16% | 32.56 | 98.27% | 16.43 |
| 21D6 | DNDDNNDDD | 56.41% | 32.89 | 72.69% | 15.72 |
| 9D5 | DDNDDNNND | 51.55% | 33.09 | 112.16% | 15.96 |
| 30K4 | NNNKKNNKK | 57.26% | 33.3 | 76.53% | 16.61 |
| 25D7 | NDDDDDDND | 78.56% | 33.6 | 88.70% | 16.72 |

**Table 9.** Third qPCR Screen.

| | | NM_001570-[22348]-01 | | Human B2M Reference Gene | |
|---|---|---|---|---|---|
| DNA Name | Sequence (5'-3') | Efficiency | C(t) | Efficiency | C(t) |
| 4K3 | NNNKNKNNK | 56.92% | 33.8 | 67.80% | 16.29 |
| 24K5 | KNNKKNNKK | 34.58% | 33.84 | 89.81% | 15.81 |
| 24K4 | NNKKNKNNK | 61.81% | 33.93 | 66.70% | 15.72 |
| 54D6 | NNDDDDDND | 39.75% | 33.98 | 93.20% | 15.81 |
| 54K5 | NNKKNKKNK | 63.39% | 34.13 | 85.45% | 17.44 |
| 54D5 | NNDDNDDND | 62.24% | 34.16 | 75.84% | 15.94 |
| 16K7 | KNKKNKKKK | 40.51% | 34.26 | 79.08% | 18.25 |
| 36K5 | NKKKNKNNK | 50.88% | 34.38 | 108.12% | 15.96 |
| 1D8 | DDDDDNDDD | 37.02% | 34.5 | 76.79% | 15.31 |
| 4K7 | KKKNNKKKK | 58.18% | 35.23 | 104.15% | 15.6 |
| 5K6 | KKKKNKNNK | 37.82% | 35.25 | 83.70% | 16.31 |
| 61D5 | NNDDDDNND | 61.24% | 35.49 | 68.12% | 15.45 |
| 16K5 | KNNKNNKKK | 44.56% | 35.71 | 81.32% | 16.19 |
| 1K9 | KKKKKKKKK | 46.60% | 36.66 | 80.65% | 16.01 |
| 34K5 | NKKNNNKKK | 48.57% | 37.47 | 89.07% | 17.38 |
| 32D4 | NNNDDNDND | 27.18% | 39.28 | 98.38% | 16.09 |
| 65D5 | NNNDDDDND | N/A | N/A | 76.74% | 14.21 |
| 13D7 | DDDNDDDND | N/A | N/A | 50.90% | 14.83 |
| 38K5 | NKNKNKNKK | N/A | N/A | 54.94% | 15.63 |
| 1D9 | DDDDDDDDD | N/A | N/A | 104.78% | 15.64 |
| 22D7 | NDDDNDDDD | N/A | N/A | 58.80% | 15.7 |
| 30D5 | DNNNDDNDD | N/A | N/A | 56.15% | 15.76 |
| 31D5 | DNNNDDDND | N/A | N/A | 84.80% | 16.11 |
| 24D7 | NDDDDDNDD | N/A | N/A | 82.34% | 16.23 |
| 19D7 | DNDDDDDND | N/A | N/A | 70.53% | 16.28 |
| 18D7 | DNDDDDNDD | N/A | N/A | 84.99% | 16.31 |
| 12K4 | KNNNKKNNK | N/A | N/A | 87.09% | 16.93 |
| 27D5 | DNNNDNDDD | N/A | N/A | 96.08% | 17.04 |

The 14 WTA products with the lowest Cts (shaded in Table 9), as well as those amplified with 1 K9 and 1D9 primers, were subjected to the fourth qPCR screen (i.e., screens 4a-4f). The 1 K9 and 1D9 primers were carried along because current WGA and WTA primers comprise a K9 region and D9 was the first generation attempt at increasing degeneracy relative to K. As before, all reactions were conducted in a single 96-well plate. Table 10 presents the efficiency of amplification and Ct values for each reaction. Of the 16 interrupted N library synthesis primers, five were dropped from further consideration due to either a combination of high Ct for NBM_003234 qPCR and/or a lower number of possible WTA amplicons from the human genome. The remaining 11 primers were sorted by Ct for each of the six qPCRs of the fourth screen. At each sorting, a rank number was assigned (1= highest rank, 11 lowest) to each primer. The resulting rank numbers were summed for each primer design (see Table 11). The rank number sums were sorted to provide a ranking of the most successful primers. The process revealed that 9 of the 11 interrupted N primers had similar abilities to provide significant quantities of amplifiable template for the fourth screen.

**Table 10.** Fourth qPCR Screen.

| DNA name | Sequence (5'-3') | ATP6V1G1 | | CTNNB1 | | GAPDH | | GPI | | NM 000942 | | NM 003234 | |
|---|---|---|---|---|---|---|---|---|---|---|---|---|---|
| | | Eff(%) | C(t)1 | Eff(%) | C(t)2 | Eff(%) | C(t)3 | Eff(%) | C(t)4 | Eff(%) | C(t)5 | Eff(%) | C(t)6 |
| 8K6 | KKKNNKKNK | 84.47 | 19.35 | 83.60 | 18.62 | 88.78 | 15.84 | 90.48 | 18.31 | 97.87 | 17.41 | 83.50 | 20.87 |
| 27K4 | NNNKNKKNK | 49.20 | 20.19 | 63.10 | 19.17 | 81.44 | 14.09 | 84.73 | 18.71 | 86.54 | 16.79 | 77.68 | 22.2 |
| 25K4 | NNNKNKNKK | 69.36 | 22.42 | 66.44 | 18.28 | 73.52 | 15.21 | 62.90 | 18.24 | 91.64 | 17.46 | 58.02 | 21.19 |
| 19K4 | NKNNKNNKK | 62.45 | 21.83 | 83.07 | 19.91 | 56.60 | 15.64 | 82.17 | 18.51 | 70.15 | 17.09 | 71.07 | 20.3 |
| 11K4 | KNNNKNKNK | 33.47 | 25.21 | 87.30 | 19.04 | 73.08 | 15.66 | 78.07 | 17.86 | 88.31 | 18.21 | 64.93 | 20.33 |
| 1D9 | DDDDDDDDD | 61.76 | 18.93 | 74.91 | 19.16 | 72.22 | 14.71 | 69.12 | 19.08 | 109.4 | 18.65 | 8.90 | 30.82 |
| 3K7 | KKKKNKKNK | 61.35 | 19.81 | 98.62 | 20.67 | 91.77 | 15.99 | 80.76 | 19.34 | 105.5 | 16.77 | 76.88 | 20.55 |
| 15K4 | NKNKNKNNK | 59.48 | 23.21 | 77.49 | 19.78 | 83.23 | 15.38 | 57.47 | 18.97 | 80.35 | 17.04 | 75.72 | 20.94 |
| 61K5 | NNKKKKNNK | 82.20 | 20.29 | 75.98 | 19.16 | 76.76 | 14.89 | 79.66 | 19.56 | 85.31 | 17.48 | 48.52 | 32.1 |
| 41D5 | NDNNNDDDD | 94.84 | 20.81 | 76.62 | 20.16 | 83.12 | 15.98 | 84.88 | 18.83 | 98.27 | 19.03 | 84.51 | 21.26 |
| 1K9 | KKKKKKKKK | 86.38 | 23.0 | 66.86 | 24.69 | 79.44 | 17.21 | 72.72 | 19.87 | 78.99 | 19.21 | N/A | N/A |
| 55K6 | NNKNKKKKK | 77.20 | 21.52 | 74.61 | 19.56 | 65.61 | 16.03 | 72.48 | 18.64 | 83.75 | 17.27 | N/A | N/A |
| 24K7 | NKKKKKNKK | 84.59 | 22.12 | 71.78 | 20.23 | 75.70 | 17.81 | 61.66 | 17.29 | 59.52 | 17.34 | 21.89 | 27.98 |
| 54K6 | NNKKKKKNK | 70.42 | 23.57 | 69.26 | 18.07 | 63.88 | 17.43 | 68.88 | 19.92 | 72.48 | 18 | 1.93 | 35.48 |
| 6K7 | KKKKKNNKK | 41.50 | 26.69 | 55.10 | 18.35 | 77.54 | 16.28 | 53.17 | 20.63 | 96.60 | 17.1 | 14.08 | 27.67 |
| 16D7 | DNDDNDDDD | 15.56 | 27.37 | 70.17 | 19.69 | 66.02 | 15.19 | 61.02 | 18.68 | 67.09 | 18.55 | N/A | N/A |

**Table 11.** Ranking of Primers After Fourth qPCR Screen.

| DNA Name | Sequence (5'-3') | Sort 1 | Sort 2 | Sort 3 | Sort 4 | Sort 5 | Sort 6 | Sort Sums |
|---|---|---|---|---|---|---|---|---|
| 8K6 | KKKNNKKNK | 2 | 2 | 8 | 3 | 5 | 4 | 24 |
| 27K4 | NNNKNKKNK | 4 | 6 | 1 | 5 | 2 | 8 | 26 |
| 25K4 | NNNKNKNKK | 8 | 1 | 4 | 2 | 6 | 6 | 27 |
| 19K4 | NKNNKNNKK | 7 | 8 | 6 | 4 | 4 | 1 | 30 |
| 11K4 | KNNNKNKNK | 11 | 3 | 7 | 1 | 8 | 2 | 32 |
| 1D9 | DDDDDDDDD | 1 | 4 | 2 | 8 | 9 | 9 | 33 |
| 3K7 | KKKKNKKNK | 3 | 10 | 10 | 9 | 1 | 3 | 36 |
| 15K4 | NKNKNKNNK | 10 | 7 | 5 | 7 | 3 | 5 | 37 |
| 61K5 | NNKKKKNNK | 5 | 5 | 3 | 10 | 7 | 10 | 40 |
| 41D5 | NDNNNDDDD | 6 | 9 | 9 | 6 | 10 | 7 | 47 |
| 1K9 | KKKKKKKKK | 9 | 11 | 11 | 11 | 11 | 11 | 64 |

In parallel to these experiments, the number of possible human transcriptome derived amplicons resulting from each of the 384 primer designs was determined bioinformatically. Of the nine sequences identified in the four qPCR screens, eight were ranked according the number of potential amplicons produced from the human transcriptome (1 D9 was dropped from further evaluation because of amplicon loss in qPCR screen 3). This analysis identified five sequences (i.e., 11 K4, 15K4, 19K4, 25K4, and 27K4), with each producing approximately one million amplicons from the human transcriptome.

**Example 4. Additional Screens to Identify the Exemplary Primers.**

(a) amplify degraded RNA

A desirable aspect of the WTA process is the ability to amplify degraded RNAs. The top 9 interrupted N library synthesis primers from screen 4 (see Table 11) plus 1 K9 and 1 D9 primers were used to amplify NaOH-digested RNAs. Briefly, to 5 μg of liver total RNA in 20 μl of water was added 20 μl of 0.1 M NaOH. The mixture was incubated at 25° C for 0 minutes to 12 minutes. At times 0, 1, 2, 3, 4, 6, 8 and 12 minutes, 2 μl aliquots were removed and quenched in 100 μl of 10 mM Tris-HCl, pH 7. WTAs were performed similar to those described above. That is, for library synthesis: 2 μl NaOH-digested RNA, 2 μl of 5 μM of a library synthesis primer, heat 70° C for 5 min, add 4 μl of 2X MMLV buffer, 10 U/μl MMLV, and 1 mM dNTPs; incubate at 42° C for 15 minutes; and dilute with 30 μl of $H_2O$. For amplification: 8 μl of diluted library, 12 μl of amplification mix (2X SYBR® Green JUMPSTART™ Taq READYMIX™ and 5 μM universal primer). Analysis of the WTA products by agarose gel electrophoresis revealed that all except 1 K9 and 1 D9 library synthesis primers produced relatively high levels of WTA amplicons (see Figure 3).

(b) WTA screens

Another desirable feature of an ideal library synthesis primer is minimal or no primer dimer formation. The 11 interrupted N primers used in the above- described degraded RNA experiment were subjected to WTA except in the absence of template. Library synthesis was also performed in the presence of either MMLV reverse transcriptase or both MMLV and Klenow exo- minus DNA polymerase. Library amplification was also catalyzed by either JUMPSTART™ Taq or KLENTAQ® (Sigma- Aldrich) . Figure 4 reveals that synthesis with the combination of MMLV and Klenow exo-minus DNA polymerase and amplification with JUMPSTART™ Taq DNA polymerase provided higher levels of amplicons. Furthermore, this experiment revealed that primer dimer formation was not a significant problem with any of these 11 library synthesis primers (see gels without RNA template) .

(c) final selection

The preferred library synthesis primers would be primers that provide a maximum number of amplicons without a loss of sensitivity due to intermolecular and/or intramolecular primer specific interactions (e.g., primer dimers). Thus, the qPCR culling experiments, the primer dimer analyses, and the bioinformatics analyses revealed five interrupted N sequences that satisfied these requirements. That is, five sequences (i.e., 11 K4, 15K4, 19K4, 25K4, and 27K4) that when used for library synthesis yielded WTA products that provided amplifiable template for all qPCR screens, yielded minimal quantities of primer dimers in the absence of template, and were capable of producing at least a million WTA amplicons from the human transcriptome.

Although one of these preferred sequences could be randomly selected for use as a library synthesis primer, it was reasoned that a mixture of some or all of these sequences may be preferable. Conversely, a mixture of some or all of them could also permit detrimental primer-primer interactions. These possibilities were investigated by performing WTA in which the libraries were synthesized using individual primers or a mixture of some or all five of the preferred primers, as well as primers comprising K9, D9, or N9 sequences. Potentially detrimental interactions were examined by performing library synthesis with high concentrations of the library synthesis primer(s). Thus, standard WTA reactions library were performed in the presence of 10 μM, 2 μM, 0.4 μM or 0.08 μM of the library synthesis primers. WTA products were assayed by agarose gel electrophoresis. WTA products were also analyzed with SYBR® green mediated qPCR amplification using NBM_001570 primers (SEQ ID NOs:33 and 34).

As shown in Figure 5, the yield of WTA products was dependent upon the concentration of the library synthesis primer(s). Furthermore, evidence of primer dimers was present only at the highest concentration of the N9 primer (see N lanes). The possibility of primer interactions was estimated by calculating the delta Cts from qPCR for each primer/primer combination. That is, the difference in Ct between 10 μM and 2 μM, between 2 μM and 0.4 μM, and between 0.4 μM and 0.08 μM. A negative delta Ct was interpreted as a detrimental primer-primer interaction. It was found that 15K4 alone had modest detrimental interactions at high concentrations, while almost any combination that contained 15K4 and 19K4 was also significantly detrimental. Additionally, the combination of 19K4 and 25K4 also showed a negative interaction.

**Table 12.** qPCR using individual primers or primer combinations.

| Primers* | Ct(1)** | Ct(2)** | Ct(3)** | Ct(4)** | ΔCt(2-1) | ΔCt(3-2) | ΔCt(4-3) |
|---|---|---|---|---|---|---|---|
| 11, 15, 19, 25, 27 | 22.11 | 22.63 | 23.61 | 25.02 | 0.52 | 0.98 | 1.41 |
| 15, 19, 25, 27 | 22.44 | 24.72 | 22.91 | 26.61 | 2.28 | -1.81 | 3.7 |
| 11, 19, 25, 27 | 21.7 | 22.73 | 24.28 | 25.97 | 1.03 | 1.55 | 1.69 |
| 11, 15, 25, 27 | 23.06 | 23.26 | 23.34 | 28.91 | 0.2 | 0.08 | 5.57 |
| 11, 15, 19, 27 | 23.58 | 23.68 | 24.16 | 24.35 | 0.1 | 0.48 | 0.19 |
| 11, 15, 19, 25 | 24.73 | 23.34 | 26.0 | 25.82 | -1.39 | 2.66 | -0.18 |
| 11,15,19 | 23.78 | 22.82 | 24.51 | 28.36 | -0.96 | 1.69 | 3.85 |
| 11,15,25 | 23.18 | 23.73 | 28.05 | 29.4 | 0.55 | 4.32 | 1.35 |
| 11,15,27 | 22.73 | 23.03 | 23.07 | 27.99 | 0.3 | 0.04 | 4.92 |
| 11,15,27 | 22.28 | 23.7 | 22.25 | 27.15 | 1.42 | -1.45 | 4.9 |
| 11,19,25 | 19.67 | 22.47 | 22.68 | 27.62 | 2.8 | 0.21 | 4.94 |
| 11,19,27 | 18.67 | 20.09 | 25.11 | 25.49 | 1.42 | 5.02 | 0.38 |
| 11,25,27 | 22.1 | 23.45 | 19.93 | 22.12 | 1.35 | -3.52 | 2.19 |
| 15,19,25 | 24.21 | 21.51 | 22.65 | 25.06 | -2.7 | 1.14 | 2.41 |
| 15,25,27 | 23.42 | 23.71 | 23.65 | 24.96 | 0.29 | -0.06 | 1.31 |
| 19,25,27 | 23.42 | 22.36 | 23.21 | 27.16 | -1.06 | 0.85 | 3.95 |
| 11 | 23.17 | 24.09 | 22.8 | 27.86 | 0.92 | -1.29 | 5.06 |
| 15 | 23.5 | 22.06 | 23.32 | 24.78 | -1.44 | 1.26 | 1.46 |
| 19 | 23.73 | 23.79 | 23.82 | 28.97 | 0.06 | 0.03 | 5.15 |
| 25 | 23.25 | 23.0 | 24.0 | 24.8 | -0.25 | 1.0 | 0.8 |
| 27 | 23.67 | 23.27 | 23.74 | 27.17 | -0.4 | 0.47 | 3.43 |
| K | 22.69 | 22.27 | 22.3 | 27.98 | -0.42 | 0.03 | 5.68 |
| D | 23.74 | 23.73 | 24.43 | 28.33 | -0.01 | 0.7 | 3.9 |

(continued)

| Primers* | Ct(1)** | Ct(2)** | Ct(3)** | Ct(4)** | ΔCt(2-1) | ΔCt(3-2) | ΔCt(4-3) |
|---|---|---|---|---|---|---|---|
| N | 24.29 | 24.78 | 21.59 | 24.98 | 0.49 | -3.19 | 3.39 |
| * 11 = 11 K4 primer, 15 = 15K4 primer, 19 = 19K4 primer, 25 = 25K4 primer, 27 = 27K4 primer.<br>** 1 = 10 μM, 2 = 2 μM, 3 = 0.4 μM, 4 = 0.08 μM. | | | | | | | |

Aside from any possible negative impact the combination of primers might have, their ability to prime divergent sequences was probed by pair-wise alignment of the individual sequences. The 5 interrupted N were aligned so as to have the greatest number of Ns overlapping among the primers (see Table 13). Furthermore, pair-wise K-N mismatches were tallied for each possible pairing (see Table 14).

**Table 13.** Pair-wise Alignment.

| Name | Sequence (5'-3') | | | | | | | | | | |
|---|---|---|---|---|---|---|---|---|---|---|---|
| 11K4 | K | N | N | N | K | N | K | N | K | | |
| 15K4 | | N | K | N | K | N | K | N | N | K | |
| 19K4 | | | N | K | N | N | K | N | N | K | K |
| 25K4 | | N | N | N | K | N | K | N | K | K | |
| 27K4 | | N | N | N | K | N | K | K | N | K | |

**Table 14.** Mismatches.

| | 11K4 | 15K4 | 19K4 | 25K4 | 27K4 |
|---|---|---|---|---|---|
| 11K4 | | 2 | 3 | 0 | 2 |
| 15K4 | | | 2 | 2 | 2 |
| 19K4 | | | | 3 | 3 |
| 25K4 | | | | | 2 |
| 27K4 | | | | | |

These analyses revealed that the greatest divergence within this set of primers was with 11 K4, 19K4 and 27K4 primers. Thus, maximum priming divergence with minimal primer interaction occurred with the mixture of primers comprising 11 K4 (i.e., KNNNKNKNK), 19K4 (i.e., NKNNKNNKK), and 27K4 (i.e., NNNKNKKNK).

## Claims

1. A plurality of degenerate oligonucleotides, each oligonucleotide comprising the formula $N_mX_pZ_q$, wherein N, X and Z are degenerate nucleotides as follows:

   N is a 4-fold degenerate nucleotide selected from adenosine (A), cytidine (C), guanosine (G), or thymidine/uridine (T/U);
   X is a 3-fold degenerate nucleotide selected from B, D, H, or V, wherein B is C, G, or T/U; D is A, G, or T/U; H is A, C, or T/U; and V is A, C, or G;
   Z is a 2-fold degenerate nucleotide selected from K, M, R, or Y, wherein K is G or T/U; M is A or C; R is A or G; and Y is C or T/U; wherein
   m, p, and q are integers, with m being either 0 or from 2 to 20, and p and q each being from 0 to 20; provided, however, that either no two integers are 0 or both m and q are 0, and further provided that oligonucleotides comprising N have at least one X or Z residue separating two of the N residues; and

   wherein when no integers are 0 and the plurality of oligonucleotides comprise formula $N_mX_pZ_q$ the oligonucleotides range from 4 to 60 nucleotides in length; when m is 0 and the plurality of oligonucleotides comprise formula $X_pZ_q$, the sum total of p and q is at least 6; when p is 0 and the plurality of oligonucleotides comprise formula $N_mZ_q$, the sum total of m and q is at least 6; when q is 0 and the plurality of oligonucleotides comprise formula $N_mX_p$, the sum total of m and p is at least 6; and when both m and q are 0 and the formula of the oligonucleotides is $X_p$, p is from 8 to 20.

2. The plurality of oligonucleotides of claim 1, wherein one integer is 0 and the formula of the oligonucleotides is $N_mX_p$, $N_mZ_q$, or $X_pZ_q$, wherein m is from 2 to 8, p and q are each from 1 to 8, and the sum total of the two integers is 9.

3. The plurality of oligonucleotides of claim 2, wherein the oligonucleotides comprising N have no more than three consecutive N residues.

4. The plurality of oligonucleotides of claim 3, wherein each of the oligonucleotide primers has a sequence selected from KNNNKNKNK, NKNNKNNKK, or NNNKNKKNK.

5. The plurality of oligonucleotides of any one of the preceding claims, wherein each oligonucleotide further comprises a sequence of non-degenerate nucleotides at the 5' end, the non-degenerate sequence being constant among the plurality of oligonucleotides, and the constant non-degenerate sequence being about 14 nucleotides to about 24 nucleotides in length.

6. A method for amplifying a population of target nucleic acids, the method comprising:

   (a) contacting the population of target nucleic acids with a plurality of oligonucleotide primers which are a plurality of oligonucleotides according to any one of the preceding claims to form a plurality of nucleic acid-primer duplexes,
   (b) replicating the plurality of nucleic acid-primer duplexes to create a library of replicated strands, wherein the amount of replicated strands exceeds the amount of target nucleic acids used in step (a), indicating amplification of the population of target nucleic acids.

7. The method of claim 6, wherein replication of the target nucleic acid is catalyzed by an enzyme selected from Exo-Minus Klenow DNA polymerase, Exo-Minus T7 DNA polymerase, Phi29 DNA polymerase, Bst DNA polymerase, Bca polymerase, Vent DNA polymerase, 9°Nm DNA polymerase, MMLV reverse transcriptase, AMV reverse transcriptase, HIV reverse transcriptase, a variant thereof, or a mixture thereof.

8. The method of claim 6 or claim 7, wherein the target nucleic acid is fragmented by a method selected from mechanical, chemical, thermal, or enzymatic means prior to contact with the plurality of oligonucleotide primers.

9. The method of any one of claims 6 to 8, further comprising amplifying the library of replicated strands using a polymerase chain reaction.

**10.** The method of claim 9, wherein the amplification utilizes at least one primer selected from a primer having substantial complementary to a constant region at the ends of the replicated strands, or a pair of primers.

**11.** The method of any one of claims 8 to 10 , wherein the amplified library is labeled by incorporation of at least one modified nucleotide during the polymerase chain reaction, the modified nucleotide selected from a fluorescently-labeled nucleotide, aminoallyl-dUTP, bromo-dUTP, or a digoxigenin-labeled nucleotide.

**12.** The method of any one of claims 8 to 11, wherein the target nucleic acid is DNA, the replication is catalyzed by Exo-Minus Klenow DNA polymerase, and the amplification is catalyzed by Taq DNA polymerase; or wherein the target nucleic acid is RNA, the plurality of oligonucleotide primers further comprises an oligo dT primer, the replication is catalyzed by MMLV reverse transcriptase and/or Exo-Minus Klenow DNA polymerase and in particular MMLV reverse transcriptase, and the amplification is catalyzed by Taq DNA polymerase.

**13.** A kit for amplifying a target nucleic acid, the kit comprising:

(a) a plurality of oligonucleotides according to any one of claims 1 to 5; and
(b) a replicating enzyme, which is optionally selected from Exo-Minus Klenow DNA polymerase, Exo-Minus T7 DNA polymerase, Phi29 DNA polymerase, Bst DNA polymerase, Bca polymerase, Vent DNA polymerase, 9°Nm DNA polymerase, MMLV reverse transcriptase, AMV reverse transcriptase, HIV reverse transcriptase, a variant thereof, or mixture thereof.

**14.** The kit of claim 13, further comprising one or more of

(i) an oligo dT primer in the plurality of oligonucleotide primers; and
(ii) a thermostable DNA polymerase selected from Taq DNA polymerase, a Pfu DNA polymerase, or a combination thereof.

**Patentansprüche**

**1.** Mehrzahl von degenerierten Oligonucleotiden, wobei jedes Oligonucleotid die Formel $N_mX_pZ_q$ umfasst, wobei es sich bei N, X und Z um die folgenden degenerierten Nucleotide handelt:

N ist ein 4-fach degeneriertes Nucleotid, ausgewählt aus Adenosin (A), Cytidin (C), Guanosin (G) oder Thymidin/Uridin (T/U);
X ist ein 3-fach degeneriertes Nucleotid, ausgewählt aus B, D, H oder V, wobei B die Bedeutung C, G oder T/U hat; D die Bedeutung A, G oder T/U hat; H die Bedeutung A, C oder T/U hat; und V die Bedeutung A, C oder G hat;
Z ist ein 2-fach degeneriertes Nucleotid, ausgewählt aus K, M, R oder Y, wobei K die Bedeutung G oder T/U hat; M die Bedeutung A oder C hat; R die Bedeutung A oder G hat; und Y die Bedeutung C oder T/U hat;
wobei m, p und q ganze Zahlen bedeuten, wobei m entweder 0 oder 2 bis 20 bedeutet und p und q jeweils 0 bis 20 bedeuten; jedoch mit der Maßgabe, dass entweder nicht zwei ganze Zahlen den Wert 0 haben oder m und q beide den Wert 0 haben, und mit der weiteren Maßgabe, dass Oligonucleotide, die N umfassen, mindestens einen X- oder Z-Rest aufweisen, der zwei der N-Reste voneinander trennt; und
wobei dann, wenn keine der ganzen Zahlen den Wert 0 hat und die Mehrzahl der Oligonucleotide die Formel $N_mX_pZ_q$ umfasst, die Oligonucleotide eine Länge von 4 bis 60 Nucleotiden aufweisen; wenn m den Wert 0 hat und die Mehrzahl der Oligonucleotide die Formel $X_pZ_q$ umfasst, die Summe von p und q mindestens 6 beträgt; wenn p den Wert 0 hat und die Mehrzahl der Oligonucleotide die Formel $N_mZ_q$ umfasst, die Summe von m und q mindestens 6 beträgt; wenn q den Wert 0 hat und die Mehrzahl der Oligonucleotide die Formel $N_mX_p$ umfasst, die Summe von m und p mindestens 6 beträgt; und wenn m und q beide den Wert 0 haben und die Oligonucleotide die Formel $X_p$ aufweisen, p einen Wert von 8 bis 20 hat.

**2.** Mehrzahl von Oligonucleotiden nach Anspruch 1, wobei eine ganze Zahl den Wert 0 hat und die Oligonucleotide die Formeln $N_mX_p$, $N_mZ_q$ oder $X_pZ_q$ aufweisen, wobei m einen Wert von 2 bis 8 hat und p und q jeweils einen Wert von 1 bis 8 haben und die Summe der beiden ganzen Zahlen 9 beträgt.

**3.** Mehrzahl von Oligonucleotiden nach Anspruch 2, wobei die Oligonucleotide, die N umfassen, nicht mehr als drei aufeinander folgende N-Reste aufweisen.

**4.** Mehrzahl von Oligonucleotiden nach Anspruch 3, wobei jeder der Oligonucleotid-Primer eine Sequenz aufweist die ausgewählt ist aus KNNNKNKNK, NKNNKNNKK oder NNNKNKKNK.

**5.** Mehrzahl von Oligonucleotiden nach einem der vorstehenden Ansprüche, wobei jedes Oligonucleotid ferner eine Sequenz von nichtdegenerierten Nucleotiden am 5'-Ende aufweist, wobei die nicht-degenerierte Sequenz unter der Mehrzahl von Oligonucleotiden konstant ist und die konstante nicht-degenerierte Sequenz eine Länge von etwa 14 Nucleotiden bis etwa 24 Nucleotiden aufweist.

**6.** Verfahren zur Amplifikation einer Population von Zielnucleinsäuren, wobei das Verfahren folgendes umfasst:

(a) das Kontaktieren der Population von Zielnucleinsäuren mit einer Mehrzahl von Oligonucleotid-Primern, bei der es sich um eine Mehrzahl von Oligonucleotiden nach einem der vorstehenden Ansprüche handelt, um eine Mehrzahl von Nucleinsäure-Primer-Duplexen zu bilden,
(b) das Replizieren der Mehrzahl von Nucleinsäure- Primer- Duplexen, um eine Bibliothek von replizierten Strängen zu erzeugen, wobei die Anzahl von replizierten Strängen die Anzahl der in Stufe (a) eingesetzten Zielnucleinsäuren übersteigt, was eine Amplifikation der Population von Zielnucleinsäuren anzeigt.

**7.** Verfahren nach Anspruch 6, wobei die Replikation der Zielnucleinsäure durch ein Enzym katalysiert wird, das ausgewählt ist aus Exo- Minus- Klenow- DNA- Polymerase, Exo- Minus- T7- DNA- Polymerase, Phi29- DNA- Polymerase, Bst- DNA- Polymerase, Bca- Polymerase, Vent- DNA- Polymerase, 9°Nm- DNA- Polymerase, reverse MMLV- Transcriptase, reverse AMV- Transcriptase, reverse HIV- Transcriptase, einer Variante davon oder einem Gemisch davon.

**8.** Verfahren nach Anspruch 6 oder 7, wobei die Zielnucleinsäure durch ein Verfahren fragmentiert wird, das ausgewählt ist aus mechanischen, chemischen, thermischen oder enzymatischen Mitteln, bevor der Kontakt mit der Mehrzahl von Oligonucleotid-Primern erfolgt.

**9.** Verfahren nach einem der Ansprüche 6 bis 8, ferner umfassend das Amplifizieren der Bibliothek von replizierten Strängen unter Anwendung einer Polymerase-Kettenreaktion.

**10.** Verfahren nach Anspruch 9, wobei die Amplifikation sich mindestens eines Primers bedient, der ausgewählt wird aus einem Primer, der im Wesentlichen komplementär mit einer konstanten Region an den Enden der replizierten Stränge ist, oder einem Paar von Primern.

**11.** Verfahren nach einem der Ansprüche 8 bis 10, wobei die amplifizierte Bibliothek durch Einbau von mindestens einem modifizierten Nucleotid während der Polymerase-Kettenreaktion markiert wird, wobei das modifizierte Nucleotid ausgewählt wird aus einem fluoreszierend markierten Nucleotid, Aminoallyl-dUTP, Brom-dUTP oder einem Digoxigenin-markierten Nucleotid.

**12.** Verfahren nach einem der Ansprüche 8 bis 11, wobei es sich bei der Zielnucleinsäure um DNA handelt, die Replikation durch Exo- Minus- Klenow- DNA- Polymerase katalysiert wird und die Amplifikation durch Taq- DNA- Polymerase katalysiert wird; oder wobei es sich bei der Zielnucleinsäure um RNA handelt, die Mehrzahl von Oligonucleotid-Primern ferner einen oligo- dT- Primer umfasst, die Replikation durch reverse MMLV- Transcriptase und/ oder Exo-Minus- Klenow- DNA- Polymerase und insbesondere durch reverse MMLV- Transcriptase katalysiert wird und die Amplifikation durch Taq- DNA- Polymerase katalysiert wird.

**13.** Kit zum Amplifizieren einer Zielnucleinsäure, wobei das Kit folgendes umfasst:

(a) eine Mehrzahl von Oligonucleotiden nach einem der Ansprüche 1 bis 5;
(b) ein Replikationsenzym, das optional ausgewählt ist aus Exo- Minus- Klenow- DNA- Polymerase, Exo- Minus- T7- DNA- Polymerase, Phi29- DNA- Polymerase, Bst- DNA- Polymerase, Bca- Polymerase, Vent- DNA- Polymerase, 9°Nm- DNA- Polymerase, reverser MMLV- Transcriptase, reverser AMV- Transcriptase, reverser HIV- Transcriptase, einer Variante davon oder einem Gemisch davon.

**14.** Kit nach Anspruch 13, ferner umfassend einen oder mehrere der Bestandteile

(i) ein oligo-dT-Primer in der Mehrzahl der Oligonucleotid-Primer und
(ii) eine thermostabile DNA- Polymerase, die ausgewählt ist aus Tac- DNA- Polymerase, einer Pfu- DNA-Polymerase oder einer Kombination davon.

## Revendications

1. Pluralité d'oligonucléotides dégénérés, chaque oligonucléotide comprenant la formule NmXpZq, dans laquelle N, X et Z sont des nucléotides dégénérés, comme suit:

   N est un nucléotide dégénéré 4 fois choisi parmi l'adénosine (A), la cytidine (C), la guanosine (G), ou de la thymidine / uridine (T/U);
   X est un nucléotide dégénéré 3 fois choisi parmi B, D, H, ou V, dans lequel B est C, G, ou T / U ; D est A, G, ou T / U ; H est A, C ou T / U ; et V est A, C, ou G ;
   Z est un nucléotidique 2 fois dégénéré choisi parmi K, M, R, ou Y, dans lequel K est G ou T/U ; M est A ou C; R est A ou G ; et Y est C ou T/U ; dans lequel
   m, p, et q sont des nombres entiers, avec m étant soit 0 soit de 2 à 20, et p et q étant chacun de 0 à 20 ; à condition, toutefois, que soit deux entiers ne sont pas égaux 0 ou que m et q sont égaux à 0, et en outre à condition que les oligonucléotides comprenant N aient au moins un résidu X ou Z séparant deux des résidus N ; et

   dans lequel, lorsque aucun des nombres entiers est égal à 0 et la pluralité d'oligonucléotides comprend la formule $N_mX_pZ_q$ les oligonucléotides vont de 4 à 60 nucléotides de longueur; lorsque m est égal à 0 et la pluralité d'oligonucléotides comprend la formule XpZq, la somme totale de p et q est au moins 6; lorsque p est 0 et la pluralité d'oligonucléotides comprend la formule $N_mZ_q$, la somme totale de m et q est au moins 6; lorsque q est 0 et la pluralité d'oligonucléotides comprend la formule $N_mX_p$, la somme totale de m et p est au moins 6 ; et quand à la fois m et q sont 0 et la formule des oligonucléotides est $X_p$, p est de 8 à 20.

2. Pluralité d'oligonucléotides selon la revendication 1, où un nombre entier est 0 et la formule des oligonucléotides est $N_mX_p$, $N_mZ_q$ ou $X_pZ_q$, dans lequel m est de 2 à 8, p et q sont chacun de 1 à 8, et la somme totale des deux entiers est neuf.

3. Pluralité d'oligonucléotides selon la revendication 2, où les oligonucléotides comprenant N n'ont pas plus de trois résidus N consécutifs.

4. Pluralité d'oligonucléotides selon la revendication 3, où chacunes des amorces oligonucléotidiques a une séquence choisie dans KNNNKNKNK, NKNNKNNKK, ou NNNKNKKNK.

5. Pluralité d'oligonucléotides selon l'une quelconque des revendications précédentes, où chaque oligonucléotide comprend en outre une séquence de nucléotides non-dégénérés à l'extrémité 5', la séquence non-dégénérée étant constante parmi la pluralité d'oligonucléotides, et la séquence constante non-dégénérée étant environ 14 nucléotides à environ 24 nucléotides de long.

6. Procédé d'amplification d'une population d'acides nucléiques cibles, le procédé comprenant:

   (a) la mise en contact de la population d'acides nucléiques cibles avec une pluralité d'amorces oligonucléotidiques qui sont une pluralité d'oligonucléotides selon l'une quelconque des revendications précédentes pour former une pluralité de duplex acide nucléique-amorce,
   (b) la réplication de la pluralité de duplex acide nucléique-amorce pour créer une librairie de brins répliqués, où la quantité de brins répliqués dépasse la quantité d'acides nucléiques cibles utilisée à l'étape (a), indiquant l'amplification de la population d'acides nucléiques cibles.

7. Procédé selon la revendication 6, dans lequel la réplication de l'acide nucléique cible est catalysée par une enzyme choisie parmi l'ADN polymérase Exo-Minus Klenow, l'ADN polymérase T7 Exo-Minus, l'ADN polymérase Phi29, l'ADN polymérase Bst, la polymérase Bca, l'ADN polymerase Vent, l'ADN polymérase 9°Nm, la transcriptase inverse du MMLV, la transcriptase inverse du AMV, la transcriptase inverse du VIH, un variant de celles-ci, ou un mélange de celles-ci.

8. Procédé selon la revendication 6 ou la revendication 7, dans lequel l'acide nucléique cible est fragmenté par un procédé choisi parmi des moyens mécaniques, chimiques, thermiques, ou enzymatiques avant contact avec la pluralité d'amorces oligonucléotidiques.

**9.** Procédé selon l'une quelconque des revendications 6 à 8, comprenant en outre l'amplification de la librairie des brins répliqués en utilisant une amplification en chaîne par polymérase.

**10.** Procédé selon la revendication 9, dans lequel l'amplification utilise au moins une amorce choisie parmi une amorce ayant une complémentarité substantielle d'une région constante aux extrémités des brins répliqués, ou une paire d'amorces.

**11.** Procédé selon l'une quelconque des revendications 8 à 10, dans lequel la librairie amplifiée est marquée par incorporation d'au moins un nucléotide modifié au cours de l'amplification en chaîne par polymérase, le nucléotide modifié choisi parmi un nucléotide marqué par fluorescence, un aminoallyle-dUTP, un bromo-dUTP, ou un nucléotide marqué à la digoxigénine.

**12.** Procédé selon l'une quelconque des revendications 8 à 11, dans lequel l'acide nucléique cible est de l'ADN, la réplication est catalysée par l'ADN polymérase Exo-Minus Klenow, et l'amplification est catalysée par l'ADN polymérase Taq ; ou dans lequel l'acide nucléique cible est un ARN, la pluralité d'amorces oligonucléotidiques comprend en outre une amorce oligo dT, la réplication est catalysée par la transcriptase inverse du MMLV et/ou l'ADN polymérase exo-Minus Klenow et en particulier la transcriptase inverse du MMLV, et l'amplification est catalysée par l'ADN polymérase Taq.

**13.** Kit pour amplifier un acide nucléique cible, le kit comprenant:

(a) une pluralité d'oligonucléotides selon l'une quelconque des revendications 1 à 5 ; et
(b) une enzyme de réplication, qui est éventuellement choisie parmi l'ADN polymérase Exo-Minus Klenow, l'ADN polymérase Exo-Minus T7, l'ADN polymérase Phi29, l'ADN polymérase Bst, la Bca polymérase, l'ADN polymerase Vent, l'ADN polymérase 9°Nm, la transcriptase inverse du MMLV, la transcriptase inverse du AMV, la transcriptase inverse du VIH, une variante de celles-ci, ou d'un mélange de celles-ci.

**14.** Kit selon la revendication 13, comprenant en outre un ou plusieurs de :

(i) une amorce oligo dT dans la pluralité d'amorces oligonucléotidiques ; et
(ii) une ADN polymérase thermostable choisie parmi l'ADN polymérase Taq, une ADN polymérase Pfu, ou une combinaison de celles-ci.

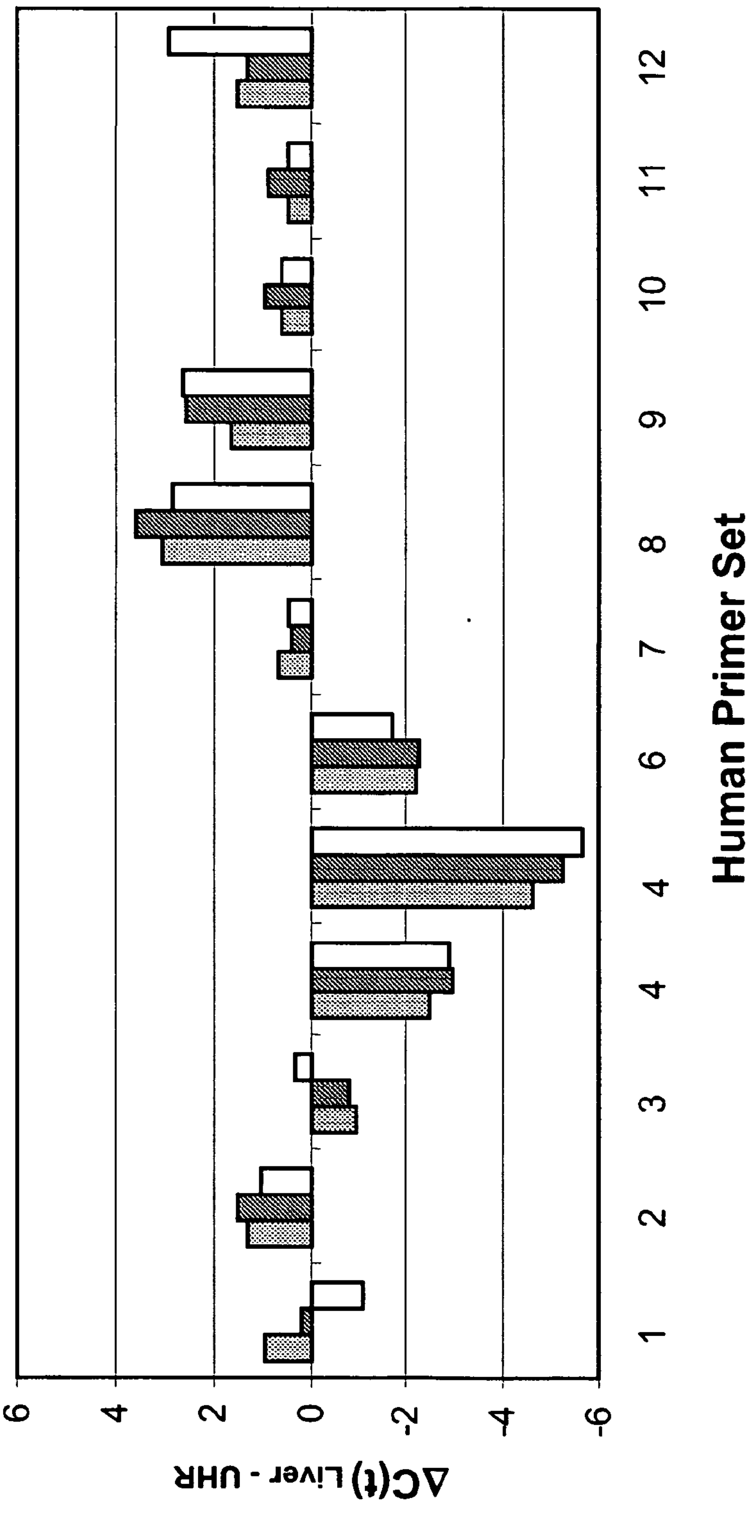


FIG. 1

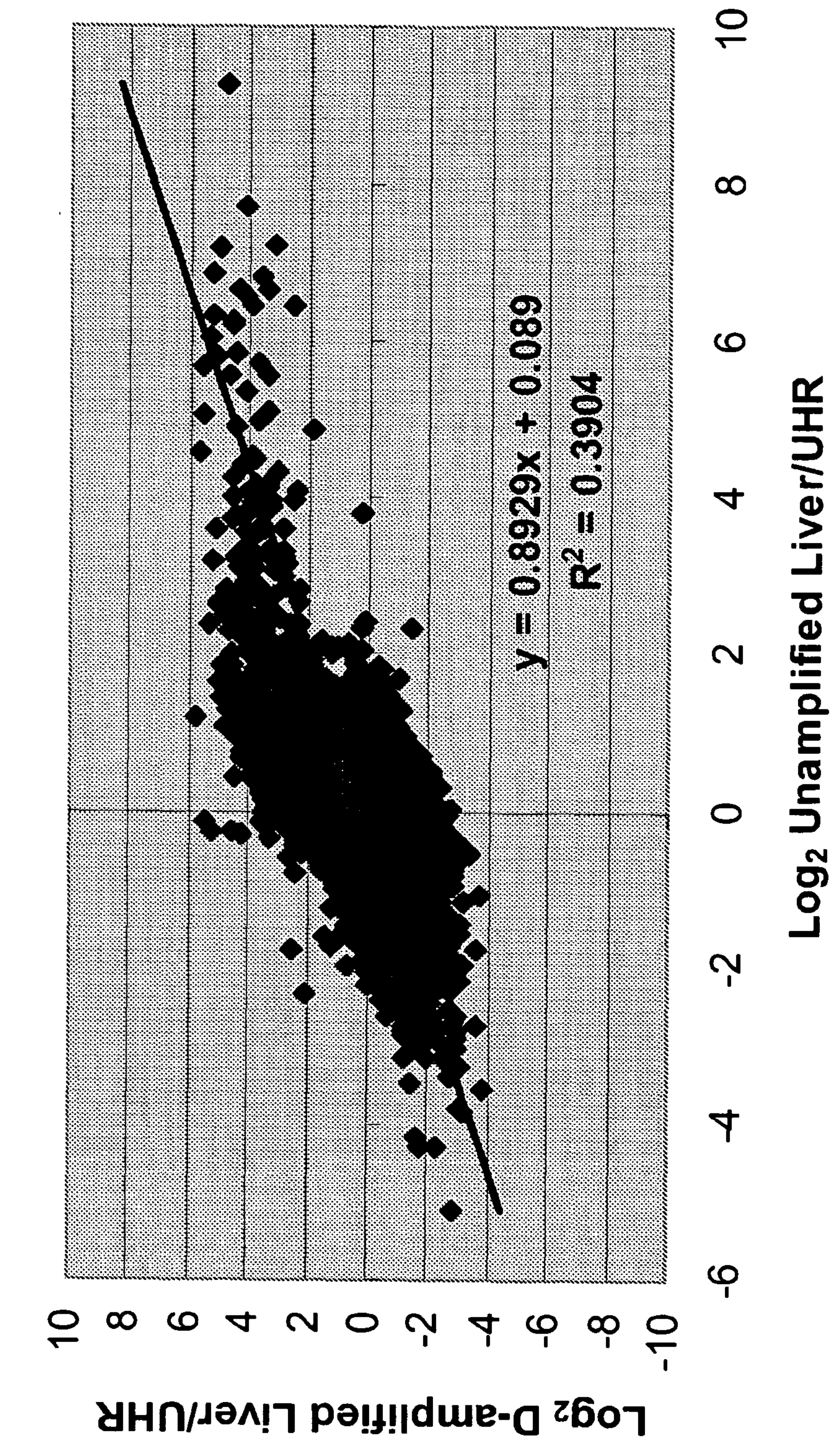
y = 0.8929x + 0.089
$R^2$ = 0.3904
Log$_2$ Unamplified Liver/UHR
Log$_2$ D-amplified Liver/UHR
-6
-4
-2
0
2
4
6
8
10
-10
-8
-6
-4
-2
0
2
4
6
8
10

FIG. 2A

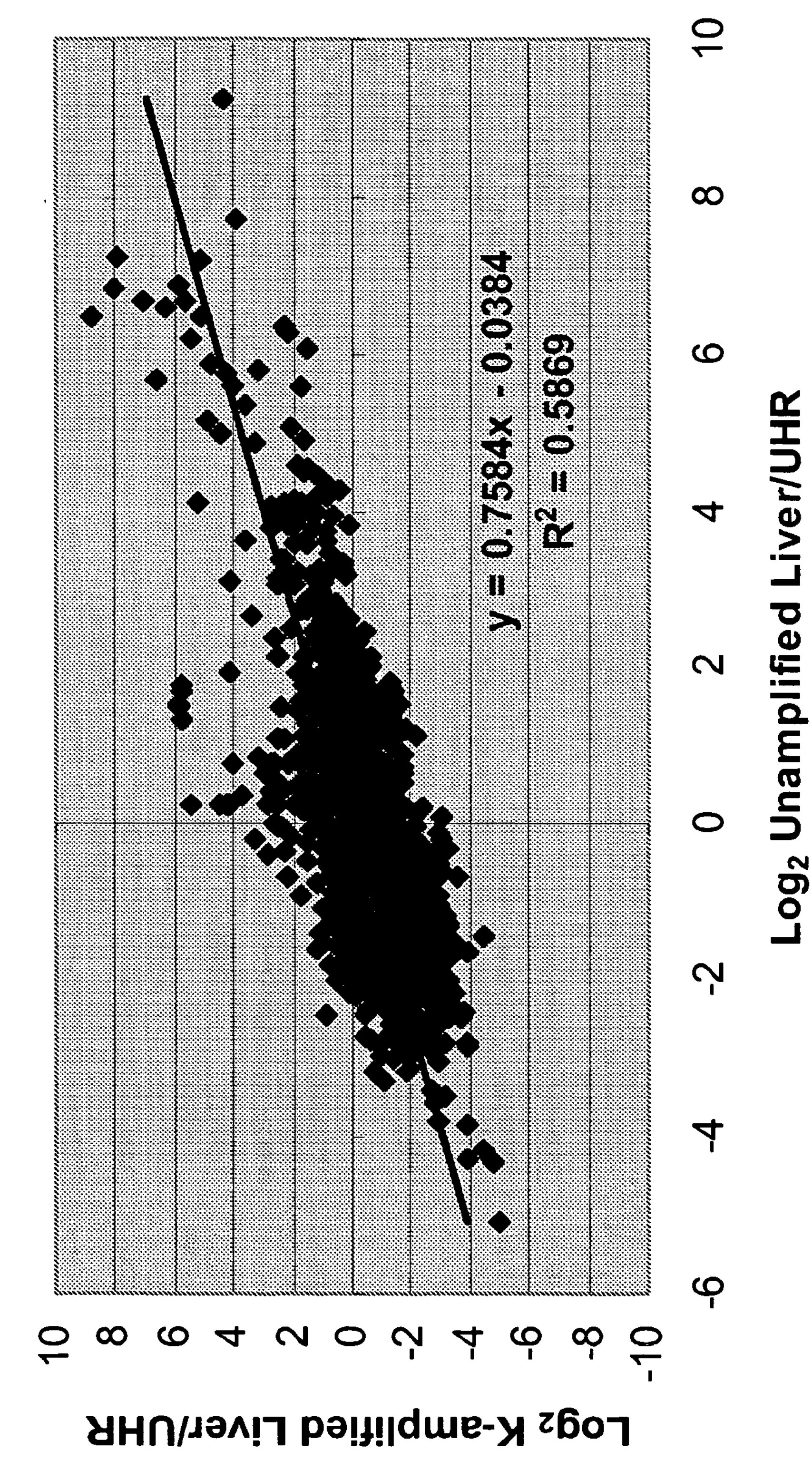
y = 0.7584x - 0.0384
$R^2$ = 0.5869
Log$_2$ Unamplified Liver/UHR
Log$_2$ K-amplified Liver/UHR
-6
-4
-2
0
2
4
6
8
10
-10
-8
-6
-4
-2
0
2
4
6
8
10

FIG. 2B

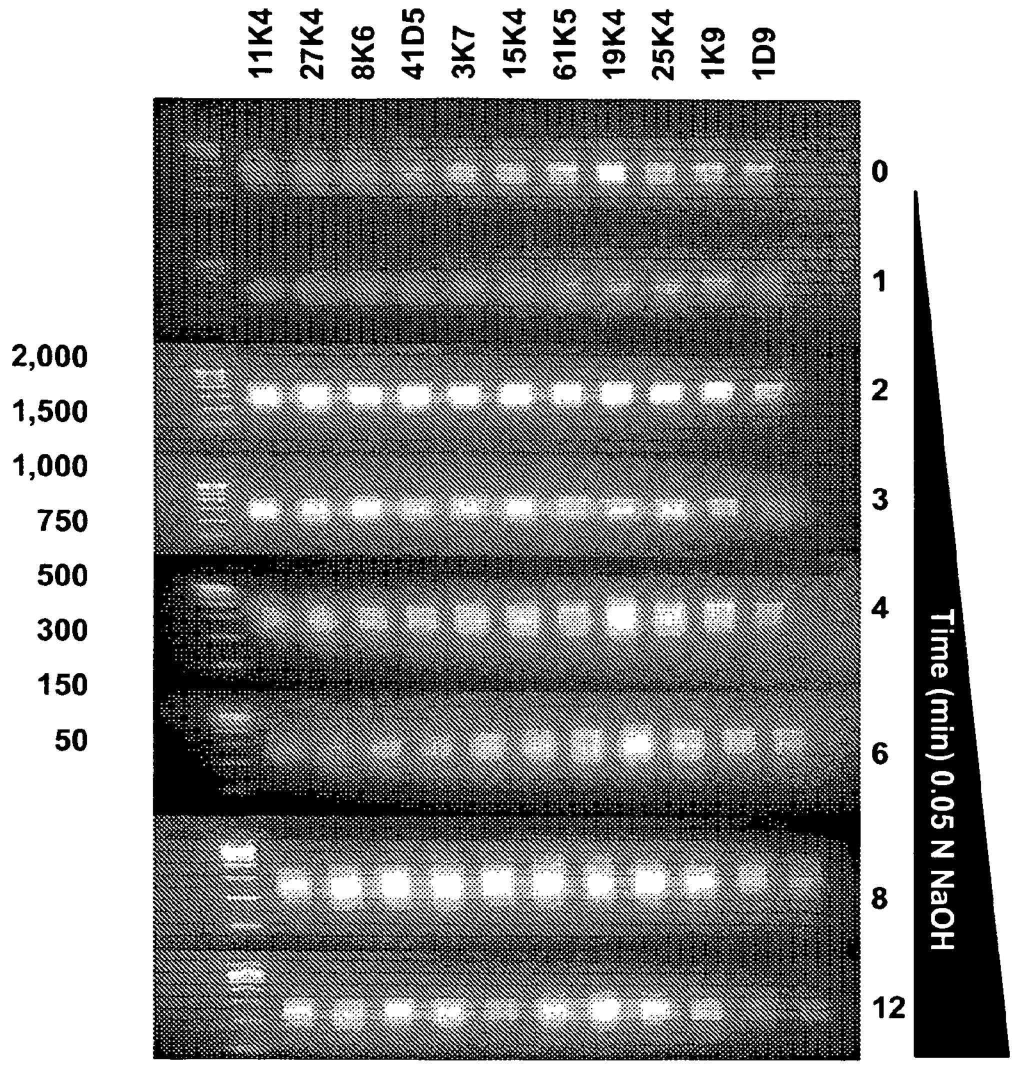
11K4
27K4
8K6
41D5
3K7
15K4
61K5
19K4
25K4
1K9
1D9
2,000
1,500
1,000
750
500
300
150
50
0
1
2
3
4
6
8
12
Time (min) 0.05 N NaOH

**FIG. 3**

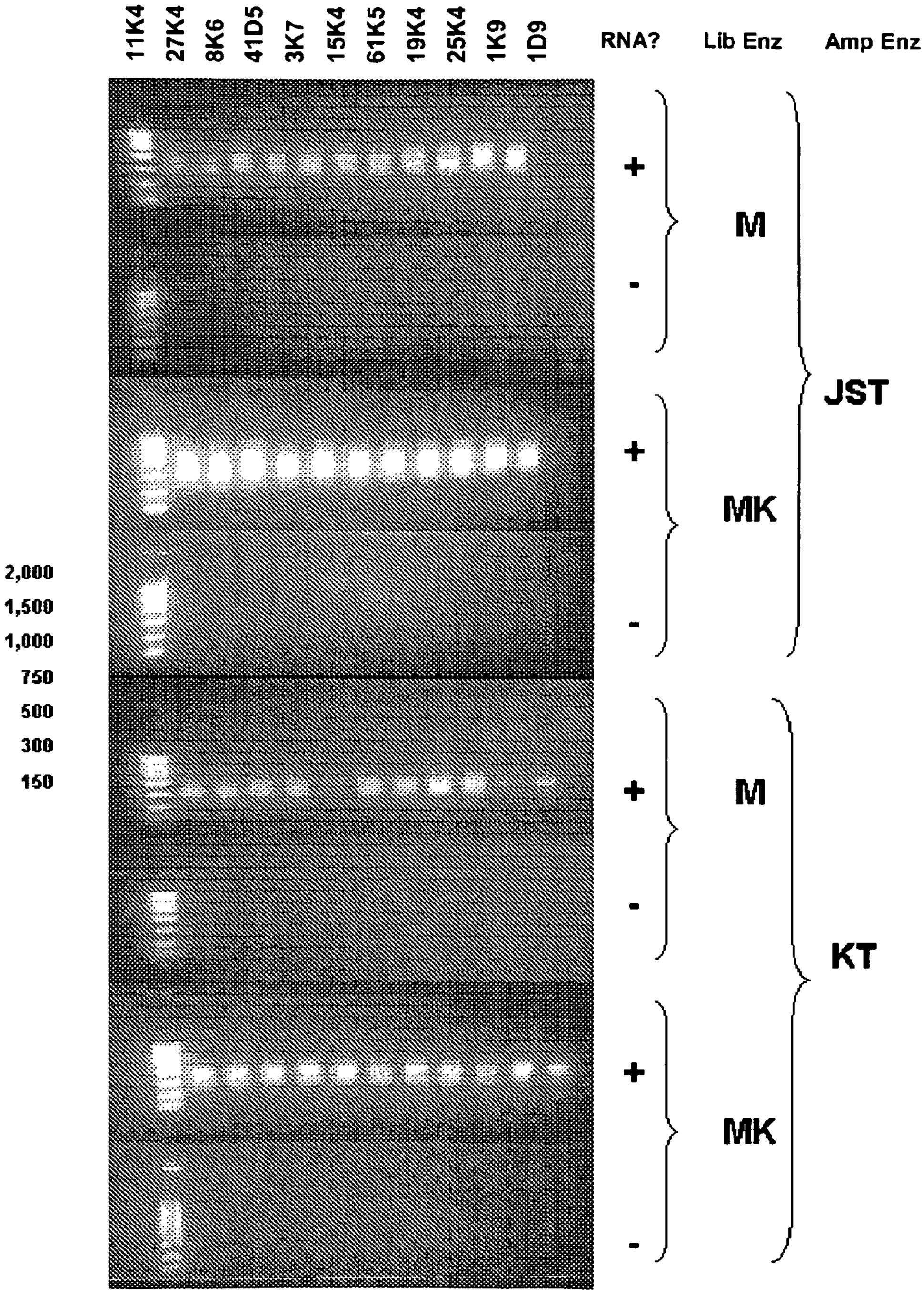
11K4
27K4
8K6
41D5
3K7
15K4
61K5
19K4
25K4
1K9
1D9
RNA?
Lib Enz
Amp Enz
2,000
1,500
1,000
750
500
300
150
+
-
M
MK
JST
KT

FIG. 4

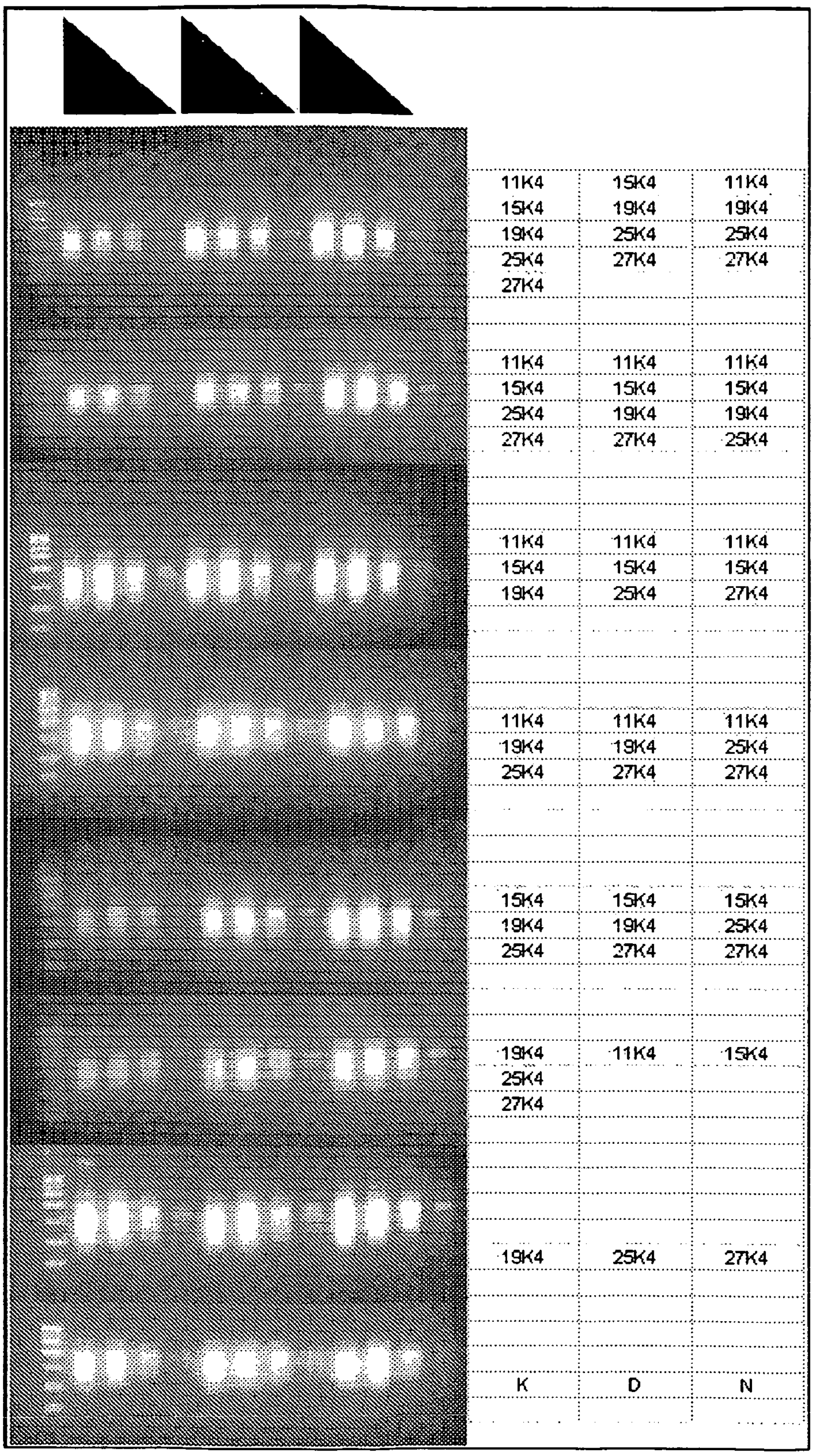
11K4 15K4 19K4 25K4 27K4
15K4 19K4 25K4 27K4
11K4 19K4 25K4 27K4
11K4 15K4 25K4 27K4
11K4 15K4 19K4 27K4
11K4 15K4 19K4 25K4
11K4 15K4 19K4
11K4 15K4 25K4
11K4 15K4 27K4
11K4 19K4 25K4
11K4 19K4 27K4
11K4 25K4 27K4
15K4 19K4 25K4
15K4 19K4 27K4
15K4 25K4 27K4
19K4 25K4 27K4
11K4
15K4
19K4
25K4
27K4
K
D
N

FIG. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004081225 A
- US 20050202490 A
- US 11872272 B


### Non-patent literature cited in the description


- **CHEUNG et al.** *Proc. Natl. Acad. Sci. USA,* 1996, vol. 93, 14676-14679
- **SAMBROOK et al.** Molecular Cloning: A Laboratory Manual. Cold Spring Harbor Press, 1989

SEQUENCE LISTING

<110> SIGMA-ALDRICH CO. WARD, BRIAN HEUERMANN, KENNETH E.

<120> DEGENERATE OLIGONUCLEOTIDES AND THEIR USES

<130> 47497-129823

<150> US 11/872,272<151> 2007-10-15

<160> 48

<170> PatentIn version 3.5

<210> 1
<211> 44
<212> DNA

<213> Artificial

<220>
<223> HOMO SAPIENS

<400> 1
gtaggttgag gataggaggg ttaggttttt ttttttttt tttt 44

<210> 2
<211> 34
<212> DNA
<213> Artificial

<220>
<223> HOMO SAPIENS

<400> 2
gtaggttgag gataggaggg ttaggddddd dddd 34

<210> 3
<211> 25
<212> DNA
<213> Artificial

<220>
<223> HOMO SAPIENS

<400> 3
gtaggttgag gataggaggg ttagg 25

<210> 4
<211> 20
<212> DNA
<213> Artificial

<220>
<223> HOMO SAPIENS

<400> 4
tgcttagacc cgtagtttcc 20

<210> 5
<211> 21
<212> DNA
<213> Artificial

<220>
<223> HOMO SAPIENS

<400> 5
cttgacaaaa tgctgtgttc c 21

<210> 6
<211> 20
<212> DNA
<213> Artificial

<220>

<223> HOMO SAPIENS

<400> 6
cgtttaattc tgtggccagg 20

<210> 7
<211> 20
<212> DNA
<213> Artificial

<220>
<223> HOMO SAPIENS

<400> 7
agccaagtac cccgactacg 20

<210> 8
<211> 25
<212> DNA
<213> Artificial

<220>
<223> HOMO SAPIENS

<400> 8
tgttaacaat ttgcataaca aaagc 25

<210> 9
<211> 25
<212> DNA
<213> Artificial

<220>
<223> HOMO SAPIENS

<400> 9
tgattaattt gcgagactaa ctttg 25

<210> 10
<211> 23
<212> DNA
<213> Artificial

<220>
<223> HOMO SAPIENS

<400> 10
gtttcgaatc ccaggaatta agc 23

<210> 11
<211> 23
<212> DNA
<213> Artificial

<220>
<223> HOMO SAPIENS

<400> 11

cacaatcagc aacaaaatca tcc 23

<210> 12
<211> 20
<212> DNA
<213> Artificial

<220>
<223> HOMO SAPIENS

<400> 12
gcaaacaaag catgcttcaa 20

<210> 13
<211> 20
<212> DNA
<213> Artificial

<220>
<223> HOMO SAPIENS

<400> 13
ttctcccagc tttgagacgt 20

<210> 14
<211> 25
<212> DNA
<213> Artificial

<220>
<223> HOMO SAPIENS

<400> 14
tatttaaaat gtgggcaaga tatca 25

<210> 15
<211> 25
<212> DNA
<213> Artificial

<220>
<223> HOMO SAPIENS

<400> 15
tggtgtaaat aaagaccttg ctatc 25

<210> 16
<211> 21
<212> DNA
<213> Artificial

<220>
<223> HOMO SAPIENS

<400> 16
tttgttactt gctaccctga g 21

<210> 17

<211> 21
<212> DNA
<213> Artificial

<220>
<223> HOMO SAPIENS

<400> 17
caaccatcat cttccacagt c 21

<210> 18
<211> 20
<212> DNA
<213> Artificial

<220>
<223> HOMO SAPIENS

<400> 18
agaccacacc agaaaccctg 20

<210> 19
<211> 22
<212> DNA
<213> Artificial

<220>
<223> HOMO SAPIENS

<400> 19
gaattttggt ttcttgcttt gg 22

<210> 20
<211> 23
<212> DNA
<213> Artificial

<220>
<223> HOMO SAPIENS

<400> 20
ccagggttcg aatctcagtc tta 23

<210> 21
<211> 23
<212> DNA
<213> Artificial

<220>
<223> HOMO SAPIENS

<400> 21
gatttctaaa cttacggccc cac 23

<210> 22
<211> 25
<212> DNA
<213> Artificial

<220>
<223> HOMO SAPIENS

<400> 22
aaagagtgtc ttgtcttgac ttatc 25

<210> 23
<211> 25
<212> DNA
<213> Artificial

<220>
<223> HOMO SAPIENS

<400> 23
ttatctgagc ccttaatagt aaatc 25

<210> 24
<211> 20
<212> DNA
<213> Artificial

<220>
<223> HOMO SAPIENS

<400> 24
aatcaaaagg ccaacagtgg 20

<210> 25
<211> 21
<212> DNA
<213> Artificial

<220>
<223> HOMO SAPIENS

<400> 25
ttcagtgtta atggagccag g 21

<210> 26
<211> 20
<212> DNA
<213> Artificial

<220>
<223> HOMO SAPIENS

<400> 26
tctcagagca gagtttgggc 20

<210> 27
<211> 19
<212> DNA
<213> Artificial

<220>
<223> HOMO SAPIENS

<400> 27
cctgcacttg gacctgacc 19

<210> 28
<211> 22
<212> DNA
<213> Artificial

<220>
<223> HOMO SAPIENS

<400> 28
gtggtgtgtt gggtgtgttt gg 22

<210> 29
<211> 20
<212> DNA
<213> Artificial

<220>
<223> HOMO SAPIENS

<400> 29
ctggaacggt gaaggtgaca 20

<210> 30
<211> 23
<212> DNA
<213> Artificial

<220>
<223> HOMO SAPIENS

<400> 30
aagggacttc ctgtaacaat gca 23

<210> 31
<211> 25
<212> DNA
<213> Artificial

<220>
<223> HOMO SAPIENS

<400> 31
ctcagttggt gtgcccaaag tttca 25

<210> 32
<211> 24
<212> DNA
<213> Artificial

<220>
<223> HOMO SAPIENS

<400> 32
tagcagagtt acttctaagg gttc 24

<210> 33
<211> 21
<212> DNA
<213> Artificial

<220>
<223> HOMO SAPIENS

<400> 33
gatcatcctg aactggaaac c 21

<210> 34
<211> 24
<212> DNA
<213> Artificial

<220>
<223> HOMO SAPIENS

<400> 34
gcctttctta cagaagctgc caaa 24

<210> 35
<211> 22
<212> DNA
<213> Artificial

<220>
<223> HOMO SAPIENS

<400> 35
cggcatcttc aaacctccat ga 22

<210> 36
<211> 29
<212> DNA
<213> Artificial

<220>
<223> HOMO SAPIENS

<400> 36
gcctgccgtg tgaaccatgt gactttgtc 29

<210> 37
<211> 20
<212> DNA
<213> Artificial

<220>
<223> HOMO SAPIENS

<400> 37
tggacaacct cttggctttt 20

<210> 38
<211> 20
<212> DNA

<213> Artificial

<220>
<223> HOMO SAPIENS

<400> 38
taaaatgcca ctccacagca 20

<210> 39
<211> 20
<212> DNA
<213> Artificial

<220>
<223> HOMO SAPIENS

<400> 39
ttgaaaatcc agcgtggaca 20

<210> 40
<211> 20
<212> DNA
<213> Artificial

<220>
<223> HOMO SAPIENS

<400> 40
tcgagtcatt gcatactgtc 20

<210> 41
<211> 19
<212> DNA
<213> Artificial

<220>
<223> HOMO SAPIENS

<400> 41
gaaggtgaag gtcggagtc 19

<210> 42
<211> 20
<212> DNA
<213> Artificial

<220>
<223> HOMO SAPIENS

<400> 42
gaagatggtg atgggatttc 20

<210> 43
<211> 20
<212> DNA
<213> Artificial

<220>

<223> HOMO SAPIENS

<400> 43
aggctgctgc cacataaggt 20

<210> 44
<211> 20
<212> DNA
<213> Artificial

<220>
<223> HOMO SAPIENS

<400> 44
ccaaggctcc aagcatgaat 20

<210> 45
<211> 22
<212> DNA
<213> Artificial

<220>
<223> HOMO SAPIENS

<400> 45
caaagtcacc gtcaaggtgt at 22

<210> 46
<211> 25
<212> DNA
<213> Artificial

<220>
<223> HOMO SAPIENS

<400> 46
ggaacagtct ttccgaagag accaa 25

<210> 47
<211> 25
<212> DNA
<213> Artificial

<220>
<223> HOMO SAPIENS

<400> 47
cagactaaca acagatttcg ggaat 25

<210> 48
<211> 24
<212> DNA
<213> Artificial

<220>
<223> HOMO SAPIENS

<400> 48

gaggaagtga tactccactc tcat 24